(19) Europäisches Patentamt
European Patent Office
Office européen des brevets

(11) EP 4 129 978 A1

(12) EUROPEAN PATENT APPLICATION
published in accordance with Art. 153(4) EPC

(43) Date of publication:
**08.02.2023 Bulletin 2023/06**

(21) Application number: **21776705.2**

(22) Date of filing: **26.03.2021**

(51) International Patent Classification (IPC):
***C07C 403/24** (1990.01)* ***C07J 41/00** (1974.07)*
***C07C 69/76** (1968.09)* ***C07C 323/26** (1990.01)*
***C07F 5/02** (1968.09)* ***C07H 15/252** (1985.01)*
***C07D 305/14** (1974.07)* ***C07D 491/22** (1974.07)*
***A61K 47/69** (2017.01)* ***A61K 47/36** (1990.01)*

(52) Cooperative Patent Classification (CPC):
**A61K 47/36; A61K 47/69; C07C 69/76; C07C 323/26; C07C 403/24; C07D 305/14; C07D 491/22; C07F 5/02; C07H 15/252; C07J 41/00**

(86) International application number:
**PCT/KR2021/003765**

(87) International publication number:
**WO 2021/194298 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2020 US 202062994932 P**

(71) Applicants:
- **Therabest Co., Ltd.**
  **Seoul 06656 (KR)**
- **INDUSTRIAL COOPERATION FOUNDATION JEONBUK NATIONAL UNIVERSITY**
  **Jeonju-si, Jeollabuk-do 54896 (KR)**

(72) Inventors:
- **HWANG, Do Won**
  **Suwon-si Gyeonggi-do 16700 (KR)**
- **KI, Young Wook**
  **Yongin-si Gyeonggi-do 16853 (KR)**
- **LEE, Dongwon**
  **Jeonju-si Jeollabuk-do 54873 (KR)**
- **YOO, Donghyuck**
  **Hwaseong-si Gyeonggi-do 18487 (KR)**

(74) Representative: **HGF**
**HGF Limited**
**1 City Walk**
**Leeds LS11 9DX (GB)**

# (54) NANOPARTICLES COMPRISING DRUG DIMERS, AND USE THEREOF

(57) The present invention relates to a nanoparticle comprising a drug dimer and a use thereof. The nanoparticle comprising a drug dimer of the present invention can increase the drug content and improve the dispersibility of the drug. In addition, the nanoparticle has increased targeting efficiency. Therefore, an effective pharmacological effect can be obtained by using a drug in a less amount, and thus the nanoparticle has excellent commercial applicability.

EP 4 129 978 A1

FIG. 4

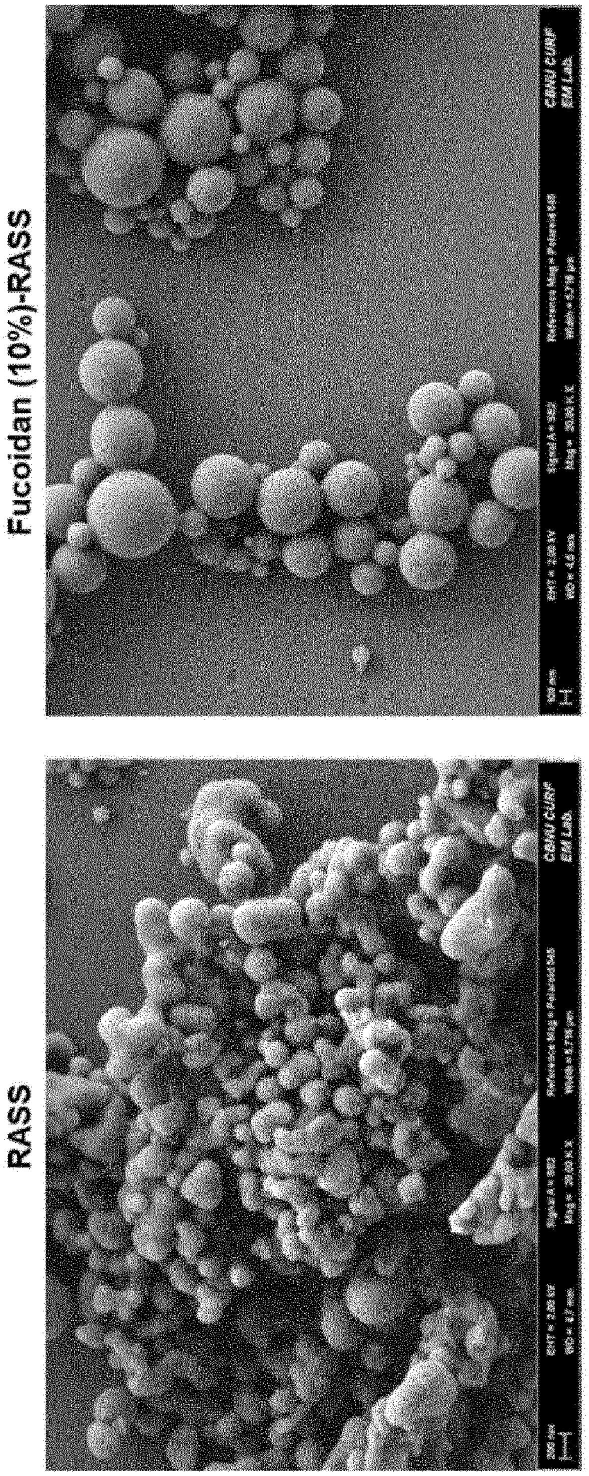

## Description

### Technical Field

The present invention relates to a nanoparticle comprising a drug dimer and a use thereof.

### Background Art

Hydrophobic drugs have difficulty in drug delivery despite their excellent pharmacological effects. Therefore, in order to increase the utility of hydrophobic drugs, many studies have been conducted on drug delivery. In particular, polymeric nanoparticles have been used for drug delivery systems to increase the low solubility of hydrophobic drugs and increase the circulation rate *in vivo,* but there are limitations in that methods of preparing them are complex and the drug content is low. In order to overcome these limitations, a dimer compound is synthesized by attaching hydrophobic drug monomers to both sides of a linker, and nanoparticles having a high drug content are prepared through self-assembly (Milena Menozzi et al., JPharmSci, June 1, 1984). However, the nanoparticles comprising the dimer compounds have a disadvantage in that stability and particle size are not homogenous.

### Detailed Description of Invention

### Technical Problem

Accordingly, as a result of studying to develop various drug dimers, the present inventors prepared a novel drug dimer. In addition, the present inventors developed a method of preparing a nanoparticle comprising a drug dimer, in which the nanoparticle has an increased stability and a homogenous particle size. In addition, when using a linker that decomposes in a specific environment, such as in the presence of GSH (Glutathione) or hydrogen peroxide, it was confirmed that it exhibits a more effective effect of treating a disease by releasing a drug monomer under a specific condition. Based on the above, the present inventors completed the present invention.

### Solution to Problem

In order to achieve the above object, in an aspect of the present invention, there is provided a novel drug dimer and a nanoparticle comprising the same.
In another aspect of the present invention, there is provided a nanoparticle comprising the drug dimer and fucoidan.
In another aspect of the present invention, there is provided a pharmaceutical composition comprising the nanoparticles as an active ingredient.

### Effects of Invention

A nanoparticle comprising a drug dimer and fucoidan, which is one embodiment of the present invention, can increase the drug content and improve the dispersibility of the drug. In addition, the nanoparticle has increased targeting efficiency. Therefore, an effective pharmacological effect can be obtained by using a drug in a less amount, and thus the toxicity of the drug can be significantly reduced. Therefore, the nanoparticle has excellent commercial applicability.

### Brief Description of Drawings

FIG. 1 illustrates a $^{1}$H NMR spectrum of a retinoid dimer compound (RASS).
FIG. 2 illustrates a mass spectrometer spectrum of a retinoid dimer compound (RASS).
FIG. 3 illustrates results obtained by confirming RASS nanoparticles comprising fucoidan by TEM and SEM.
FIG. 4 illustrates results obtained by confirming the shape and size of nanoparticles according to the presence or absence of fucoidan by SEM.
FIG. 5 illustrates the size distributions of nanoparticles according to the presence or absence of fucoidan.
FIG. 6 illustrates results obtained by confirming the size of particles according to the content of fucoidan by SEM. It was confirmed that the higher the content of fucoidan, the larger the size of particles.
FIG. 7 is a view illustrating SEM images for confirming the size of particles according to the content of bovine serum albumin (BSA). It was confirmed that the higher the content of BSA, the smaller the size of particles.

FIG. 8 illustrates results obtained by confirming the death rate of lung cancer cells (A549) and prostate cancer cells (DU145) according to treatment of retinoid dimer nanoparticles comprising fucoidan.

FIG. 9 illustrates results obtained by confirming the cancer targeting of retinoid dimer nanoparticles according to the presence or absence of fucoidan.

FIGs. 10 and 11 illustrate results obtained by confirming the cancer therapeutic effect of retinoid dimer nanoparticles according to the presence or absence of fucoidan.

FIG. 12 illustrates a result obtained by confirming the liver toxicity evaluation of retinoid dimer nanoparticles comprising fucoidan (Fu-RASS).

FIG. 13 illustrates a result obtained by analyzing the *in vivo* stability of retinoid nanoparticles comprising fucoidan (Fu-RASS).

FIG. 14 illustrates a $^{1}H$ NMR spectrum of a UDCA dimer compound (ssUDCA).

FIG. 15 illustrates a $^{13}C$ NMR spectrum of a UDCA dimer compound (ssUDCA).

FIG. 16 illustrates a mass spectrometer spectrum of a UDCA dimer compound (ssUDCA).

FIG. 17 is a view illustrating SEM images of nanoparticles comprising UDCA-SS-UDCA (ssUDCA). It was confirmed that the nanoparticles were agglomerated in shape, but the nanoparticles comprising fucoidan formed a spherical shape.

FIG. 18 illustrates results obtained by confirming the particle size after freeze-drying nanoparticles comprising UDCA-SS-UDCA and fucoidan.

FIG. 19 illustrates an image obtained by measuring the potential difference of nanoparticles comprising UDCA-SS-UDCA. The nanoparticles comprising fucoidan have specificity because they have a negative charge compared to nanoparticles, and the charge can be utilized as an index to determine the presence or absence of fucoidan.

FIG. 20 illustrates results obtained by confirming that UDCA-SS-UDCA nanoparticles comprising fucoidan have a high effect of selectively targeting cancer.

FIG. 21 illustrates a $^{1}H$ NMR spectrum of a PB dimer compound (ssPB).

FIG. 22 illustrates a $^{13}C$ NMR spectrum of a PB dimer compound (ssPB).

FIG. 23 illustrates a mass spectrometer spectrum of a PB dimer compound (ssPB).

FIG. 24 illustrates a size distribution and an electron microscope image of nanoparticles comprising a PB dimer compound (ssPB) according to the addition ratio of fucoidan.

FIG. 25 illustrates a result obtained by evaluating the cytotoxicity of ssPB nanoparticles comprising fucoidan.

FIG. 26 illustrates results obtained by confirming whether or not ssPB nanoparticles comprising fucoidan induce the apoptosis of cancer cells through flow cytometry.

FIG. 27 illustrates results obtained by confirming whether or not ssPB nanoparticles specifically target a tumor according to the presence or absence of fucoidan.

FIG. 28 illustrates a result obtained by analyzing ALT of mice injected with ssPB nanoparticles comprising fucoidan.

FIG. 29 illustrates a $^{1}H$ NMR spectrum of sBR, which is a PB dimer compound.

FIG. 30 illustrates a $^{13}C$ NMR spectrum of sBR.

FIG. 31 illustrates a mass spectrometer spectrum of sBR.

FIG. 32 illustrates results obtained by confirming sBR nanoparticles according to the content of fucoidan by SEM and TEM.

FIG. 33 is a view illustrating the size and distribution of sBR nanoparticles.

FIG. 34 illustrates results obtained by analyzing the size of nanoparticles according to the content of fucoidan when sBR nanoparticles are prepared.

FIG. 35 illustrates a result obtained by analyzing the surface potential of nanoparticles according to the content of fucoidan when sBR nanoparticles are prepared.

FIG. 36 illustrates a result obtained by confirming the generation of sBR nanoparticles comprising fucoidan through a SEM image.

FIG. 37 illustrates a result obtained by analyzing ALT of mice injected with nanoparticles comprising fucoidan.

FIG. 38 illustrates a result obtained by evaluating toxicity to important organs of mice injected with nanoparticles comprising fucoidan.

FIG. 39 illustrates a $^{1}H$ NMR spectrum of ssBR, which is a PB dimer compound.

FIG. 40 illustrates a $^{13}C$ NMR spectrum of ssBR.

FIG. 41 illustrates a mass spectrometer spectrum of ssBR.

FIG. 42 illustrates a size distribution of ssBR nanoparticles according to the presence or absence of fucoidan.

FIG. 43 illustrates transmission electron microscope images of ssBR nanoparticles according to the content of fucoidan added.

FIG. 44 illustrates a result obtained by confirming the generation of ssBR nanoparticles comprising fucoidan through SEM images.

FIG. 45 illustrates a result obtained by analyzing the effect of reducing glutathione in SW620 cells by ssBR nanoparticles.

FIG. 46 illustrates a result obtained by confirming the toxicity evaluation of ssBR nanoparticles on SW620 cells.

FIG. 47 is an image showing whether or not ssBR nanoparticles comprising fucoidan specifically target a tumor.

FIG. 48 illustrates a result obtained by evaluating liver toxicity of ssBR nanoparticles comprising fucoidan.

FIG. 49 illustrates photographs of stained organ of mice to which ssBR nanoparticles comprising fucoidan were administered.

FIG. 50 illustrates a comparison of $^1$H NMR spectra of paclitaxel dimer (PTX-SS-PTX) and paclitaxel (PTX).

FIG. 51 illustrates results obtained by confirming the size and distribution of PTX-SS-PTX nanoparticles according to the presence or absence of fucoidan by SEM and DLS.

FIG. 52 illustrates results obtained by evaluating the tumor targeting ability of PTX-SS-PTX nanoparticles according to the presence or absence of fucoidan.

FIG. 53 illustrates a $^1$H NMR spectrum of a doxorubicin dimer (DOX-SS-DOX).

FIG. 54 illustrates results obtained by analyzing the size and distribution of DOX-SS-DOX nanoparticles according to the presence or absence of fucoidan using a particle size analyzer and SEM.

FIG. 55 illustrates a comparison of $^1$H NMR analysis results of CPT dimer and CPT.

FIG. 56 illustrates a result obtained by analyzing the size and distribution of CPT-SS-CPT nanoparticles according to the presence or absence of fucoidan using a particle size analyzer and SEM.

FIG. 57 is a schematic diagram of an experimental method for comparing the toxicity of doxorubicin dimer nanoparticles comprising fucoidan with the toxicity of doxorubicin.

FIG. 58 is a table showing a result obtained by measuring the volume of the tumor and the weight of the mice in the mice injected with tumor cells in order to confirm the toxicities of a control, doxorubicin, and doxorubicin dimer nanoparticles comprising fucoidan. G5 refers to a negative control, G6 refers to a group administered with doxorubicin, and G7 refers to a group administered with doxorubicin dimer nanoparticles comprising fucoidan.

FIG. 59 is a schematic diagram of changes in the volume of the tumor in the mice injected with tumor cells upon administration of a control, doxorubicin, and doxorubicin dimer nanoparticles comprising fucoidan.

FIG. 60 is a schematic diagram of changes in the body weight in the mice injected with tumor cells upon administration of a control, doxorubicin, and doxorubicin dimer nanoparticles comprising fucoidan.

## Best Mode for Carrying out the Invention

As used herein, the term "solvate" refers to a compound solvated in an organic or inorganic solvent. The solvate is, for example, a hydrate.

As used herein, the term "salt" refers to an inorganic and organic acid addition salt of a compound. The pharmaceutically acceptable salt may be a salt that does not cause serious irritation to the organism to which the compound is administered, and does not impair the biological activity and physical properties of the compound. The inorganic acid salt may be hydrochloride, bromate, phosphate, sulfate, or disulfate. The organic acid salt may be formate, acetate, propionate, lactate, oxalate, tartrate, malate, maleate, citrate, fumarate, besylate, camsylate, edisylate, trichloroacetate, trifluoroacetate, benzoate, gluconate, methanesulfonate, glycolate, succinate, 4-toluenesulfonate, galacturonate, embonate, glutamate, ethanesulfonate, benzenesulfonate, p-toluenesulfonate, or aspartate. The metal salt may be a calcium salt, a sodium salt, a magnesium salt, a strontium salt, or a potassium salt.

As used herein, the term "fucoidan" is a sulfated polysaccharide having a sticky viscous structure, and is a component generally contained in brown algae such as seaweed and kelp, and has a molecular weight of 20 kDa on average, and is a substance in which fucose, which is a basic sugar, and sulfuric acid groups are bound. Fucoidan is known to have various physiological and biological activities such as antioxidants, anticoagulants, anticancer agents, and antibiotics.

As used herein, the term "nanoparticle comprising fucoidan" refers to the presence of fucoidan in the nanoparticle. The term may be expressed as a nanoparticle coated with fucoidan. In addition, the term "nanoparticle comprising a drug dimer and fucoidan" refers to a nanoparticle formed by aggregation of fucoidan and a drug dimer, and fucoidan may be present inside and/or outside the nanoparticle.

As used herein, the term "retinoid" refers to a natural or synthetic derivative of vitamin A, and is classified into retinol, which is retinoid that is naturally present in the human body, vitamin A, retinaldehyde, retinal, vitamin A aldehyde, and retinoic acid. Retinoid is activated in the form of retinoic acid *in vivo,* and is used in the treatment of several skin diseases through various actions. Retinoic acid activated *in vivo* not only regulates the proliferation and differentiation of keratinocytes, but also inhibits sebaceous glands and regulates immunity. Therefore, it is widely used in the treatment of diseases such as acne and psoriasis or malignant tumors such as skin cancer and T cell lymphoma.

As used herein, the term "ursodeoxycholic acid (UDCA)" is a major component of Bear’s gall, which is gall bladder of a bear, and has strong detoxification ability. Therefore, the UDCA is used as a therapeutic agent for liver diseases by activating liver detoxification and metabolic functions and preventing cholesterol from accumulating in the

liver. Currently, the UDCA is reported to be effective in treating colorectal cancer, liver cancer, pancreatic cancer and the like in addition to bile or bile duct disease.

As used herein, the term "4-(hydroxymethyl)phenyl benzoate" is also referred to as PB, and is decomposed *in vivo* to generate quinone methide (QM). The quinone methide is known to have anticancer activity.

As used herein, the term "paclitaxel (PTX)" is an anticancer substance that is most often used for the treatment of breast cancer, ovarian cancer, head and neck cancer, kaposi's sarcoma, non-small cell lung cancer and the like.

As used herein, the term "doxorubicin (DOX)" is an anthracycline-based anticancer agent that is widely used in breast cancer, lung cancer, lymphoma, gastrointestinal cancer and sarcoma, and causes immunological death i.e., immunogenic cell death (ICD) against cancer cells.

As used herein, the term "camptothecin (CPT)" is an alkaloid component isolated from the bark and stem of a tree called *Camptotheca acuminata,* and is a topoisomerase inhibitor. It has a wide range of anticancer activity and is well known as a first-line drug used in the treatment of metastatic rectal cancer. In addition, it is also used in the treatment of lung cancer, ovarian cancer, mammary cancer, gastric cancer, pancreatic cancer and the like.

Hereinafter, the present invention will be described in more detail.

**Retinoid dimer and nanoparticle comprising the same**

One aspect of the present invention provides a retinoid dimer compound represented by formula 1a below, a solvate or a pharmaceutically acceptable salt thereof:

[Formula 1a]

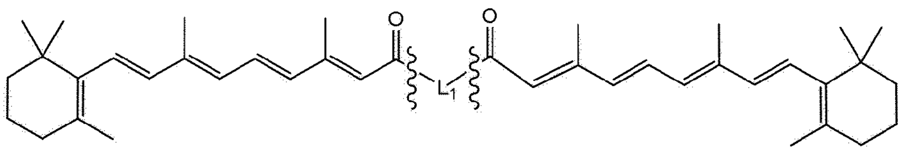

in formula 1a above,

$L_1$ may be a linker of $C_{2-10}$ and include -S-, -SS-, -SSS-, -SeSe-, or -Se-.

The linker may be any one selected from the group consisting of

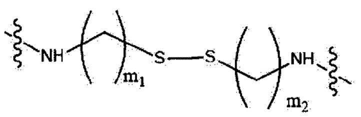 , 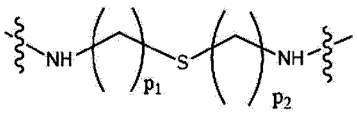 , 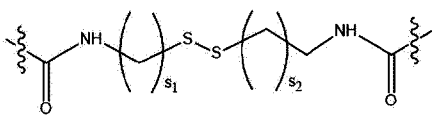 ,

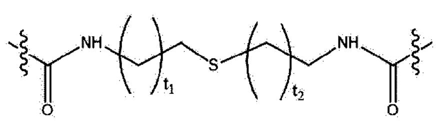 , 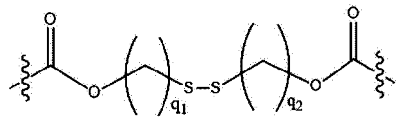

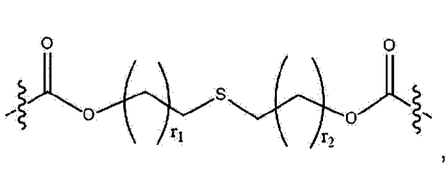 , 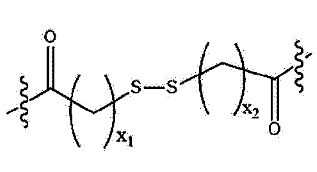 and 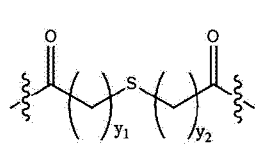 ,

and $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ may be each an integer of 1 to 10.

In one embodiment, $L_1$ may be

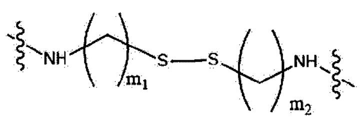

or

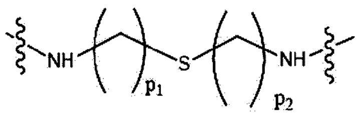

.

$m_1$, $m_2$, $p_1$ and $p_2$ are each an integer of 1 to 10. Specifically, $m_1$, $m_2$, $p_1$ and $p_2$ may be each 1, 2, 3, 4, 5, 6, 7, 8 or 10.

Here, the dimer compound may be one in which any one or two monomers of the compounds represented by formulas 1b to 1d below are bound to each other:

[Formula 1b]

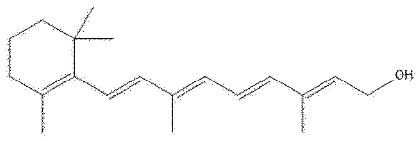

;

[Formula 1c]

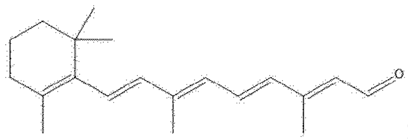

;

and

[Formula 1d]

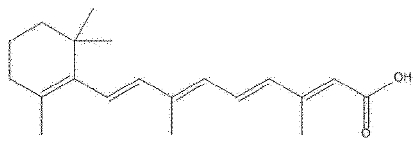

.

One embodiment of the dimer compound may be represented by formula 1e below:

[Formula 1e]

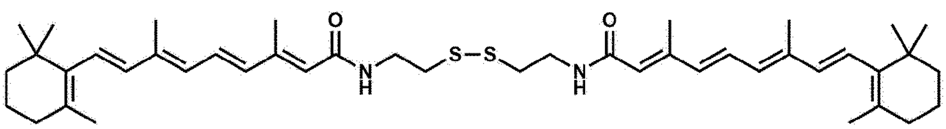

.

Another aspect of the present invention provides a nanoparticle comprising the retinoid dimer compound.

Here, the size of the nanoparticle may range from 200 nm to 1,500 nm. Specifically, the size of the nanoparticle may range from 200 nm to 500 nm.

Another aspect of the present invention provides a nanoparticle comprising the retinoid dimer compound and fucoidan.

Here, the size of the nanoparticle may range from 200 nm to 300 nm. Here, the fucoidan may be comprised in an amount of 10 to 30 parts by weight based on 100 parts by weight of the dimer compound.

Another aspect of the present invention provides a pharmaceutical composition comprising the nanoparticles. Here, the nanoparticle may be a nanoparticle comprising the retinoid dimer compound, or a nanoparticle comprising the retinoid dimer compound and fucoidan.

The pharmaceutical composition may be for the prevention or treatment of cancer or skin disease.

**Ursodeoxycholic acid dimer and nanoparticle comprising the same**

One aspect of the present invention provides a ursodeoxycholic acid dimer compound represented by formula 2a below, a solvate or a pharmaceutically acceptable salt thereof:

[Formula 2a]

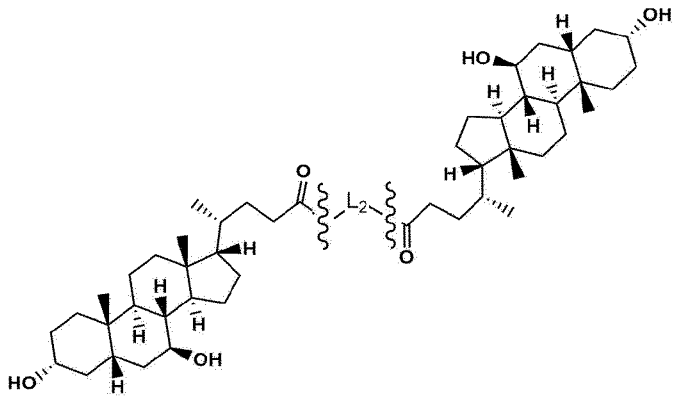

in formula 2a above,

$L_2$ may be a linker of $C_{2-10}$ and include -S-, -SS-, -SSS-, -SeSe-, or -Se-.

The linker may be any one selected from the group consisting of

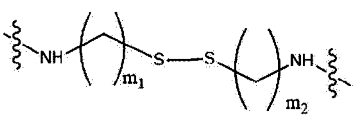
,
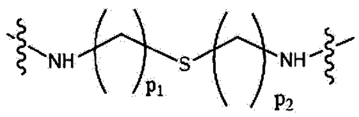
,
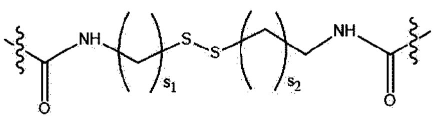
,
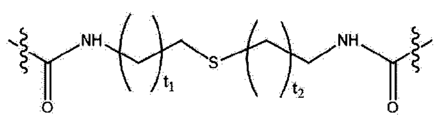
,
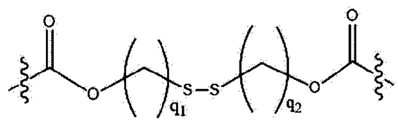
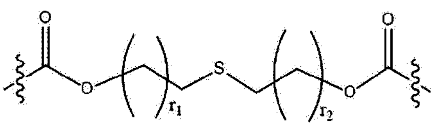
,
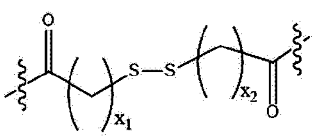
and
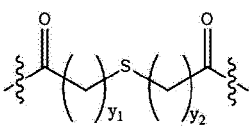

and $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ may be each an integer of 1 to 10.

In one embodiment, $L_2$ may be

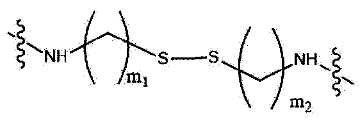

or

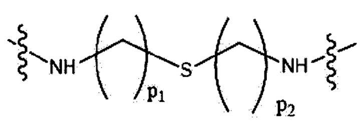
.

$m_1$, $m_2$, $p_1$ and $p_2$ are each an integer of 1 to 10. Specifically, $m_1$, $m_2$, $p_1$ and $p_2$ may be each 1, 2, 3, 4, 5, 6, 7, 8 or 10.

Here, the dimer compound may be one in which a compound represented by formula 2b below is bound to each other:

[Formula 2b]

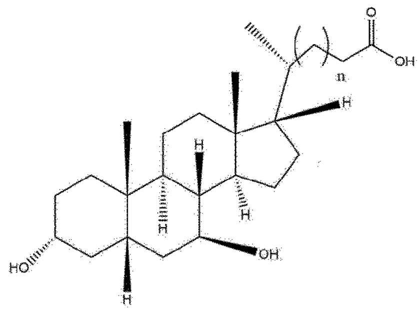

in formula 2b above, n is each an integer of 1 to 10.

One embodiment of the dimer compound may be represented by formula 2c below:

[Formula 2c]

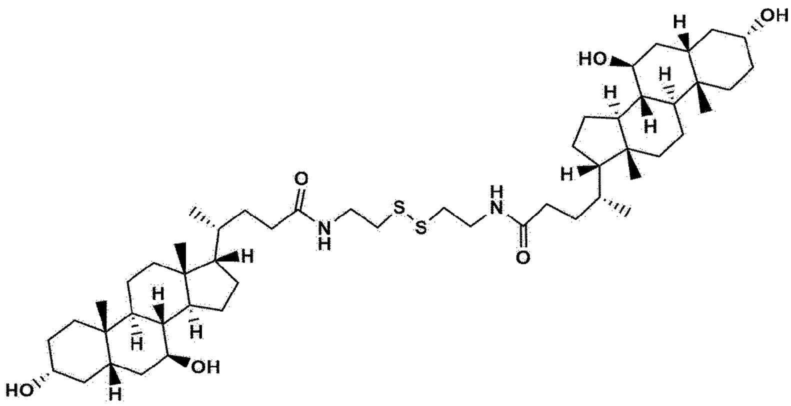

Another aspect of the present invention provides a nanoparticle comprising the ursodeoxycholic acid dimer compound.

Here, the size of the nanoparticle may range from 200 nm to 500 nm.

Another aspect of the present invention provides a nanoparticle comprising the ursodeoxycholic acid dimer compound and fucoidan.

Here, the size of the nanoparticle may range from 200 nm to 500 nm. Here, the fucoidan may be comprised in an amount of 10 to 30 parts by weight based on 100 parts by weight of the dimer compound.

Another aspect of the present invention provides a pharmaceutical composition comprising the nanoparticles. Here, the nanoparticle may be a nanoparticle comprising the ursodeoxycholic acid dimer compound, or a nanoparticle comprising the ursodeoxycholic acid dimer compound and fucoidan.

The pharmaceutical composition may be for the prevention or treatment of liver disease, cancer, or cardiovascular disease.

**4-(hydroxymethyl)phenyl benzoate dimer and nanoparticle comprising the same**

One aspect of the present invention provides a 4-(hydroxymethyl)phenyl benzoate dimer compound represented by formula 3a below, a solvate, or a pharmaceutically acceptable salt thereof:

[Formula 3a]

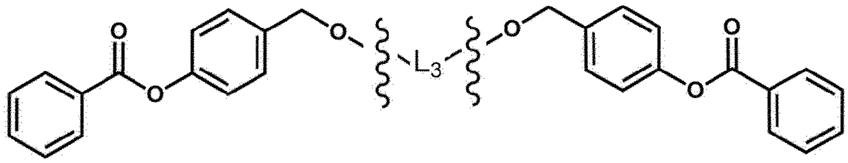

in formula 3a above,

$L_3$ may be a linker of $C_{2-10}$ and include -S-, -SS-, -SSS-, -SeSe-, or -Se-.

The linker may be any one selected from the group consisting of

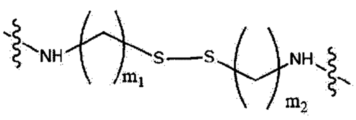 , 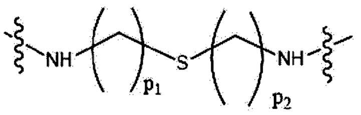 , 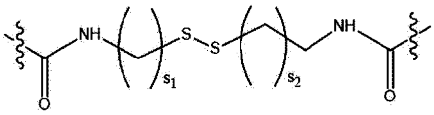 ,

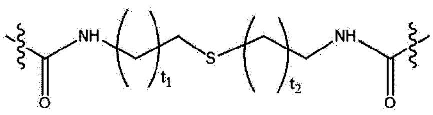 , 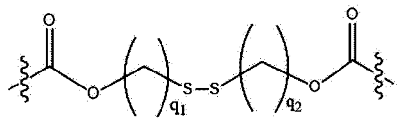

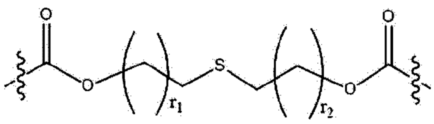 , 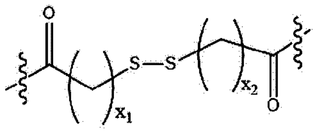 and 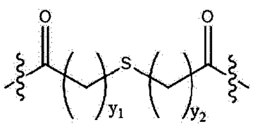 ,

and $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ may be each an integer of 1 to 10.

In one embodiment, $L_3$ may be

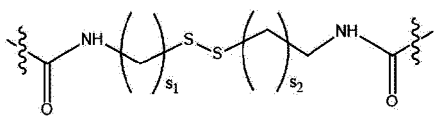

or

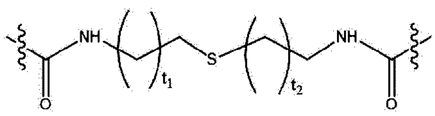 .

$s_1$, $s_2$, $t_1$ and $t_2$ are each an integer of 1 to 10. Specifically, $s_1$, $s_2$, $t_1$ and $t_2$ may be each 1, 2, 3, 4, 5, 6, 7, 8 or 10.

Here, the dimer compound may be one in which a compound represented by formula 3b below is bound to each other:

[Formula 3b]

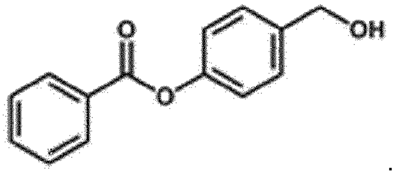 .

One embodiment of the dimer compound may be represented by formula 3c below:

[Formula 3c]

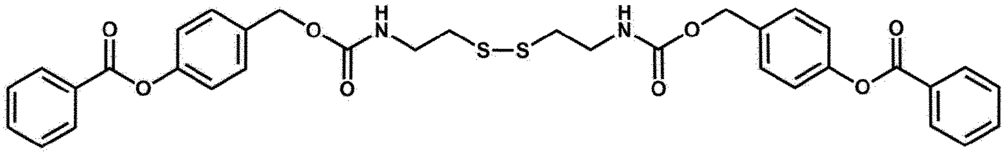

.

Another aspect of the present invention provides a nanoparticle comprising the 4-(hydroxymethyl)phenyl benzoate dimer compound.

Here, the size of the nanoparticle may range from 200 nm to 600 nm.

Another aspect of the present invention provides a nanoparticle comprising the 4-(hydroxymethyl)phenyl benzoate dimer compound and fucoidan.

Here, the size of the nanoparticle may range from 100 nm to 500 nm. Here, the fucoidan may be comprised in an amount of 10 to 30 parts by weight based on 100 parts by weight of the dimer compound.

Another aspect of the present invention provides a pharmaceutical composition comprising the nanoparticles. Here, the nanoparticle may be a nanoparticle comprising the 4-(hydroxymethyl)phenyl benzoate dimer compound, or a nanoparticle comprising the 4-(hydroxymethyl)phenyl benzoate dimer compound and fucoidan.

The pharmaceutical composition may be for the prevention or treatment of cancer.

**4-(hydroxymethyl)phenylboronic acid pinacol ester dimer and nanoparticle comprising the same**

One aspect of the present invention provides a 4-(hydroxymethyl)phenylboronic acid pinacol ester dimer compound represented by formula 4a below, a solvate or a pharmaceutically acceptable salt thereof:

[Formula 4a]

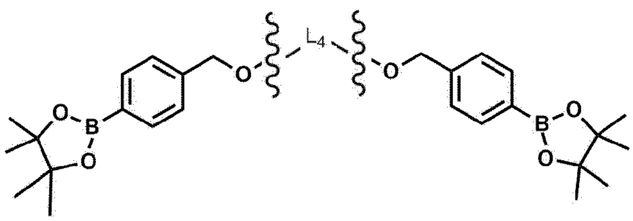

in formula 4a above, $L_4$ may be a linker of $C_{2-10}$ and include -S-, -SS-, -SSS-, -SeSe-, or -Se-.

The linker may be any one selected from the group consisting of

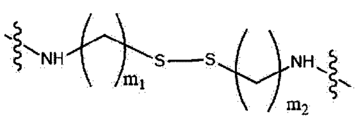
,
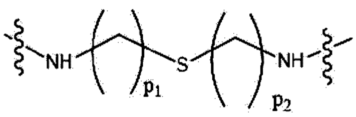
,
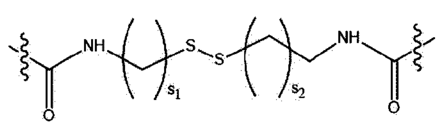
,
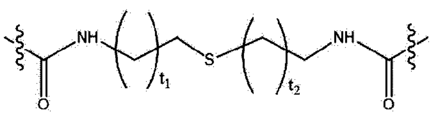
,
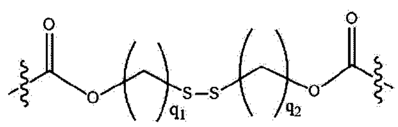

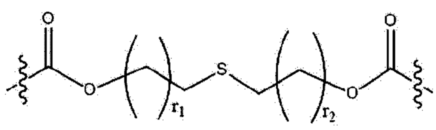
,
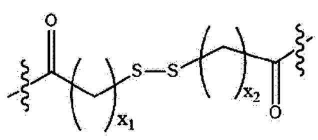
and
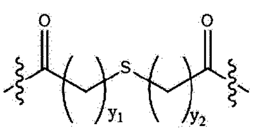
,

and $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ may be each an integer of 1 to 10.

In one embodiment, $L_4$ may be any one selected from the group consisting of

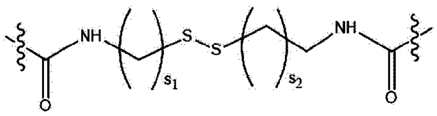
,
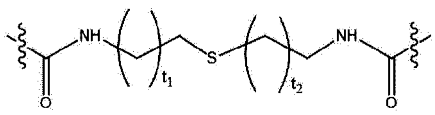
,
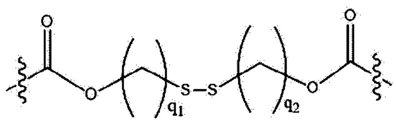
and
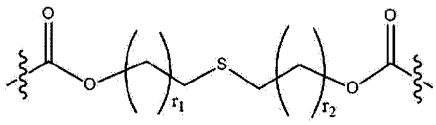
.

$s_1$, $s_2$, $t_1$, $t_2$, $q_1$, $q_2$, $r_1$ and $r_2$ are each an integer of 1 to 10. $s_1$, $s_2$, $t_1$, $t_2$, $q_1$, $q_2$, $r_1$ and $r_2$ may be each 1, 2, 3, 4, 5, 6, 7, 8 or 10.

Here, the dimer compound may be one in which a compound represented by formula 4b below is bound to each other:

[Formula 4b]

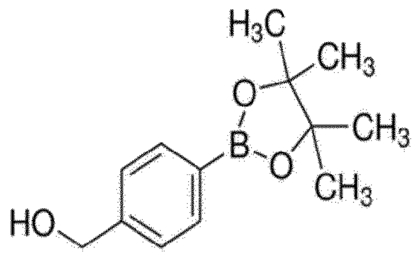
.

One embodiment of the dimer compound may be represented by formula 4c or formula 4d below:

[Formula 4c]

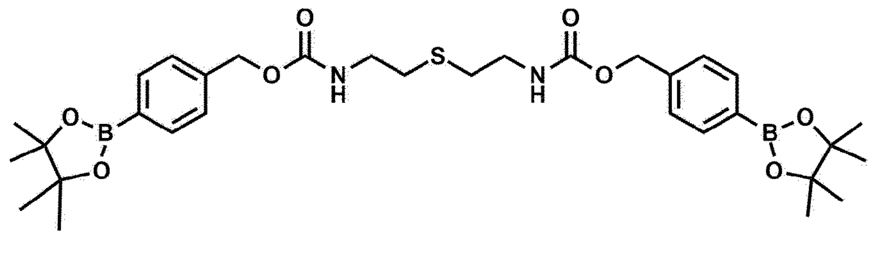
;

or

[Formula 4d]

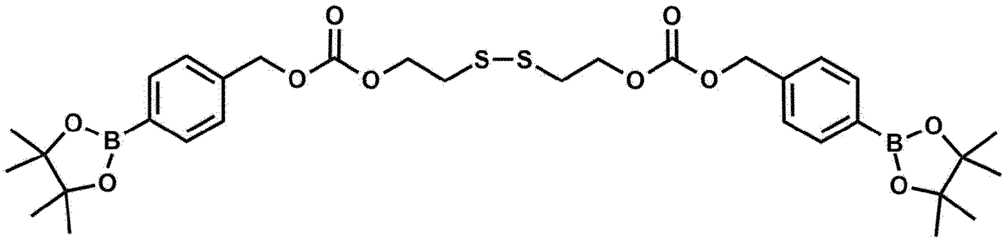
.

Another aspect of the present invention provides a nanoparticle comprising the 4-(hydroxymethyl)phenylboronic acid pinacol ester dimer compound.

Here, the size of the nanoparticle may range from 100 nm to 800 nm.

Another aspect of the present invention provides a nanoparticle comprising the 4-(hydroxymethyl)phenylboronic acid pinacol ester dimer compound and fucoidan.

Here, the size of the nanoparticle may range from 200 nm to 500 nm. Here, the fucoidan may be comprised in an amount of 10 to 30 parts by weight based on 100 parts by weight of the dimer compound.

Another aspect of the present invention provides a pharmaceutical composition comprising the nanoparticles. Here, the nanoparticle may be a nanoparticle comprising the 4-(hydroxymethyl)phenylboronic acid pinacol ester dimer compound, or a nanoparticle comprising the 4-(hydroxymethyl)phenylboronic acid pinacol ester dimer compound and fucoidan.

The pharmaceutical composition may be for the prevention or treatment of cancer.

**Paclitaxel dimer and nanoparticle comprising the same**

One aspect of the present invention provides a paclitaxel dimer compound represented by formula 5a below, a solvate or a pharmaceutically acceptable salt thereof:

[Formula 5a]

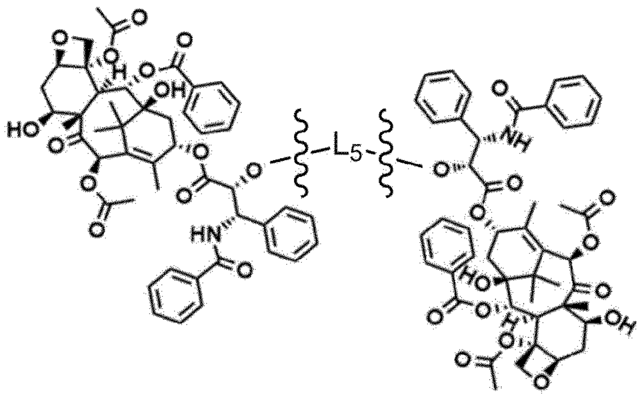

in formula 5a above, $L_5$ may be a linker of $C_{2-10}$ and include -S-, -SS-, -SSS-, -SeSe-, or -Se-.

The linker may be any one selected from the group consisting of

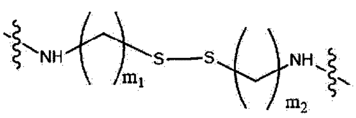,
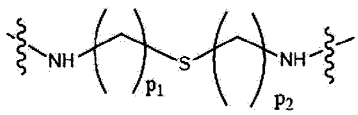,
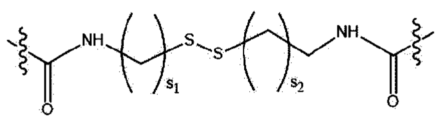,

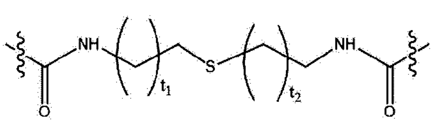,
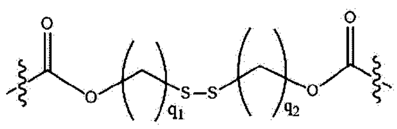

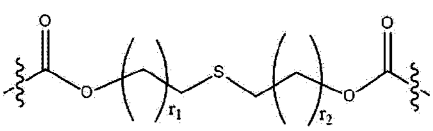,
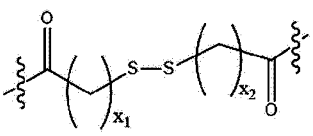
and
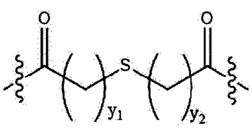,

and $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ may be each an integer of 1 to 10.

In one embodiment, $L_5$ may be

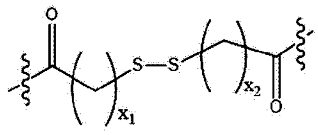 or 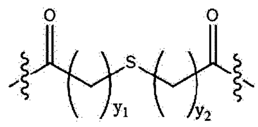.

$x_1$, $x_2$, $y_1$ and $y_2$ are each an integer of 1 to 10. Specifically, $x_1$, $x_2$, $y_1$ and $y_2$ may be each 1, 2, 3, 4, 5, 6, 7, 8 or 10.

Here, the dimer compound may be one in which a compound represented by formula 5b below is bound to each other:

[Formula 5b]

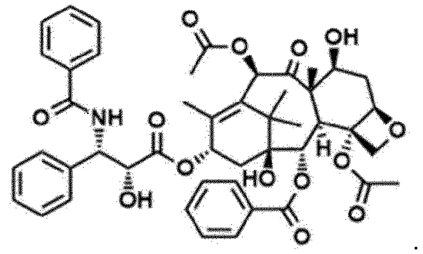.

One embodiment of the dimer compound may be represented by formula 5c below:

[Formula 5c]

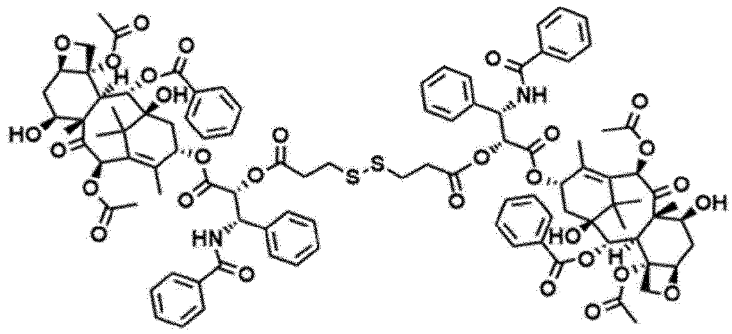.

Another aspect of the present invention provides a nanoparticle comprising the paclitaxel dimer compound.

Here, the size of the nanoparticle may range from 100 nm to 400 nm.

Another aspect of the present invention provides a nanoparticle comprising the paclitaxel dimer compound and fucoidan.

Here, the size of the nanoparticle may range from 100 nm to 300 nm. Here, the fucoidan may be comprised in an amount of 10 to 30 parts by weight based on 100 parts by weight of the dimer compound.

Another aspect of the present invention provides a pharmaceutical composition comprising the nanoparticle. Here, the nanoparticle may be a nanoparticle comprising the paclitaxel dimer compound, or a nanoparticle comprising the paclitaxel dimer compound and fucoidan.

The pharmaceutical composition may be for the prevention or treatment of cancer.

**Doxorubicin dimer and nanoparticle comprising the same**

One aspect of the present invention provides a doxorubicin dimer compound represented by formula 6a below, a solvate or a pharmaceutically acceptable salt thereof:

[Formula 6a]

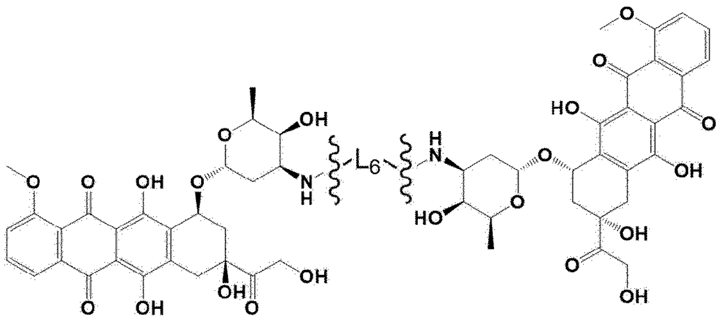

in formula 6a above, $L_6$ may be a linker of $C_{2-10}$ and include -S-, -SS-, -SSS-, -SeSe-, or -Se-.

The linker may be any one selected from the group consisting of

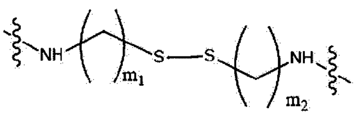 , 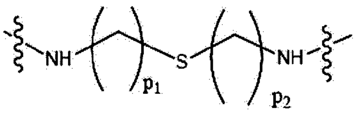 , 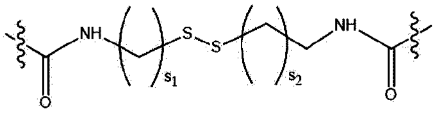 ,

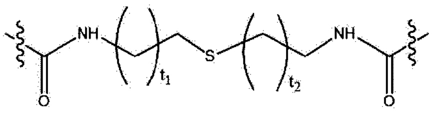 , 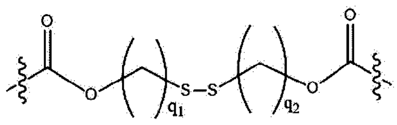

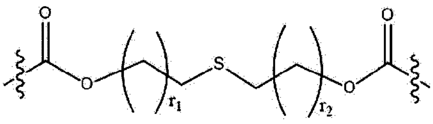 , 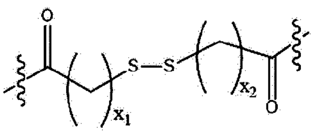 and 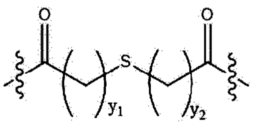 ,

and $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ may be each an integer of 1 to 10.

In one embodiment, $L_6$ may be

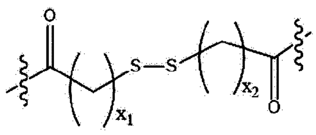 or 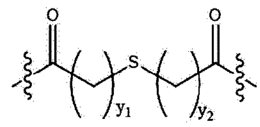 .

$x_1$, $x_2$, $y_1$ and $y_2$ are each an integer of 1 to 10. Specifically, $x_1$, $x_2$, $y_1$ and $y_2$ may be each 1, 2, 3, 4, 5, 6, 7, 8 or 10.

Here, the dimer compound may be one in which a compound represented by formula 6b below is bound to each other:

[Formula 6b]

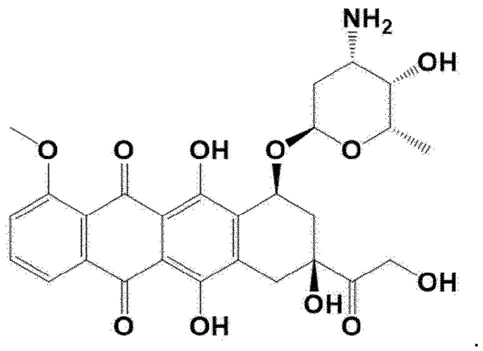

One embodiment of the dimer compound may be represented by formula 6c below:

[Formula 6c]

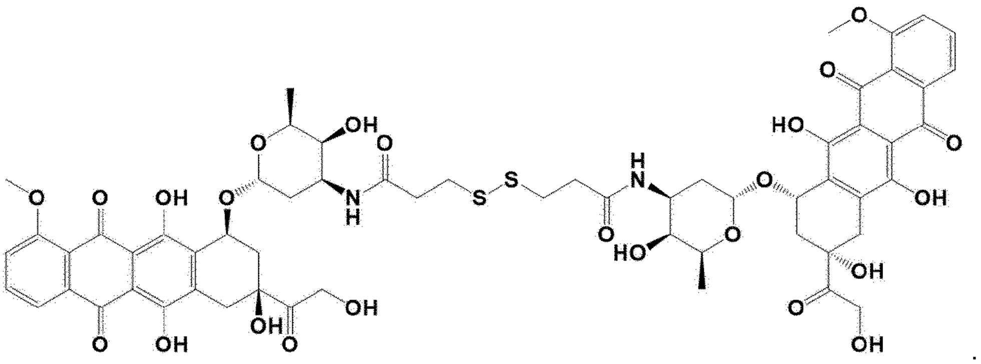

Another aspect of the present invention provides a nanoparticle comprising the doxorubicin dimer compound.

Here, the size of the nanoparticle may range from 150 nm to 200 nm.

Another aspect of the present invention provides a nanoparticle comprising the doxorubicin dimer compound and fucoidan.

Here, the size of the nanoparticle may range from 100 nm to 200 nm. Here, the fucoidan may be comprised in an amount of 10 to 30 parts by weight based on 100 parts by weight of the dimer compound.

Another aspect of the present invention provides a pharmaceutical composition comprising the nanoparticles. Here, the nanoparticle may be a nanoparticle comprising the doxorubicin dimer compound, or a nanoparticle comprising the doxorubicin dimer compound and fucoidan.

The pharmaceutical composition may be for the prevention or treatment of cancer.

**Camptothecin dimer and nanoparticle comprising the same**

One aspect of the present invention provides a camptothecin dimer compound represented by formula 7a below, a solvate or a pharmaceutically acceptable salt thereof:

[Formula 7a]

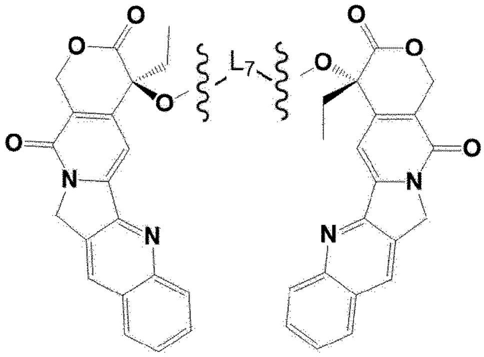

in formula 7a above, $L_7$ may be a linker of $C_{2-10}$ and include -S-, -SS-, -SSS-, -SeSe-, or -Se-.

The linker may be any one selected from the group consisting of

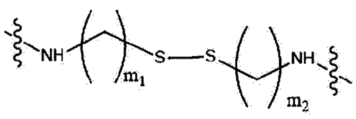
,
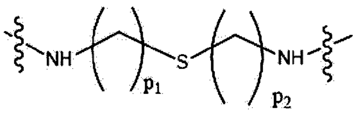
,
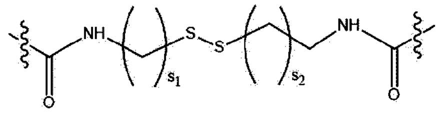
,
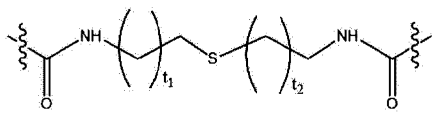
,
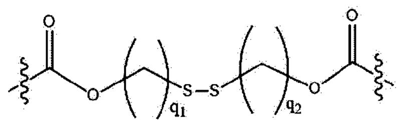
,
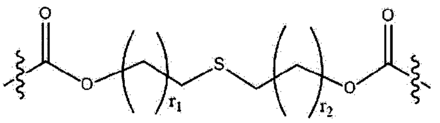
,
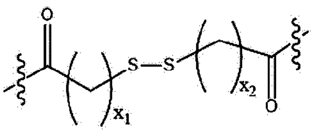
and
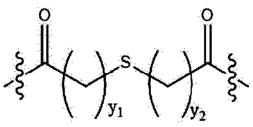
,

and $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ may be each an integer of 1 to 10.

In one embodiment, $L_7$ may be

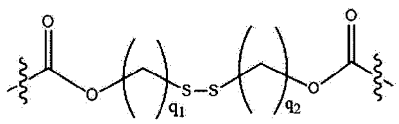

or

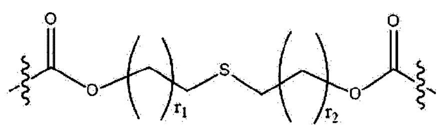
.

$q_1$, $q_2$, $r_1$ and $r_2$ are each an integer of 1 to 10. Specifically, $q_1$, $q_2$, $r_1$ and $r_2$ may be each 1, 2, 3, 4, 5, 6, 7, 8 or 10.

Here, the dimer compound may be one in which a compound represented by formula 7b below is bound to each other:

[Formula 7b]

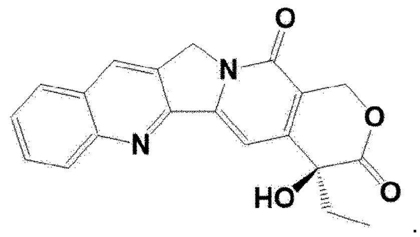

One embodiment of the dimer compound may be represented by formula 7c below:

[Formula 7c]

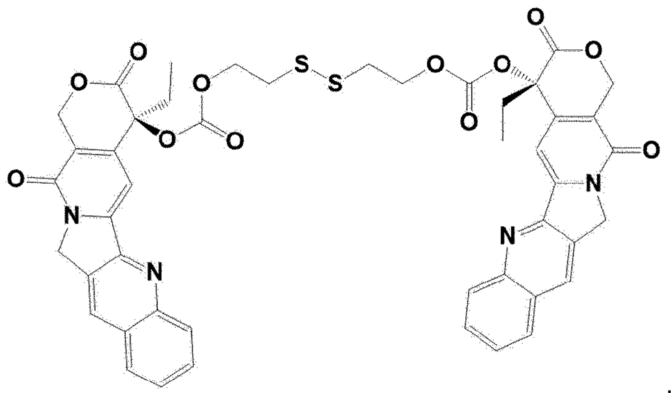

Another aspect of the present invention provides a nanoparticle comprising the camptothecin dimer compound.

Here, the size of the nanoparticle may range from 100 nm to 600 nm.

Another aspect of the present invention provides a nanoparticle comprising the camptothecin dimer compound and fucoidan.

Here, the size of the nanoparticle may range from 100 nm to 300 nm. Here, the fucoidan may be comprised in an amount of 10 to 30 parts by weight based on 100 parts by weight of the dimer compound.

Another aspect of the present invention provides a pharmaceutical composition comprising the nanoparticles. Here, the nanoparticle may be a nanoparticle comprising the camptothecin dimer compound, or a nanoparticle comprising the camptothecin dimer compound and fucoidan.

The pharmaceutical composition may be for the prevention or treatment of cancer.

**Nanoparticle comprising drug dimer and fucoidan and use thereof**

One aspect of the present invention provides a nanoparticle comprising a dimer compound consisting of structural formula (I) below:

A-L-A (I)

in structural formula (I) above, A may be a drug containing two or more aromatic rings, in which -OH, -NH, and/or -COOH is present. Specifically, the drug may be any one selected from the group consisting of retinoid, ursodeoxycholic acid, 4-(hydroxymethyl)phenyl benzoate, 4-(hydroxymethyl)phenylboronic acid pinacol ester, paclitaxel, doxorubicin, and camptothecin.

L may be a linker of $C_{2-10}$ and include -S-, -SS-, -SSS-, -SeSe-, or -Se-. Specifically, L may be any one selected from the group consisting of

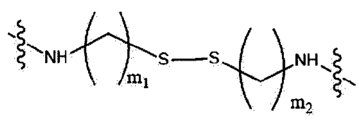

,

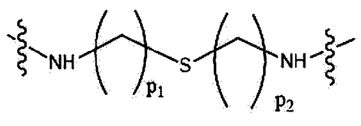 , 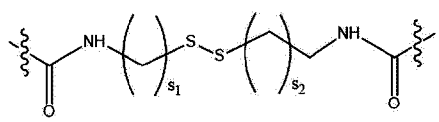

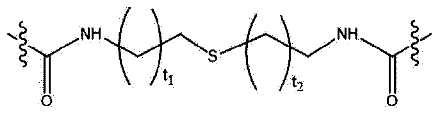 , 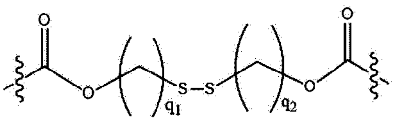

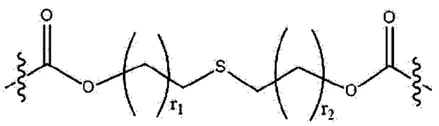 , 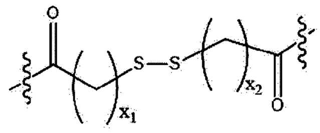 and 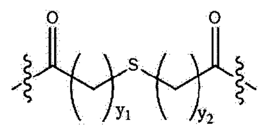 .

Here, $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ are each an integer of 1 to 10. Specifically, $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ may be each 1, 2, 3, 4, 5, 6, 7, 8 or 10.

The linking site between the drug and the linker may consist of a structure capable of hydrolysis such as ester, amide, carbonate, carbamate, urea and the like. Specifically, the linker may be decomposed by glutathione or reactive oxygen species.

As used herein, the term "nanoparticle comprising a drug dimer" may be expressed in various ways. For example, when -SS- is included as a linker, it may be described as a nanoparticle comprising a retinoid-SS-retinoid dimer or a retinoid-SS-retinoid dimer nanoparticle. In addition, when -S- is included as a linker, it may be described as a nanoparticle comprising a retinoid-S-retinoid dimer or a retinoid-S-retinoid dimer nanoparticle.

Here, the nanoparticle may further comprise fucoidan. Here, the fucoidan may be included in an amount of 30% or less of the total content of the nanoparticles. Specifically, the drug dimer and fucoidan may have a weight ratio of 70:30 to 95:5. In one embodiment, the drug dimer and fucoidan may have a weight ratio of 70:30, 75:25, 80:20, 85:15, 90:10, or 95:5.

Here, the nanoparticle comprising fucoidan may have an excellent effect of targeting to p-Selectin by fucoidan.

Another aspect of the present invention provides a pharmaceutical composition comprising the nanoparticles.

As used herein, the term "prevention" refers to any action that inhibits or delays the onset of cancer, skin disease, liver disease, or cardiovascular disease by the administration of the pharmaceutical composition. As used herein, the term "treatment" refers to any action that improves or beneficially modifies symptoms of a disease related to cancer, skin disease, liver disease, or cardiovascular disease by the administration of the pharmaceutical composition.

The pharmaceutical composition may comprise a pharmaceutically acceptable carrier. The carrier is used in the sense of including an excipient, diluent or adjuvant. The carrier may be selected from the group consisting of, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, physiological saline, a buffer such as PBS, methyl hydroxybenzoate, propyl hydroxybenzoate, talc, magnesium stearate, and mineral oil. The composition may comprise a filler, an anti-aggregating agent, a lubricant, a wetting agent, a flavoring agent, an emulsifying agent, a preservative, or a combination thereof.

The pharmaceutical composition may be prepared in any formulation according to conventional methods. The composition may be formulated, for example, in an oral formulation (for example, a powder, a tablet, a capsule, a syrup, a pill, or a granule), or parenteral formulation (for example, an injection). In addition, the composition may be prepared as a systemic formulation or a topical formulation.

In the pharmaceutical composition, the solid preparation for oral administration may be a tablet, a pill, a powder, a granule, or a capsule. The solid preparation may further comprise an excipient. The excipient may be, for example, starch, calcium carbonate, sucrose, lactose, or gelatin. In addition, the solid preparation may further comprise a lubricant such as magnesium stearate or talc. In the pharmaceutical composition, the liquid preparation for oral administration may be a suspension, an internal solution, an emulsion, or a syrup. The liquid preparation may comprise water or liquid paraffin. The liquid preparation may comprise an excipient, for example, a wetting agent, a sweetening agent, a flavoring agent, or a preservative. In the pharmaceutical composition, the preparation for parenteral administration may be a sterile aqueous solution, a non-aqueous solvent, a suspension, an emulsion, a freeze-dried preparation and/or a suppository. The non-aqueous solvent or suspension may comprise a vegetable oil or an ester. The vegetable oil may be, for example, propylene glycol, polyethylene glycol, or olive oil. The ester may be, for example, ethyl oleate. The base

of the suppository may be witepsol, macrogol, tween 61, cacao butter, laurin butter, or glycerogelatin.

The pharmaceutical composition comprises nanoparticles comprising a drug dimer compound according to one aspect or nanoparticles comprising a drug dimer compound and fucoidan as an active ingredient of the pharmaceutical composition. The "active ingredient" refers to a physiologically active substance used to achieve pharmacological activity (for example, cancer treatment).

The pharmaceutical composition may comprise nanoparticles comprising a drug dimer compound according to one aspect or nanoparticles comprising a drug dimer compound and fucoidan in an effective amount. As used herein, the term "effective amount" refers to an amount sufficient to exhibit the effect of preventing or treating a disease when administered to a subject in need of prevention or treatment. The effective amount can be appropriately selected by a person skilled in the art according to the selected cell or subject. The preferred dosage of the pharmaceutical composition may vary depending on the condition and body weight of the subject, the severity of disease, the drug formulation, the route and duration of administration, but may be appropriately selected by a person skilled in the art. However, the nanoparticles comprising a drug dimer compound or the nanoparticles comprising a drug dimer compound and fucoidan may be administered, for example, in an amount of about 0.0001 mg/kg to about 100 mg/kg, or about 0.001 mg/kg to about 100 mg/kg, which may be divided into once to 24 times a day, 1 to 7 times every 2 days to 1 week, or once to 24 times every 1 month to 12 months. In the pharmaceutical composition, the compound, solvate or pharmaceutically acceptable salt thereof may be included in an amount of about 0.0001% by weight to about 10% by weight, or about 0.001% by weight to about 1% by weight based on the total weight of the entire composition.

The method of administration may be oral or parenteral administration. The method of administration may be, for example, oral, transdermal, subcutaneous, rectal, intravenous, intraarterial, intraperitoneal, intramuscular, intrasternal, topical, intranasal, intratracheal, or intradermal route. The composition may be administered systemically or topically, and may be administered alone or in combination with other pharmaceutically active compounds.

The pharmaceutical composition may be for the prevention or treatment of a disease selected from the group consisting of cancer, skin disease, liver disease, and cardiovascular disease.

Specifically, the pharmaceutical composition may be applied to the prevention or treatment of gastric cancer, liver cancer, lung cancer, colorectal cancer, breast cancer, prostate cancer, ovarian cancer, pancreatic cancer, cervical cancer, thyroid cancer, laryngeal cancer, acute myelogenous leukemia, brain tumor, neuroblastoma, retinoblastoma, head and neck cancer, salivary gland cancer, and lymphoma.

Another aspect of the present invention provides a use of a pharmaceutical composition comprising nanoparticles comprising a dimer compound consisting of structural formula (I) according to one aspect; or nanoparticles comprising a dimer compound consisting of structural formula (I) and fucoidan for the prevention or treatment of a disease related to cancer, skin disease, liver disease, or cardiovascular disease.

The compound of structural formula (I) above is as described above. In addition, the content of fucoidan and the disease are as described above.

## Method for preventing and treating disease using nanoparticles

Another aspect of the present invention provides a method for preventing or treating a disease related to cancer, skin disease, liver disease, or cardiovascular disease, comprising: administering to a subject nanoparticles comprising a dimer compound consisting of structural formula (I) according to one aspect; or nanoparticles comprising a dimer compound consisting of structural formula (I) and fucoidan.

The compound of structural formula (I) is as described above. In addition, the subject may be a mammal, for example, a human, a mouse, a rat, a cow, a horse, a pig, a dog, a monkey, a sheep, a goat, an ape, or a cat. The subject may be a subject who suffers from or is likely to suffer from symptoms associated with the disease.

The method of administration may be oral or parenteral administration. The method of administration may be, for example, oral, transdermal, subcutaneous, rectal, intravenous, intraarterial, intraperitoneal, intramuscular, intrasternal, topical, intranasal, intratracheal, or intradermal route. The pharmaceutical composition may be administered systemically or topically, and may be administered alone or in combination with other pharmaceutically active compounds.

The preferred dosage of the pharmaceutical composition may vary depending on the condition and body weight of the patient, the severity of disease, the drug formulation, the route and duration of administration, but may be appropriately selected by a person skilled in the art. The dosage may be, for example, in the range of about 0.001 mg/kg to about 100 mg/kg, about 0.01 mg/kg to about 10 mg/kg, or about 0.1 mg/kg to about 1 mg/kg based on the adult dosage. The dosage may be administered once a day, multiple times a day, or once a week, once every two weeks, once every three weeks, or once every four weeks to once a year.

## Method of preparing nanoparticle comprising drug dimer and fucoidan

One aspect of the present invention provides a method of preparing a nanoparticle comprising a drug dimer

and fucoidan, comprising: mixing a dimer compound consisting of structural formula (I) below and fucoidan at a weight ratio of 80:20 to 95:5:

A-L-A (I)

in structural formula (I) above,
A is any one selected from the group consisting of retinoid, ursodeoxycholic acid, 4-(hydroxymethyl)phenyl benzoate, 4-(hydroxymethyl)phenylboronic acid pinacol ester, paclitaxel, doxorubicin, and camptothecin.

L may be a linker of $C_{2-10}$ and include -S-, -SS-, -SSS-, -SeSe-, or -Se-. Specifically, L is any one selected from the group consisting of

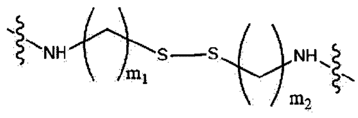
,

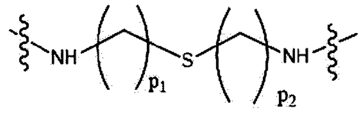
,
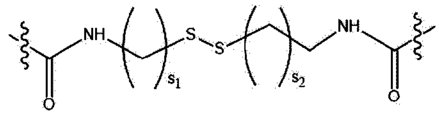

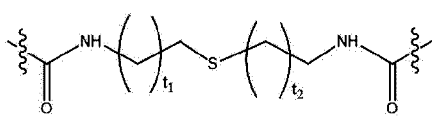
,
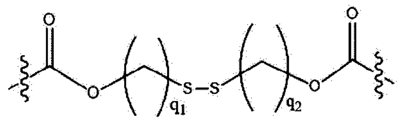

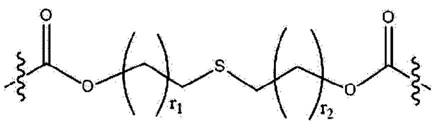
,
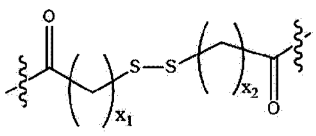
and
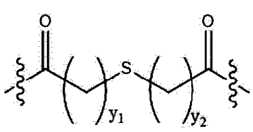
,

and $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ are each an integer of 1 to 10. Specifically, $m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ may be each 1, 2, 3, 4, 5, 6, 7, 8 or 10.

In the present specification, a nanoparticle refers to a compound formed through self-assembly of a dimer compound. In one embodiment of the present invention, the nanoparticle has a nano-unit size, and may be specifically formed in a size of 1 to 999 nm, 100 to 900 nm, 100 to 800 nm, 100 to 700 nm, 100 to 600 nm, 100 to 500 nm, 100 to 400 nm, or 100 to 300 nm. More specifically, it may be formed in a size of 200 to 300 nm. However, it is not limited thereto, and may vary depending on the type of drug.

In the present specification, a linker is a structure that connects drug monomers. For example, there are a sulfide bond-based linker, a thioketal-based linker, a selenide-based linker and the like. However, it is not limited thereto. In one embodiment of the present invention, the sulfide bond-based linker may include a sulfide bond or a disulfide bond.

In one embodiment of the present invention, the linker may be decomposed by glutathione or reactive oxygen species. However, it is not limited thereto.

In the present specification, a target cell refers to a cell having a membrane protein to which fucoidan is capable of binding. In one embodiment of the present invention, a target cell may be a cancer cell, a cardiovascular cell, or a liver cell. However, it is not limited thereto.

The weight ratio of a drug dimer and fucoidan may be 80:20 to 95:5. In one embodiment of the present invention, a dispersant may be added to the fucoidan solution in order to increase the dispersibility of the nanoparticles. Specifically, the dispersant includes polyvinyl alcohol (PVA). However, it is not limited thereto.

In one embodiment of the present invention, after preparing the nanoparticles, it may be performed by further comprising a step of freeze-drying. However, it is not limited thereto.

## Mode for Carrying out the Invention

Hereinafter, the present invention will be described in more detail by way of the following examples. However, the following examples are only for illustrating the present invention, and the scope of the present invention is not limited thereto.

## I. Nanoparticles comprising retinoid dimer compound

### Example 1. Preparation of retinoid dimer compound

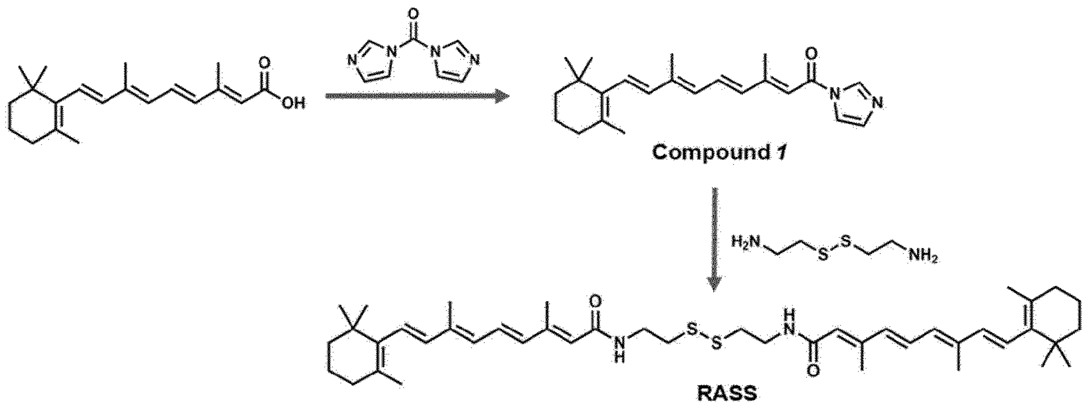

Compound 1

RASS

### Step 1: Synthesis of Compound 1

Retinoic acid (2 g) and 1,1'-carbonyldiimidazole (1.08 g) were dissolved in dichloromethane (20 mL) and then reacted at room temperature for 30 minutes. The mixture was separated and purified by silica gel chromatography (ethyl acetate/hexane, 1:1) to synthesize Compound 1.

### Step 2: Synthesis of retinoid dimer compound (RASS)

Compound 1 (2 g) and cystamine dihydrochloride (1.74 g) were dissolved in 5 mL of dimethyl sulfoxide under nitrogen filling. After raising the temperature to 47°C, the reaction was carried out while stirring for 12 hours. Dimethyl sulfoxide and water were removed through water precipitation, centrifugation, and freeze-drying processes, and then separated and purified by silica gel chromatography (ethyl acetate/hexane, 1:1) to synthesize a retinoid dimer compound (RASS) (FIGs. 1 and 2).

$^1$H NMR (400 MHz, DMSO-d6): δ 1.16 (12H, s), 1.33 (4H, m), 1.55 (4H, m), 1.72 (6H, s), 1.97 (6H, s), 2.06-2.16 (10H, m), 3.31-3.35 (8H, m), 6.10 (2H, s), 6.30-6.47 (6H, m), 6.65 (2H, d), 6.81 (2H, dd).

### Example 2. Preparation of nanoparticles comprising retinoid dimer compound

The nanoparticles were prepared by dispersing a RASS solution, which 50 mg of RASS prepared in Example 1 above was dissolved in 0.2 mL of methanol, using a syringe. Thereafter, methanol was removed using a rotary evaporator. The solution in which the nanoparticles were produced was centrifuged at 4°C for 5 minutes under a condition of 15,000xg. After centrifugation, the supernatant was removed, and the resulting pellet was dispersed in PBS, and then centrifuged once more under the same conditions. The resulting pellet was dispersed in about 2 mL of PBS, frozen with liquid nitrogen, and then freeze-dried. The completely dried nanoparticles were used by dispersing in PBS immediately before use. On the other hand, the RASS nanoparticles were excellent in re-dispersibility, so the use of PVA was not needed.

### Comparative Example 1. Preparation of nanoparticles comprising retinoid dimer compound and albumin

100 mg of RASS was dissolved in 1 mL of methanol, and then the mixture was stirred while slowly adding to 20 mL of PBS in which 20 mg to 50 mg of BSA was dissolved. The mixture was sonicated for 3 minutes and homogenized for 2 minutes using a homogenizer. After removing methanol while stirring at room temperature for 5 hours, the RASS

emulsion was centrifuged for 4 minutes at a rate of 11,000xg. The precipitated RASS nanoparticles were washed with water and then freeze-dried to obtain the RASS nanoparticles.

**Example 3. Preparation of nanoparticles comprising retinoid dimer compound and fucoidan**

50 mg of RASS prepared in Example 1 above was dissolved in 0.2 mL of methanol, and 10 mg of fucoidan was dissolved in 1 mL of PBS. The RASS solution dissolved in methanol was added dropwise to a fucoidan solution (10 mL) dissolved in PBS using a syringe, and the nanoparticles formed during sonication were dispersed. Thereafter, methanol was removed for 15 minutes under a condition of room temperature using a rotary evaporator. The solution in which the nanoparticles were produced was centrifuged at 4°C for 5 minutes under a condition of 15,000×g. After centrifugation, the supernatant was removed, and the resulting pellet was dispersed in PBS, and then centrifuged once more under the same conditions. The resulting pellet was dispersed in about 2 mL of PBS and then frozen with liquid nitrogen and then freeze-dried. The completely dried nanoparticles were used by dispersing in PBS immediately before use. On the other hand, the RASS nanoparticles were excellent in re-dispersibility, so the use of PVA was not needed (FIGs. 3, 4, 5, and 6).

In addition, in order to anaylize physical properties when preparing nanoparticles using a biologically derived polymer (albumin) other than fucoidan, the RASS nanoparticles comprising albumin were prepared as in Comparative Example 1 and compared and analyzed (FIG. 7).

As a result, when albumin was used, nanoparticles were formed when treated at a higher concentration. When fucoidan was used, nanoparticles were formed even when treated at a low concentration. Therefore, it was confirmed that nanoparticles are well formed when fucoidan is used compared to albumin.

**Experimental Example 1. Confirmation of cytotoxicity of nanoparticles comprising retinoid dimer compound (MTT)**

The retinoid dimer nanoparticles comprising fucoidan prepared in Example 3 above and retinoic acid were dispersed in PBS, and each of lung cancer A549 and prostate cancer DU145 cells was treated therewith. After 24 hours treatment, 100 μL of MTT solution was added on the cells. After 3 hours, 1 mL of DMSO was added to dissolve the crystal. After 10 minutes, the absorbance was measured at 570 nm to analyze the cell viability. As a result, it was confirmed that cell death appeared when the cells were treated with the nanoparticles at a high level (FIG. 8).

**Experimental Example 2. Confirmation of cancer targeting ability of nanoparticles comprising retinoid dimer and fucoidan**

The cancer targeting ability of each of the nanoparticles (RASS, Fu-RASS) prepared in Examples 2 and 3 above was examined. Tumorigenesis was induced by injecting about $2\times10^6$ A549 cells subcutaneously into the left leg of nude mice (average body weight of about 20 g, 8 weeks old). After about 10 days, when the size of the tumor was greater than 3x3 mm, each of the nanoparticles was intravascularly injected through the tail vein. For the tumor target image, nanoparticles carrying a fluorescent substance IR780 were used, and the targeting of the tumor was confirmed by comparative analysis for about 2 days. As a result, it was confirmed that the nanoparticles comprising fucoidan accumulate in cancer at a high concentration over time (FIG. 9).

**Experimental Example 3. Confirmation of cancer therapeutic effect of nanoparticles comprising retinoid dimer and fucoidan**

The cancer therapeutic efficacy of each of the nanoparticles (RASS, Fu-RASS) prepared in Examples 2 and 3 above was confirmed. Tumorigenesis was induced by injecting about $2\times10^6$ A549 cells subcutaneously into the left leg of nude mice (average body weight of about 20 g, 8 weeks old). After about 10 days, when the size of the tumor was greater than 3x3 mm, each of the nanoparticles was intravascularly injected daily for 3 days through the tail vein. For 30 days, the cancer size and the weight of the mice were monitored, and the therapeutic effect was compared and analyzed. As a result, the nanoparticles comprising fucoidan showed excellent cancer scavenging ability (FIGs. 10 and 11).

**Experimental Example 4. Evaluation of liver toxicity of retinoid dimer nanoparticles comprising fucoidan**

In order to evaluate liver toxicity, the retinoid (Fu-RASS) nanoparticles comprising fucoidan were intravascularly injected into normal mice at a concentration of 20 mg/kg. After 5 injections once every 3 days, blood was collected and analyzed for alanine transaminase (ALT). The liver, heart, lung, spleen, and kidney were excised, and the tissues were

stained by H&E and analyzed for safety *in vivo* through histological analysis. It was confirmed that the toxicity of the substance was less because inflammation was not expressed in other organs including liver toxicity (FIGs. 12 and 13).

**II. Nanoparticles comprising ursodeoxycholic acid dimer compound**

**Example 4. Preparation of ursodeoxycholic acid dimer (ssUDCA) compound**

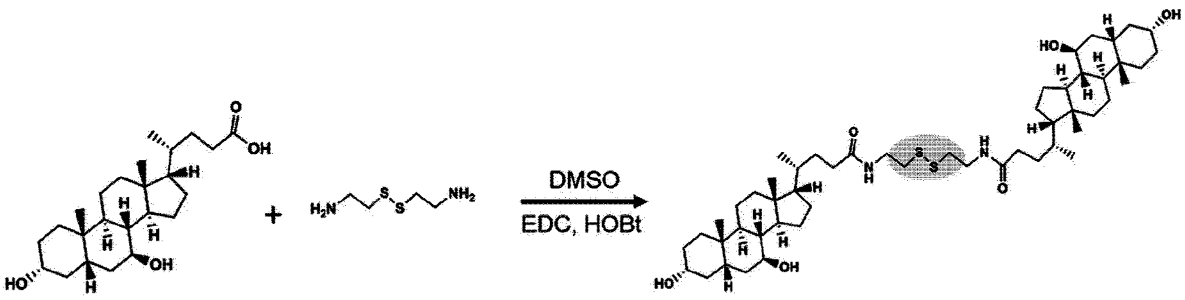


Ursodeoxycholic acid (2.54 mmol), 1-ethyl-3-(dimethylaminophenyl) carbonyldiimide (5.089 mmol), and hydroxybenzotriazole (5.089 mmol) were added to a round flask, and 20 mL DMSO was added. After completely dissolving, the flask was placed in ice water, and cystamine dihydrochloride (1.211 mmol) dissolved in 1 mL of DMSO was added dropwise while stirring. Trimethylamine (6.539 mmol) was slowly added to the reaction mixture and reacted at a temperature of 40°C for 48 hours. Trimethylamine was removed using a rotary evaporator and added to 250 ml of distilled water, and the precipitate was obtained by centrifugation at a rate of 12,000xg for 10 minutes. All water was removed through freeze-drying to obtain ssUDCA (FIGs. 14, 15, and 16).

$^1$H NMR (400 MHz, DMSO-d6): $\delta$ 0.71 (6H, s), 0.98 (12H, d), 1.00-1.31 (10H, m), 1.36-2.18 (38H, m), 2.58 (4H, s), 2.82 (4H, s), 3.32-3.51 (4H, m), 3.84 (2H, s), 4.49 (2H, s), 8.42 (2H, s).

**Example 5. Preparation of nanoparticles comprising ursodeoxycholic acid dimer compound**

30 mg of ssUDCA was dissolved in 1 mL of tetrahydrofuran and then added dropwise to 10 mL of distilled water while stirring. The mixed solution was sonicated for 1 minute to homogenize. Tetrahydrofuran was removed while stirring at room temperature for 3 hours and then centrifuged at a rate of 12,000xg for 8 minutes. The ssUDCA nanoparticles forming a precipitate were washed three times with water and freeze-dried. The dried nanoparticles were used by dispersing in PBS immediately before use. On the other hand, the UDCA nanoparticles were excellent in re-dispersibility, so the use of PVA was not needed (FIG. 17).

**Example 6. Preparation of nanoparticles comprising ursodeoxycholic acid dimer compound and fucoidan**

30 mg of ssUDCA was dissolved in 1 mL of tetrahydrofuran, and then 3 mg of fucoidan was dissolved in 10 mL of distilled water to prepare each solution. The ssUDCA solution was added dropwise using an injection needle while stirring rapidly the fucoidan solution. The mixed solution was sonicated for 1 minute to homogenize. Tetrahydrofuran was removed while stirring at room temperature for 3 hours and then centrifuged at a rate of 12,000xg for 8 minutes. The ssUDCA nanoparticles forming a precipitate were washed three times with water and freeze-dried. The dried nanoparticles were used by dispersing in PBS immediately before use. On the other hand, the UDCA nanoparticles were excellent in re-dispersibility, so the use of PVA was not needed (FIGs. 17, 18, and 19).

**Experimental Example 5. Confirmation of cancer targeting ability of ursodeoxycholic acid dimer nanoparticles comprising fucoidan**

The cancer targeting ability of each of the nanoparticles prepared in Examples 5 and 6 above was examined. Tumorigenesis was induced by injecting about $2\times10^6$ SW620 cells subcutaneously into the left leg of nude mice (average body weight of about 20 g, 8 weeks old). After about 10 days, when the size of the tumor was greater than 3x3 mm, each of the nanoparticles was intravascularly injected through the tail vein.

For the tumor target image, nanoparticles carrying a fluorescent substance IR780 were used, and the targeting of the tumor was confirmed by comparative analysis based on 12 hours. As a result, it was confirmed that the nanoparticles

comprising fucoidan have excellent cancer targeting ability (FIG. 20).

**III. Nanoparticles comprising 4-(hydroxymethyl)phenyl benzoate dimer compound**

**Example 7. Preparation of 4-(hydroxymethyl)phenyl benzoate dimer compound**

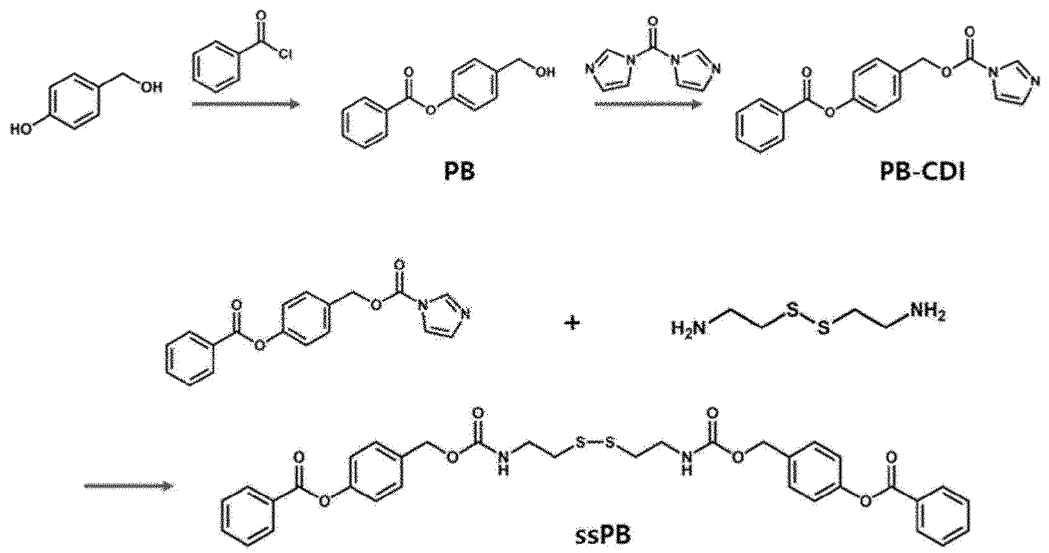

PB

PB-CDI

ssPB

**Step 1: Synthesis of PB**

Hydroxybenzyl alcohol (6.0 g) and triethylamine (6.73 mL) were dissolved in 250 mL of dichloromethane and stirred at 0°C for 30 minutes. Benzoyl chloride (6 mL) was dissolved in 50 mL of dichloromethane, and then added to a hydroxybenzyl alcohol solution, and then reacted at room temperature for 12 hours. The prepared compound (PB) was obtained through a silica column using ethyl acetate/hexane (1:3).

**Step 2: Synthesis of PB-CDI**

The prepared PB (3.0 g) and 1,1'-carbonyldiimidazole (3.0 g) were dissolved in 15 mL of dichloromethane and then reacted for 1 hour at room temperature. The synthesized substance (PB-CDI) was obtained through a silica column using ethyl acetate/hexane (1:2).

**Step 3: Synthesis of ssPB**

Cystamine dihydrochloride (0.47 g) was dissolved in 9 mL of dimethyl sulfoxide under a nitrogen environment. The prepared PB-CDI (2.0 g) was dissolved in 1 mL of dimethyl sulfoxide and then mixed with a cystamine dihydrochloride solution. It was reacted for 12 hours at a temperature of 40°C. Water and dichloromethane were added to the reaction solution to extract the product, and then dichloromethane was removed using a rotary evaporator. The product was precipitated by adding 10 mL of methanol to the concentrated product solution, and then separated through a silica column (FIGs. 21, 22, and 23).

$^1$H NMR (400 MHz, DMSO-d6): $\delta$ 2.82 (4H, t), 3.27 (4H, t), 5.02 (4H, s), 7.24 (4H, d), 7.43 (4H, d), 7.58 (4H, t), 7.73 (2H, t), 8.11 (4H, t).

**Example 8. Preparation of nanoparticles comprising 4-(hydroxymethyl)phenyl benzoate dimer compound**

30 mg of ssPB was dissolved in 1 mL of tetrahydrofuran and then added dropwise to 15 mL of distilled water using an injection needle while stirring rapidly. The mixed solution was sonicated for 1 minute to homogenize. Tetrahydrofuran was removed while stirring at room temperature for 3 hours and then centrifuged at a rate of 12,000xg for 8 minutes. The ssUDCA nanoparticles forming a precipitate were washed three times with water and freeze-dried. The dried nanoparticles were used by dispersing in PBS immediately before use. On the other hand, the UDCA nanoparticles

were excellent in re-dispersibility, so the use of PVA was not needed.

**Example 9. Preparation of nanoparticles comprising 4-(hydroxymethyl)phenyl benzoate dimer compound and fucoidan**

30 mg of ssPB was dissolved in 1 mL of tetrahydrofuran, and then 3 mg of fucoidan was dissolved in 15 mL of distilled water to prepare each solution. The ssUDCA solution was added dropwise using an injection needle while stirring rapidly the fucoidan solution. The mixed solution was sonicated for 1 minute to homogenize. Tetrahydrofuran was removed while stirring at room temperature for 3 hours and then centrifuged at a rate of 12,000xg for 8 minutes. The ssUDCA nanoparticles forming a precipitate were washed three times with water and freeze-dried. The dried nanoparticles were used by dispersing in PBS immediately before use. On the other hand, the UDCA nanoparticles were excellent in re-dispersibility, so the use of PVA was not needed (FIG. 24).

**Experimental Example 6. Confirmation of toxicity of 4-(hydroxymethyl)phenyl benzoate dimer (ssPB) nanoparticles comprising fucoidan**

The cytotoxicity of the ssPB nanoparticles was analyzed by the MTT (3-(4,5-dimethylthiazol-2-yl)-2,5-diphenyltetrazolium bromide) method. SW620 and DU145 cells were treated with the ssPB nanoparticles. After 24 hours treatment, 100 μL of MTT solution was added. After 3 hours, 1 mL of DMSO was added to dissolve the crystal. After 10 minutes, the absorbance was measured at 570 nm to analyze the cell viability. In addition, the cell viability was analyzed according to the presence or absence of N-acetylcysteine (NAC), which is an antioxidant (FIG. 25).

In order to confirm the cell apoptosis induction of the ssPB nanoparticles, a cell apoptosis marker Annexin V-FITC and a viability marker propidium iodide were used and the cells were analyzed by flow cytometer. It was confirmed that the cell death rate increased as the concentration increased (FIG. 26).

**Experimental Example 7. Confirmation of cancer targeting ability of 4-(hydroxymethyl)phenyl benzoate dimer (ssPB) nanoparticles comprising fucoidan**

The cancer targeting ability of each of the nanoparticles prepared in Examples 8 and 9 above was examined. Tumorigenesis was induced by injecting about $2 \times 10^6$ SW620 cells subcutaneously into the left leg of nude mice (average body weight of about 20 g, 8 weeks old). After about 10 days, when the size of the tumor was greater than 3x3 mm, each of the nanoparticles was intravascularly injected through the tail vein.

For the tumor target image, nanoparticles carrying a fluorescent substance IR780 were used, and the targeting of the tumor was confirmed by comparative analysis based on 24 hours. The ssPB nanoparticles to which fucoidan was added continued to accumulate into the tumor for up to 24 hours, but most of the nanoparticles to which fucoidan was not added were accumulated into the liver or excreted (FIG. 27).

**Experimental Example 8. Confirmation of liver toxicity of 4-(hydroxymethyl)phenyl benzoate dimer (ssPB) nanoparticles comprising fucoidan**

Since a high concentration of accumulation occurs in the tumor site or liver site, in order to evaluate liver toxicity, the ssPB nanoparticles comprising fucoidan were injected into normal mice through the tail vein at a concentration of 10 mg/kg or 20 mg/kg. After 5 injections once every 3 days, blood was collected and analyzed for alanine transaminase (ALT). As a result, there was a tendency to increase little by little, but all of the values were within the normal range, and the toxicity was not shown (FIG. 28).

**IV. Nanoparticles comprising 4-(hydroxymethyl)phenylboronic acid pinacol ester dimer compound**

**Example 10. Preparation of 4-(hydroxymethyl)phenylboronic acid pinacol ester dimer compound**

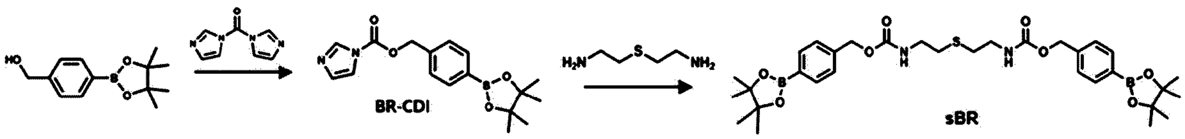

### Step 1: Synthesis of BR-CDI

4-(hydroxymethyl)phenylboronic acid pinacol ester (2.0 g) and 1,1'-carbonyldiimidazole (2.07 g) were dissolved in 25 mL of dichloromethane and then reacted for 2 hours at room temperature. The synthesized substance BR-CDI was obtained through a silica column using ethyl acetate/hexane (1:1).

### Step 2: Synthesis of sBR

2,2-thiobis(ethylamine) (1 g) and BR-CDI (5.46 g) were dissolved in 9 mL of dimethyl sulfoxide under a nitrogen environment. It was reacted at room temperature for 12 hours. The resulting sBR was separated through a silica column using ethyl acetate/hexane (1:1).

$^1$H NMR (400 MHz, DMSO-d6): δ 1.28 (24H, s), 2.58 (4H, t), 3.18 (4H, t), 5.09 (4H, s), 7.36 (4H, d), 7.41 (2H, t), 7.66 (4H, d).

### Example 11. Preparation of nanoparticles comprising 4-(hydroxymethyl)phenylboronic acid pinacol ester dimer (sBR) compound

1 mL of tetrahydrofuran in which 10 mg of sBR was dissolved was slowly added dropwise to distilled water while stirring rapidly. The mixture was sonicated for 1 minute and homogenized for 1 minute using a homogenizer. Tetrahydrofuran was removed using a rotary evaporator, a solution in which sBR was emulsified was centrifuged at a rate of 11,000xg for 4 minutes. The precipitated sBR nanoparticles were washed with water, and then freeze-dried to obtain the sBR nanoparticles.

### Example 12. Preparation of nanoparticles comprising 4-(hydroxymethyl)phenylboronic acid pinacol ester dimer (sBR) compound and fucoidan

1 mL of tetrahydrofuran in which 10 mg of sBR was dissolved was slowly added to 20 mL of PBS in which 1 mg of fucoidan was dissolved while stirring slowly. The mixture was sonicated for 1 minute and homogenized for 1 minute using a homogenizer. Tetrahydrofuran was removed using a rotary evaporator, and then a solution in which sBR was emulsified was centrifuged at a rate of 11,000xg for 4 minutes. The precipitated sBR nanoparticles were washed with water, and then freeze-dried to obtain the sBR nanoparticles.

The size and shape of the prepared nanoparticles were analyzed through a particle size analyzer and an electron microscope, and the surface potential of the nanoparticles was also compared and analyzed. It was determined that 10% of fucoidan showed the optimum concentration, and it was difficult to exhibit the properties of nanoparticles as the concentration of fucoidan increased. It was determined to be most appropriate when the concentration of fucoidan was 10 to 30% (FIGs. 32 to 36).

### Experimental Example 9. Evaluation of toxicity of 4-(hydroxymethyl)phenylboronic acid pinacol ester dimer compound nanoparticles comprising fucoidan

In order to evaluate liver toxicity, the sBR nanoparticles comprising fucoidan were intravascularly injected into normal mice at a concentration of 10 mg/kg. After 5 injections once every 3 days, blood was collected and analyzed for alanine transaminase (ALT). The liver, heart, lung, spleen, and kidney were excised, and the tissues were stained by H&E and analyzed for safety *in vivo* through histological analysis. As a result, it was confirmed that a significant toxicity was not shown (FIGs. 37 and 38).

### Example 13. Preparation of 4-(hydroxymethyl)phenylboronic acid pinacol ester dimer (ssBR) compound

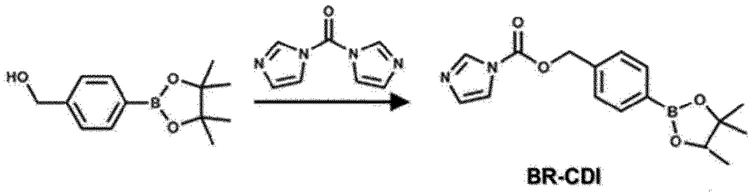

BR-CDI

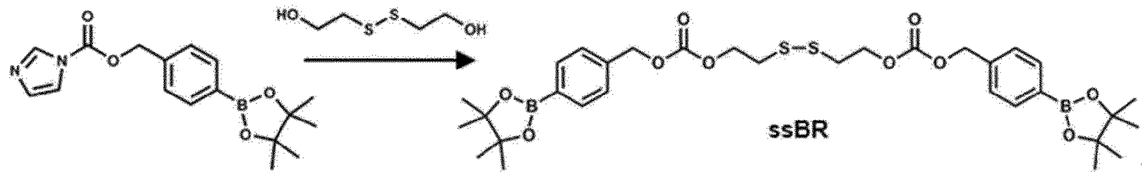

ssBR

### Step 1: Synthesis of BR-CDI

4-(hydroxymethyl) phenylboronic acid pinacol ester (2.0 g) and 1,1'-carbonyldiimidazole (2.07 g) were dissolved in 25 mL of dichloromethane and then reacted for 2 hours at room temperature. The synthesized substance BR-CDI was obtained through a silica column using ethyl acetate/hexane (1:1).

### Step 2: Synthesis of ssBR-CDI

Dithiodiethanol (1 g) and BR-CDI (4.26 g) were dissolved in 9 mL of dimethyl sulfoxide under a nitrogen environment. It was reacted at room temperature for 12 hours. The resulting sBR was separated through a silica column using ethyl acetate/hexane (1:1) (FIGs. 39 to 41).

$^1$H NMR (400 MHz, DMSO-d6): $\delta$ 1.28 (24H, s), 3.03 (4H, t), 4.44 (4H, t), 5.18 (4H, s), 7.38 (4H, d), 7.68 (4H, d).

### Example 14. Preparation of nanoparticles comprising 4-(hydroxymethyl)phenylboronic acid pinacol ester dimer (ssBR) compound

1 mL of tetrahydrofuran in which 10 mg of ssBR was dissolved was slowly added dropwise to distilled water while stirring rapidly. The mixture was sonicated for 1 minute and homogenized for 1 minute using a homogenizer. Tetrahydrofuran was removed using a rotary evaporator, and then a solution in which ssBR was emulsified was centrifuged at a rate of 11,000xg for 4 minutes, and the precipitated ssBR nanoparticles were washed with water, and then freeze-dried to obtain the ssBR nanoparticles.

### Example 15. Preparation of nanoparticles comprising 4-(hydroxymethyl)phenylboronic acid pinacol ester dimer (ssBR) compound and fucoidan

1 mL of tetrahydrofuran in which 10 mg of ssBR was dissolved was slowly added to 20 mL of PBS in which 1 mg of fucoidan was dissolved while stirring slowly. The mixture was sonicated for 1 minute and homogenized for 1 minute using a homogenizer. Tetrahydrofuran was removed using a rotary evaporator, and then a solution in which ssBR was emulsified was centrifuged at a rate of 11,000xg for 4 minutes, and the precipitated ssBR nanoparticles were washed with water, and then freeze-dried to obtain the ssBR nanoparticles. The size and distribution of the nanoparticles according to the content of fucoidan were evaluated through a particle size analyzer and a transmission electron microscope (FIGs. 42 and 43).

### Experimental Example 10. Confirmation of pharmacological effect of 4-(hydroxymethyl)phenylboronic acid pinacol ester dimer compound (ssBR) nanoparticles comprising fucoidan

The ssBR nanoparticles dispersed in PBS were added to the colorectal cancer SW620 cells. After 1 hour, the cells were separated, lysed on ice, and then centrifuged at a rate of 9,800xg. The supernatant was mixed with 50 $\mu$L of Ellman's reagent, and then the absorbance was measured at 405 nm to measure the concentration of GSH in cells (FIG. 45).

It was confirmed that GSH is easily reduced, which is the pharmacological activity effect of BR. The cytotoxicity of the ssBR nanoparticles was analyzed by the MTT (3-(4,5-dimethylthiazol-2-yl)-2,5-diphenyltetrazolium bromide) method. SW620 cells were treated with the ssBR nanoparticles. After 24 hours treatment, 100 $\mu$L of MTT solution was added. After 3 hours, 1 mL of DMSO was added to dissolve the crystal. After 10 minutes, the absorbance was measured at 570

nm to analyze the cell viability. It was confirmed that the cell death rate increased as the concentration of the nanoparticles increased (FIG. 46).

**Experimental Example 11. Confirmation of tumor targeting ability of 4-(hydroxymethyl)phenylboronic acid pinacol ester dimer compound (ssBR) nanoparticles comprising fucoidan**

The cancer targeting ability of each of the nanoparticles prepared in Examples 14 and 15 above was confirmed. Tumorigenesis was induced by injecting about $2\times10^6$ SW620 cells subcutaneously into the left leg of nude mice (average body weight of about 20 g, 8 weeks old). After about 10 days, when the size of the tumor was greater than 3x3 mm, each of the nanoparticles was intravascularly injected through the tail vein.

For the tumor target image, nanoparticles carrying a fluorescent substance IR780 were used, and the targeting of the tumor was confirmed by comparative analysis based on 24 hours. The ssPB nanoparticles to which fucoidan was added continued to accumulate into the tumor for up to 24 hours, but most of the nanoparticles to which fucoidan was not added were accumulated into the liver or excreted (FIG. 47).

**Experimental Example 12. Evaluation of toxicity of 4-(hydroxymethyl)phenylboronic acid pinacol ester dimer compound (ssBR) nanoparticles comprising fucoidan**

In order to evaluate liver toxicity, the ssBR nanoparticles to which fucoidan was added were intravascularly injected into normal mice at a concentration of 20 mg/kg. After 5 injections once every 3 days, blood was collected and analyzed for alanine transaminase (ALT). The liver, heart, lung, spleen, and kidney were excised, and the tissues were stained by H&E and analyzed for safety *in vivo* through histological analysis. As a result, it was confirmed that a significant toxicity was not shown (FIGs. 48 and 49).

**V. Nanoparticles comprising paclitaxel dimer compound**

**Example 16. Preparation of paclitaxel dimer (PTX-SS-PTX) compound**

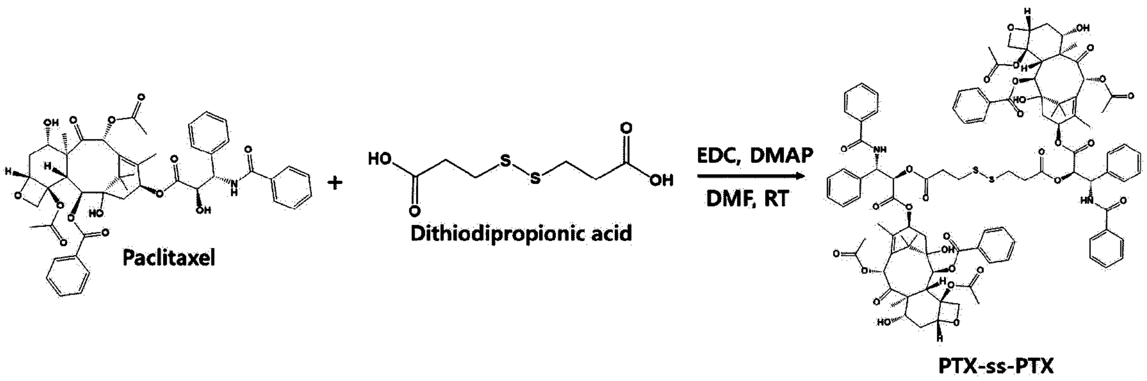


Paclitaxel (PTX) (2 g) and dithiodipropionic acid (0.25 g) were dissolved in 10 mL of dimethylformamide and then stirred for about 10 minutes. Thereafter, 1-ethyl-3-(dimethylaminophenyl) carbonyldiimide (0.90 g) and 4-dimethylaminopyridine (0.29 g) were added and further stirred for 16 hours. The reactivity was confirmed by TLC, and when all of the reaction proceeded, a solid produced by adding an excess of water was obtained by centrifugation. The obtained solid was dried, and then passed through ethyl acetate mobile phase column, and purified through recrystallization (FIG. 50).

$^1$H NMR (400 MHz, DMSO-d6): δ 1.10 (6H, s), 1.29 (2H, t), 1.58 (6H, s), 1.66 (6H, s), 1.68 (2H, d), 1.72 (6H, s), 2.00 (6H, s), 2.13 (2H, q), 2.28 (2H, q), 2.58 (6H, d), 2.72 (2H, t), 2.82 (4H, t) 3.46-3.72 (2H, dd), 3.93 (2H, d), 4.11 (4H, t), 4.32 (2H, m), 4.60-4.68 (4H, m), 5.04 (2H, t), 5.45 (2H, d), 5.83 (2H, m), 5.98 (2H, m), 6.89 (2H, s), 7.20 (2H, s), 7.28 (4H, t), 7.38 (4H, t), 7.42 (4H, m), 7.5-7.53 (4H, m), 7.60-7.65 (8H, m), 7.87 (4H, d).

**Example 17. Preparation of nanoparticles comprising paclitaxel dimer compound**

10 mg of PTX-SS-PTX was dissolved in 5 mL of THF, added dropwise to 30 mL of PBS using a syringe, and

subjected to ultrasonic irradiation to disperse the nanoparticles. Thereafter, THF was removed for 15 minutes under a condition of room temperature using a rotary evaporator. The solution from which THF was removed was frozen with liquid nitrogen and then freeze-dried. The completely dried nanoparticles were used by dispersing in PBS immediately before use.

**Example 18. Preparation of nanoparticles comprising paclitaxel dimer compound and fucoidan**

10 mg of PTX-SS-PTX was dissolved in 5 mL of THF, and 2 mg of fucoidan was dissolved in 30 mL of PBS. The PTX-SS-PTX solution was added dropwise to the fucoidan solution that was subjected to ultrasonic irradiation to form and disperse the nanoparticles. Thereafter, THF was removed for 15 minutes under a condition of room temperature using a rotary evaporator. The solution from which THF was removed was frozen with liquid nitrogen and then freeze-dried. The completely dried nanoparticles were used by dispersing in PBS immediately before use (FIG. 51).

**Experimental Example 13. Confirmation of cancer targeting ability of paclitaxel dimer nanoparticles comprising fucoidan**

The cancer targeting ability of each of the nanoparticles prepared in Examples 17 and 18 above was confirmed. Tumorigenesis was induced by injecting about $2\times10^6$ SW620 cells subcutaneously into the left leg of nude mice (average body weight of about 20 g, 8 weeks old). After about 10 days, when the size of the tumor was greater than 3x3 mm, each of the nanoparticles was intravascularly injected through the tail vein. For the tumor target image, nanoparticles carrying a fluorescent substance IR780 were used, and the targeting of the tumor was confirmed by comparative analysis based on 24 hours. It was confirmed that the ssPB nanoparticles to which fucoidan was added continued to accumulate into the tumor for up to 21 hours, but the nanoparticles to which fucoidan was not added were accumulated into the tumor in a small amount (FIG. 52).

**VI. Nanoparticles comprising doxorubicin dimer compound**

**Example 19. Preparation of doxorubicin dimer compound (DOX-SS-DOX)**

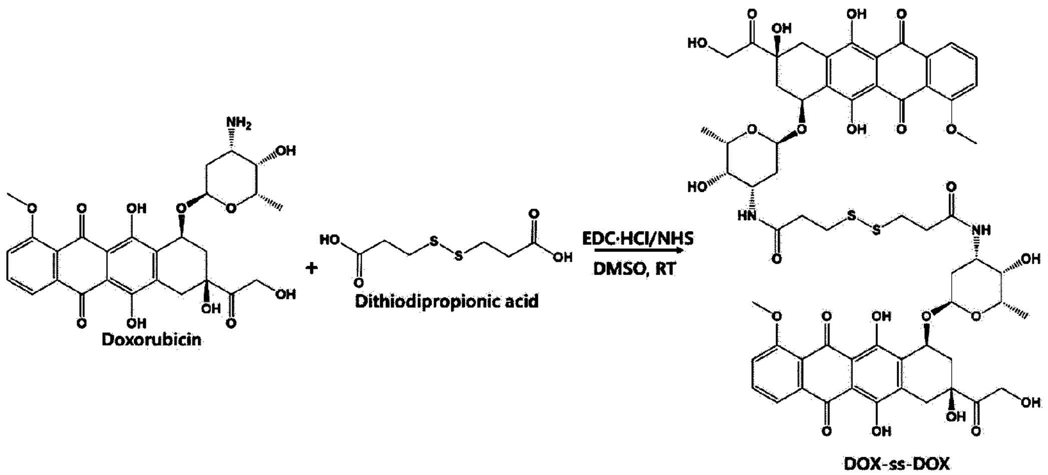


Doxorubicin (DOX) (1 g) and dithiodipropionic acid (0.157 g) were dissolved in 20 mL of dimethyl sulfoxide, and then triethylamine (0.24 μL) was added, and stirred for about 10 minutes while blocking the light. Thereafter, 1-ethyl-3-(dimethylaminophenyl) carbonyldiimide (0.5 g) and N-hydroxysuccinamide (0.198 g) were added and stirred for 16 hours while blocking the light. The reactivity was confirmed by TLC, and when all of the reaction proceeded, a solid produced by adding an excess of water was obtained by centrifugation. The obtained solid was dried, and then passed through ethyl acetate mobile phase column, and purified through recrystallization (FIG. 53).

$^1$H NMR (400 MHz, DMSO-d6): δ 1.12 (6H, s), 1.43 (2H, s), 1.84 (2H, t), 2.16 (2H, s), 2.23 (2H, s), 2.58 (4H, s), 2.89 (4H, d), 3.39 (6H, s), 3.89-3.95 (8H, m), 4.13 (2H, s), 4.53 (4H, s), 4.67 (4H, d) 4.70 (2H, s), 4.81 (2H, s), 5.21 (2H, s), 7.56 (2H, t), 7.65 (2H, d), 7.73 (2H, d), 7.83 (2H, d), 13.14 (2H, s), 13.87 (2H, s).

**Example 20. Preparation of nanoparticles comprising doxorubicin dimer (DOX-SS-DOX) compound**

10 mg of DOX-SS-DOX was dissolved in 5 mL of THF and added dropwise to distilled water that was subjected to ultrasonic irradiation to form and then disperse the nanoparticles. Thereafter, THF was removed for 15 minutes under a condition of room temperature using a rotary evaporator. The solution from which THF was removed was frozen with liquid nitrogen and then freeze-dried. The completely dried nanoparticles were used by dispersing in PBS immediately before use (FIG. 54).

**Example 21. Preparation of nanoparticles comprising doxorubicin dimer (DOX-SS-DOX) compound and fucoidan**

10 mg of DOX-SS-DOX was dissolved in 5 mL of THF, and 2 mg of fucoidan was dissolved in 30 mL of PBS. The DOX-SS-DOX solution was added dropwise to the fucoidan solution that was subjected to ultrasonic irradiation to form and then disperse the nanoparticles. Thereafter, THF was removed for 15 minutes under a condition of room temperature using a rotary evaporator. The solution from which THF was removed was frozen with liquid nitrogen and then freeze-dried. The completely dried nanoparticles were used by dispersing in PBS immediately before use. As a result of comparative analysis by a particle size analyzer and a scanning electron microscope, the nanoparticles comprising fucoidan showed excellent physical properties in terms of the size, shape, and distribution compared to the nanoparticles without fucoidan (FIG. 54).

**Experimental Example 14. Confirmation of toxicity of doxorubicin dimer nanoparticles comprising fucoidan**

An in vivo experiment was performed to confirm the toxicity of the doxorubicin dimer nanoparticles comprising fucoidan. The experiment schedule is as shown in FIG. 57. NPG mice (female, 6 weeks old) were used. Specifically, the A549 cell line was mixed 1:1 with a Matrigel matrix and then mixed so as not to generate bubbles. Thereafter, the A549 cells ($1 \times 10^6$ cells) were injected subcutaneously in the right flank of NPG immune-deficiency mice. When the tumor volume reached about 70 $mm^3$, the mice were classified into groups having a similar tumor size, and on days 3, 4 and 5, fucoidan doxorubicin was administered intravenously at a concentration of 3 mg/kg.

Specifically, the doxorubicin dimer nanoparticles comprising doxorubicin and fucoidan (hereinafter, ROD-101) were administered to the caudal vein once a day for 3 days, respectively. The drug was administered a total of three times. The tumor volume and the mouse body weight were measured on the day before drug administration, and then group separation was performed (first measurement). After the drug was administered a total of three times, on the next day, the tumor volume and the mouse body weight were measured (second measurement). After 4 days, the tumor volume and the mouse body weight were measured (third measurement).

On the other hand, as a control in this experiment, PBS-treated group (G5) and doxorubicin-treated group (G6) were used. Thereafter, the growth of the tumor was monitored every day by measuring the vertical diameter of the tumor with a caliper, and the change in viability over time was monitored to evaluate the anticancer effect using an animal model. On the other hand, the tumor volume was calculated by the following formula:

**[Equation 1]**

$$\text{volume} = 0.523L\,(W)^2$$

in which, L denotes for length, and W denotes for width.

As a result, it was confirmed that the anticancer effect of the doxorubicin dimer nanoparticles comprising fucoidan was superior to that of doxorubicin (FIG. 59). In particular, it was confirmed that the body weight of mice in the group treated with the doxorubicin dimer nanoparticles comprising fucoidan did not decrease compared to doxorubicin (FIG. 60). Therefore, it was confirmed that the stability of the drug was increased when the doxorubicin dimer nanoparticles comprising fucoidan were used.

**VII. Nanoparticles comprising camptothecin dimer compound**

**Example 22. Preparation of camptothecin dimer (CPT-SS-CPT) compound**

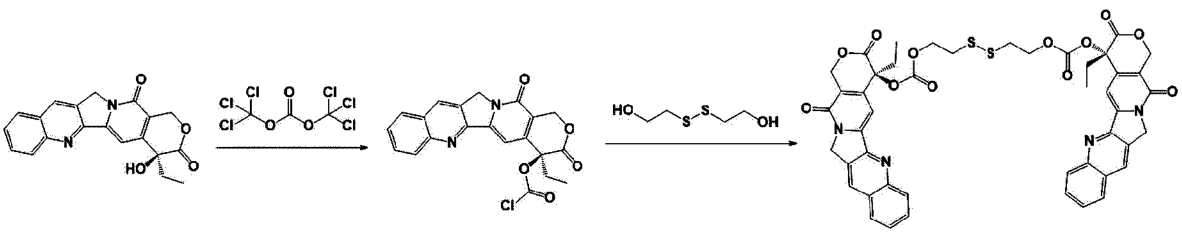

Camptothecin (CPT) (1 g) and 4-dimethylaminopyridine (0.35 g) were dispersed and dissolved in 50 mL of dichloromethane, and then stirred at a temperature of 4°C or less using an ice water bath. Triphosgene (0.34 g) was added to change a solution state. Thereafter, the reaction was maintained while stirring for 16 hours at a temperature of 4°C or less. Dithiodiethanol (0.18 g) was dissolved in a THF solution and then added dropwise to the reaction solution. The reactivity was confirmed by TLC, and when all of the reaction proceeded, it was dried and then purified through a column (FIG. 55).

$^1H$ NMR (400 MHz, DMSO-d6): δ 0.77 (6H, t), 2.14 (4H, q), 2.92 (4H, t), 4.17 (4H, t), 5.16 (4H, s), 5.46 (4H, s), 7.00 (2H, s), 7.66 (2H, t), 7.77 (2H, t), 8.06 (4H, dd), 8.61 (2H, s).

**Example 23. Preparation of nanoparticles comprising camptothecin dimer (CPT-SS-CPT) compound**

10 mg of CPT-SS-CPT was dissolved in 5 mL of THF:MeOH (10:1) and added dropwise to distilled water that was subjected to ultrasonic irradiation to form and disperse the nanoparticles. Thereafter, THF and MeOH were removed for 15 minutes under a condition of room temperature using a rotary evaporator. The solution from which the organic solvents were removed was frozen with liquid nitrogen and then freeze-dried. The completely dried nanoparticles were used by dispersing in PBS immediately before use.

**Example 24. Preparation of nanoparticles comprising camptothecin dimer (CPT-SS-CPT) compound and fucoidan**

10 mg of CPT-SS-CPT was dissolved in 5 mL of THF:MeOH (10:1), and 2 mg of fucoidan was dissolved in 30 mL of PBS. The CPT-SS-CPT solution was added dropwise to the fucoidan solution that was subjected to ultrasonic irradiation to form and then disperse the nanoparticles. Thereafter, THF and MeOH were removed for 15 minutes under a condition of room temperature using a rotary evaporator. The solution from which the organic solvents were removed was frozen with liquid nitrogen and then freeze-dried. The completely dried nanoparticles were used by dispersing in PBS immediately before use. As a result of comparative analysis by a particle size analyzer and a scanning electron microscope, it was shown that the size, shape, and distribution of the nanoparticles comprising fucoidan were improved (FIG. 56).

## Claims

1. A retinoid dimer compound represented by formula 1a below, a solvate or a pharmaceutically acceptable salt thereof:

[Formula 1a]

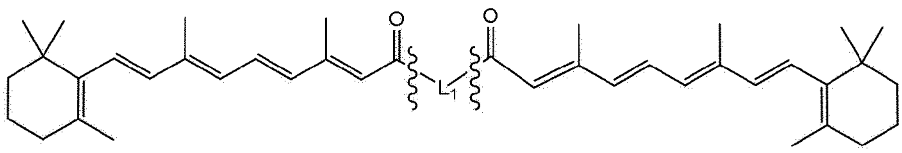

in formula 1a above,
$L_1$ is

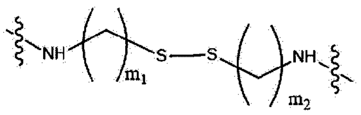 or 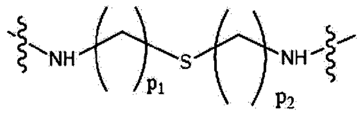 ;

and

$m_1$, $m_2$, $p_1$ and $p_2$ are each an integer of 1 to 10.

2. The retinoid dimer compound, solvate or pharmaceutically acceptable salt thereof according to claim 1, wherein the dimer compound is one in which any one or two monomers of the compounds represented by formulas 1b to 1d below are bound to each other:

[Formula 1b]

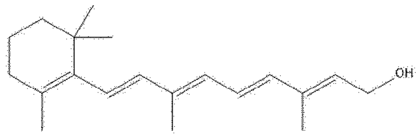

;

[Formula 1c]

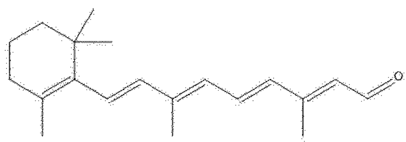

;

and

[Formula 1d]

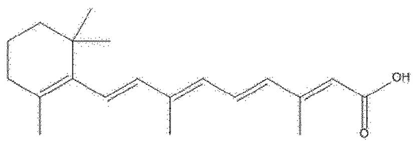

.

3. The retinoid dimer compound, solvate or pharmaceutically acceptable salt thereof according to claim 1, wherein the dimer compound is represented by formula 1e below:

[Formula 1e]

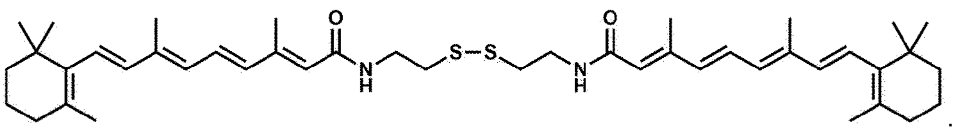

.

4. A nanoparticle comprising the retinoid dimer compound, solvate or pharmaceutically acceptable salt thereof according to any one of claims 1 to 3.

5. The nanoparticle according to claim 4, wherein the nanoparticle further comprises fucoidan.

6. The nanoparticle according to claim 5, wherein the size of the nanoparticle ranges from 200 nm to 300 nm.

7. The nanoparticle according to claim 5, wherein the fucoidan is comprised in an amount of 10 to 30 parts by weight based on 100 parts by weight of the dimer compound.

8. A pharmaceutical composition comprising the nanoparticles according to claim 5.

9. The pharmaceutical composition according to claim 8, wherein the pharmaceutical composition is for the prevention or treatment of cancer or skin disease.

10. A ursodeoxycholic acid dimer compound represented by formula 2a below, a solvate or a pharmaceutically acceptable salt thereof:

[Formula 2a]

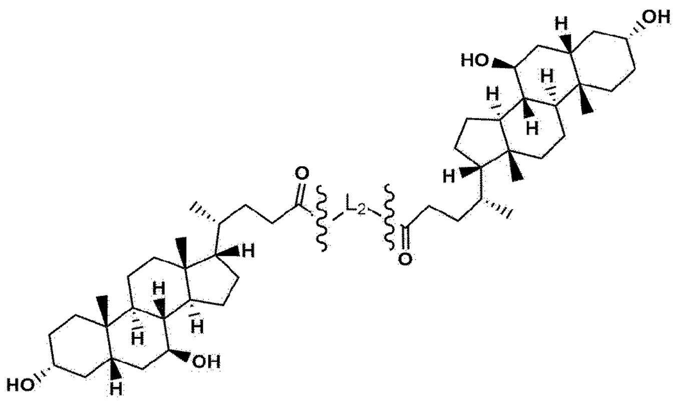

in formula 2a above,
$L_2$ is

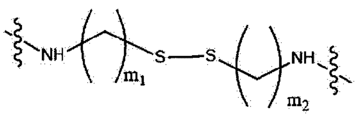

or

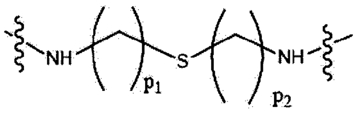

;

and
$m_1$, $m_2$, $p_1$ and $p_2$ are each an integer of 1 to 10.

11. The ursodeoxycholic acid dimer compound, solvate or pharmaceutically acceptable salt thereof according to claim 10, wherein the dimer compound is one in which a compound represented by formula 2b below is bound to each other:

[Formula 2b]

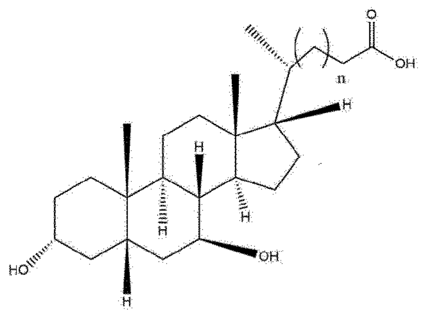

in formula 2b above,
n is each an integer of 1 to 10.

12. The ursodeoxycholic acid dimer compound, solvate or pharmaceutically acceptable salt thereof according to claim 10, wherein the dimer compound is represented by formula 2c below:

[Formula 2c]

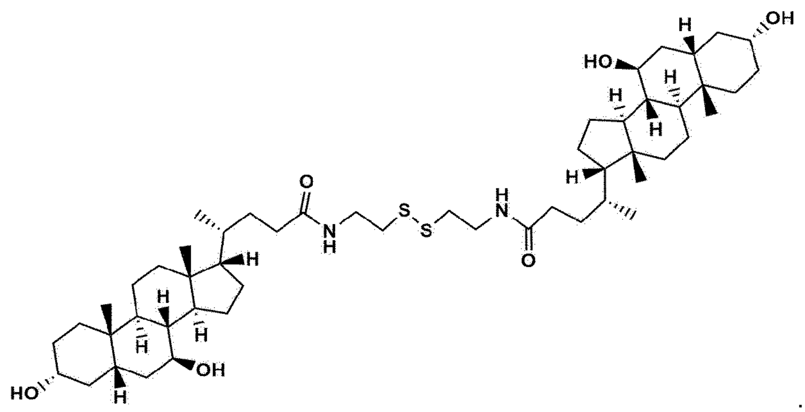

.

13. A nanoparticle comprising the ursodeoxycholic acid dimer compound, solvate or pharmaceutically acceptable salt thereof according to any one of claims 10 to 12.

14. The nanoparticle according to claim 13, wherein the nanoparticle further comprises fucoidan.

15. The nanoparticle according to claim 14, wherein the size of the nanoparticle ranges from 200 nm to 500 nm.

16. The nanoparticle according to claim 14, wherein the fucoidan is comprised in an amount of 10 to 30 parts by weight based on 100 parts by weight of the dimer compound.

17. A pharmaceutical composition comprising the nanoparticles according to claim 14.

18. The pharmaceutical composition according to claim 17, wherein the pharmaceutical composition is for the prevention or treatment of cancer, liver disease, or cardiovascular disease.

19. A 4-(hydroxymethyl)phenyl benzoate dimer compound represented by formula 3a below, a solvate or a pharmaceutically acceptable salt thereof:

[Formula 3a]

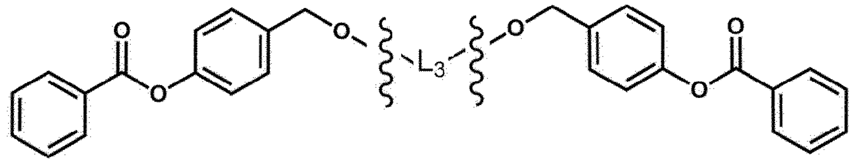

in formula 3a above,
$L_3$ is

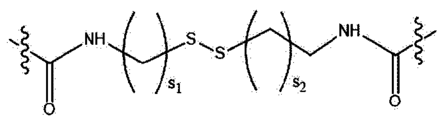

or

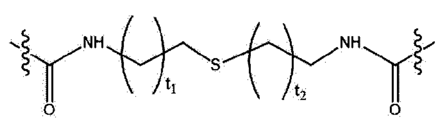

;

and
$s_1$, $s_2$, $t_1$ and $t_2$ are each an integer of 1 to 10.

20. The 4-(hydroxymethyl)phenyl benzoate dimer compound, solvate or pharmaceutically acceptable salt thereof according to claim 19, wherein the dimer compound is one in which a compound represented by formula 3b below is

bound to each other:

[Formula 3b]

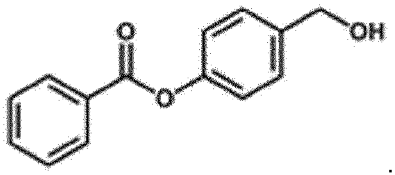

.

21. The 4-(hydroxymethyl)phenyl benzoate dimer compound, solvate or pharmaceutically acceptable salt thereof according to claim 19, wherein the dimer compound is represented by formula 3c below:

[Formula 3c]

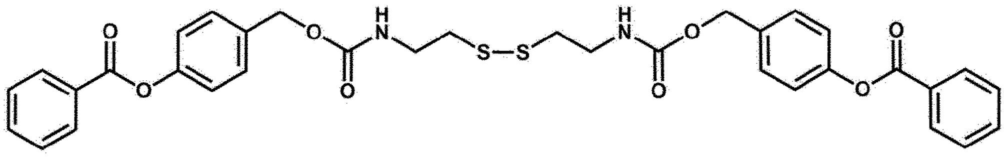

.

22. A nanoparticle comprising the 4-(hydroxymethyl)phenyl benzoate dimer compound, solvate or pharmaceutically acceptable salt thereof according to any one of claims 19 to 21.

23. The nanoparticle according to claim 22, wherein the nanoparticle further comprises fucoidan.

24. The nanoparticle according to claim 23, wherein the size of the nanoparticle ranges from 100 nm to 500 nm.

25. The nanoparticle according to claim 23, wherein the fucoidan is comprised in an amount of 10 to 30 parts by weight based on 100 parts by weight of the dimer compound.

26. A pharmaceutical composition comprising the nanoparticles according to claim 23.

27. The pharmaceutical composition according to claim 26, wherein the pharmaceutical composition is for the prevention or treatment of cancer.

28. A 4-(hydroxymethyl)phenylboronic acid pinacol ester dimer compound represented by formula 4a below, a solvate or a pharmaceutically acceptable salt thereof:

[Formula 4a]

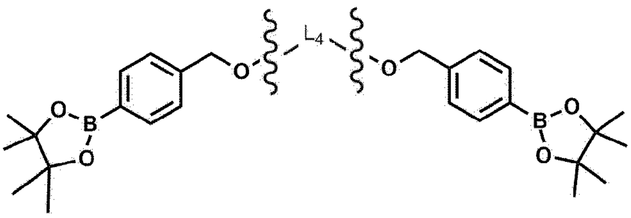

in formula 4a above,
$L_4$ is any one selected from the group consisting of

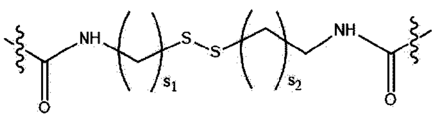

,

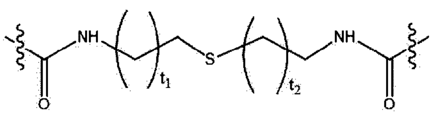

,

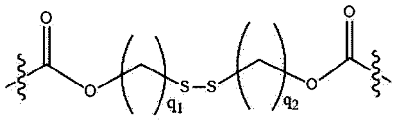

and

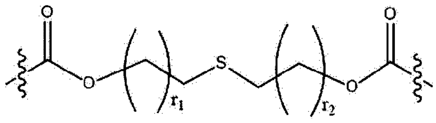

; and

$s_1$, $s_2$, $t_1$, $t_2$, $q_1$, $q_2$, $r_1$ and $r_2$ are each an integer of 1 to 10.

**29.** The 4-(hydroxymethyl)phenylboronic acid pinacol ester dimer compound, solvate or pharmaceutically acceptable salt thereof according to claim 28, wherein the dimer compound is one in which a compound represented by formula 4b below is bound to each other:

[Formula 4b]

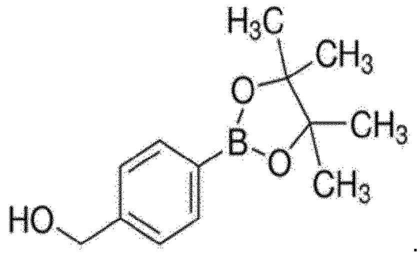

.

**30.** The 4-(hydroxymethyl)phenylboronic acid pinacol ester dimer compound, solvate or pharmaceutically acceptable salt thereof according to claim 28, wherein the dimer compound is represented by formula 4c or formula 4d below:

[Formula 4c]

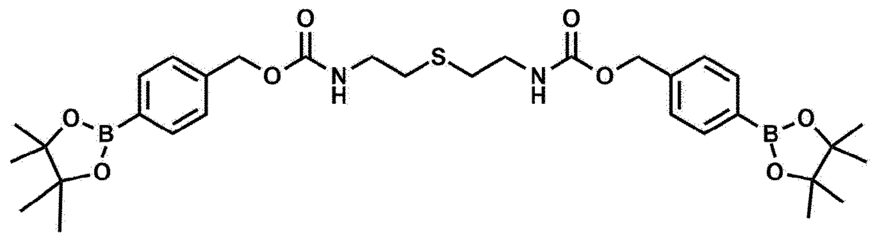

; or

[Formula 4d]

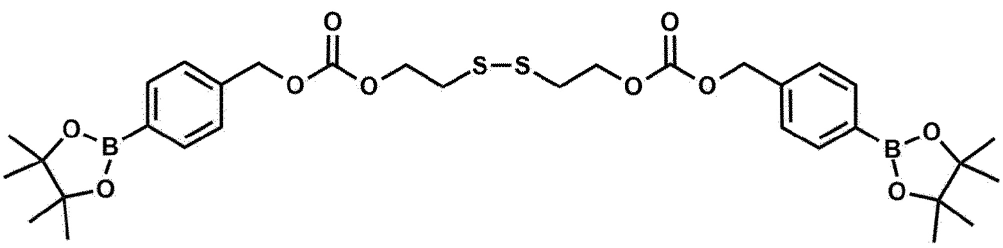

.

**31.** A nanoparticle comprising the 4-(hydroxymethyl)phenylboronic acid pinacol ester dimer compound, solvate or pharmaceutically acceptable salt thereof according to any one of claims 28 to 30.

**32.** The nanoparticle according to claim 31, wherein the nanoparticle further comprises fucoidan.

**33.** The nanoparticle according to claim 32, wherein the size of the nanoparticle ranges from 200 nm to 500 nm.

**34.** The nanoparticle according to claim 32, wherein the fucoidan is comprised in an amount of 10 to 30 parts by weight based on 100 parts by weight of the dimer compound.

**35.** A pharmaceutical composition comprising the nanoparticles according to claim 32.

**36.** The pharmaceutical composition according to claim 35, wherein the pharmaceutical composition is for the prevention or treatment of cancer.

**37.** A paclitaxel dimer compound represented by formula 5a below, a solvate or a pharmaceutically acceptable salt thereof:

[Formula 5a]

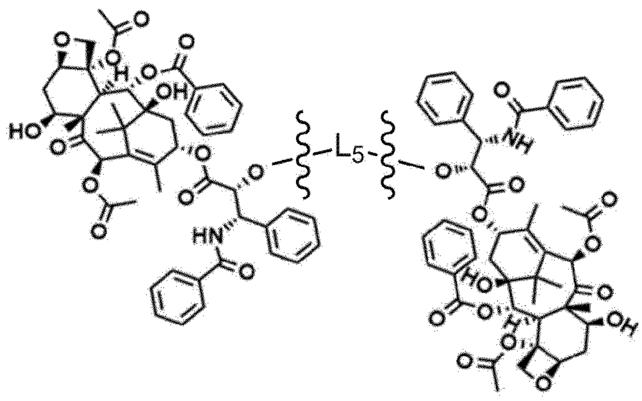

in formula 5a above,
$L_5$ is

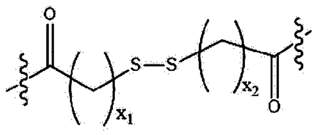
or
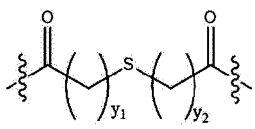
;

and
$x_1$, $x_2$, $y_1$ and $y_2$ are each an integer of 1 to 10.

**38.** The paclitaxel dimer compound, solvate or pharmaceutically acceptable salt thereof according to claim 37, wherein the dimer compound is one in which a compound represented by formula 5b below is bound to each other:

[Formula 5b]

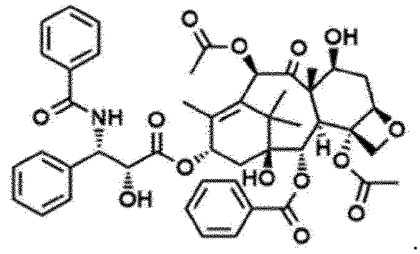

39. The paclitaxel dimer compound, solvate or pharmaceutically acceptable salt thereof according to claim 37, wherein the dimer compound is represented by formula 5c below:

[Formula 5c]

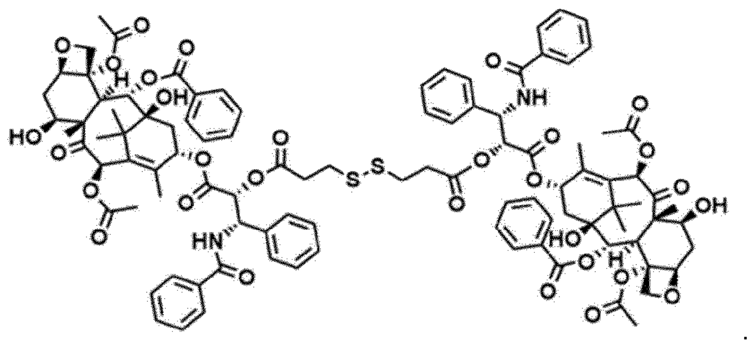

40. A nanoparticle comprising the paclitaxel dimer compound according to any one of claims 37 to 39.

41. The nanoparticle according to claim 40, wherein the nanoparticle further comprises fucoidan.

42. The nanoparticle according to claim 41, wherein the size of the nanoparticle ranges from 100 nm to 300 nm.

43. The nanoparticle according to claim 41, wherein the fucoidan is comprised in an amount of 10 to 30 parts by weight based on 100 parts by weight of the dimer compound.

44. A pharmaceutical composition comprising the nanoparticles according to claim 41.

45. The pharmaceutical composition according to claim 44, wherein the pharmaceutical composition is for the prevention or treatment of cancer.

46. A doxorubicin dimer compound represented by formula 6a below, a solvate or a pharmaceutically acceptable salt thereof:

[Formula 6a]

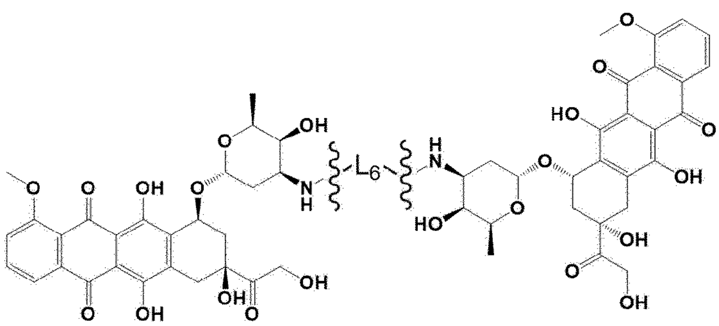

in formula 6a above,
$L_6$ is

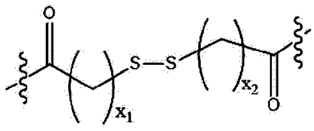

or

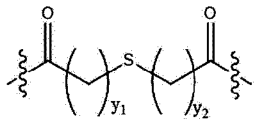

;

and
$x_1$, $x_2$, $y_1$ and $y_2$ are each an integer of 1 to 10.

**47.** The doxorubicin dimer compound, solvate or pharmaceutically acceptable salt thereof according to claim 46, wherein the dimer compound is one in which a compound represented by formula 6b below is bound to each other:

[Formula 6b]

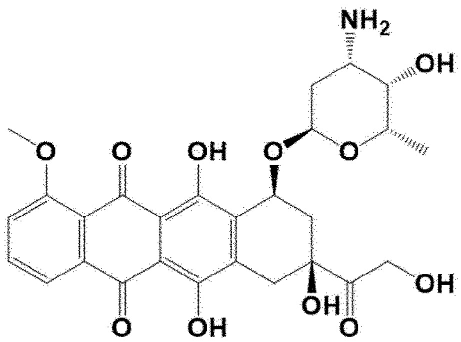

.

**48.** The doxorubicin dimer compound, solvate or pharmaceutically acceptable salt thereof according to claim 46, wherein the dimer compound is represented by formula 6c below:

[Formula 6c]

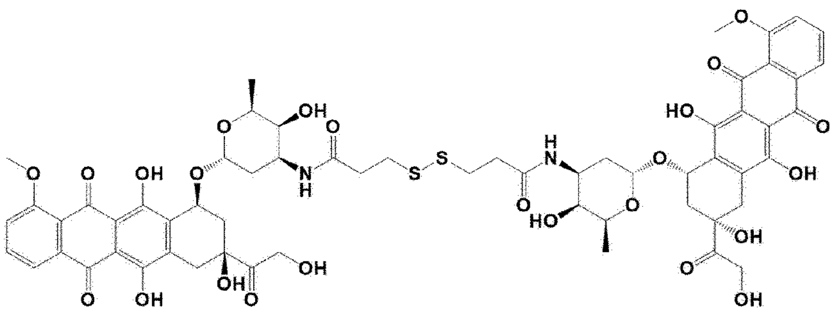

.

**49.** A nanoparticle comprising the doxorubicin dimer compound, solvate or pharmaceutically acceptable salt thereof according to any one of claims 46 to 48.

**50.** The nanoparticle according to claim 49, wherein the nanoparticle further comprises fucoidan.

**51.** The nanoparticle according to claim 50, wherein the size of the nanoparticle ranges from 100 nm to 200 nm.

**52.** The nanoparticle according to claim 50, wherein the fucoidan is comprised in an amount of 10 to 30 parts by weight based on 100 parts by weight of the dimer compound.

**53.** A pharmaceutical composition comprising the nanoparticles according to claim 50.

**54.** The pharmaceutical composition according to claim 53, wherein the pharmaceutical composition is for the prevention or treatment of cancer.

**55.** A camptothecin dimer compound represented by formula 7a below, a solvate or a pharmaceutically acceptable salt thereof:

[Formula 7a]

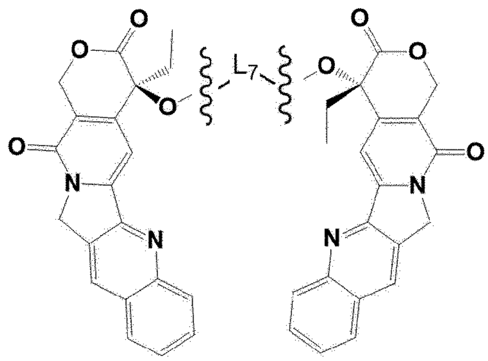

in formula 7a above,
$L_7$ is

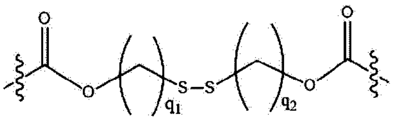
or
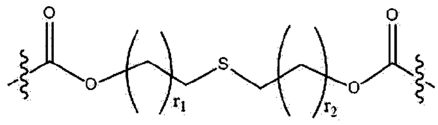
;

and
$q_1$, $q_2$, $r_1$ and $r_2$ are each an integer of 1 to 10.

**56.** The camptothecin dimer compound, solvate or pharmaceutically acceptable salt thereof according to claim 55, wherein the dimer compound is one in which a compound represented by formula 7b below is bound to each other:

[Formula 7b]

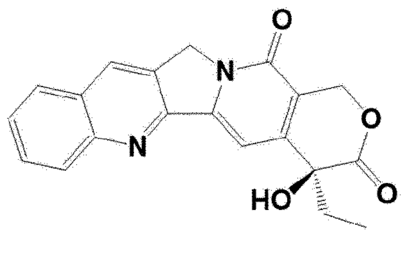
.

**57.** The camptothecin dimer compound according to claim 55, wherein the dimer compound is represented by formula 7c below:

[Formula 7c]

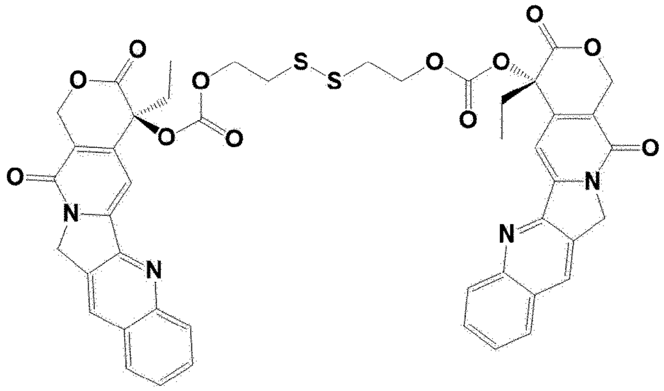

.

**58.** A nanoparticle comprising the camptothecin dimer compound, solvate or pharmaceutically acceptable salt thereof according to any one of claims 55 to 57.

**59.** The nanoparticle according to claim 58, wherein the nanoparticle further comprises fucoidan.

**60.** The nanoparticle according to claim 59, wherein the size of the nanoparticle ranges from 100 nm to 300 nm.

**61.** The nanoparticle according to claim 59, wherein the fucoidan is comprised in an amount of 10 to 30 parts by weight based on 100 parts by weight of the dimer compound.

**62.** A pharmaceutical composition comprising the nanoparticles according to claim 59.

**63.** The pharmaceutical composition according to claim 59, wherein the pharmaceutical composition is for the prevention or treatment of cancer.

**64.** A nanoparticle comprising a dimer compound consisting of structural formula (I) below:

A-L-A (I)

in structural formula (I) above,

A is any one selected from the group consisting of retinoid, ursodeoxycholic acid, 4-(hydroxymethyl)phenyl benzoate, 4-(hydroxymethyl)phenylboronic acid pinacol ester, paclitaxel, doxorubicin, and camptothecin; and
L is a linker of $C_{2-10}$ and comprises -S-, -SS-, -SSS-, -SeSe-, or -Se-.

**65.** The nanoparticle according to claim 64, wherein
L is any one selected from the group consisting of

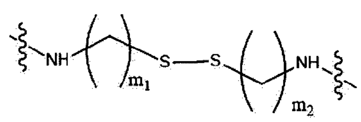

,

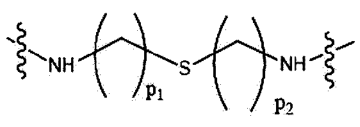

,

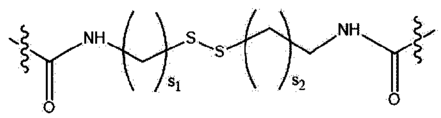

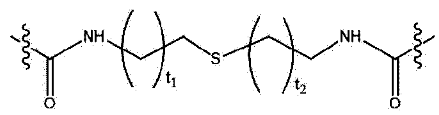,

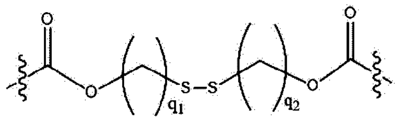

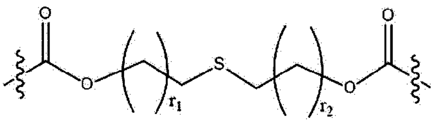,

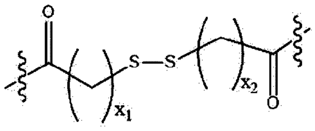 and

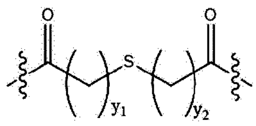

and

$m_1$, $m_2$, $p_1$, $p_2$, $s_1$, $s_2$, $t_1$, $t_2$, $q_1$, $q_2$, $r_1$, $r_2$, $x_1$, $x_2$, $y_1$ and $y_2$ are each an integer of 1 to 10.

**66.** The nanoparticle according to claim 64, wherein the nanoparticle further comprises fucoidan.

**67.** The nanoparticle according to claim 66, wherein the fucoidan is comprised in an amount of 10 to 30 parts by weight based on 100 parts by weight of the drug dimer compound.

**68.** A pharmaceutical composition comprising the nanoparticles according to claim 66.

**69.** The pharmaceutical composition according to claim 68, wherein the pharmaceutical composition is for the prevention or treatment of a disease selected from the group consisting of cancer, skin disease, liver disease, and cardiovascular disease.

**70.** A method for treating cancer, skin disease, liver disease, or cardiovascular disease, comprising: administering a pharmaceutical composition comprising the nanoparticles according to claim 66 to a subject suffering from cancer, skin disease, liver disease, or cardiovascular disease.

FIG. 1

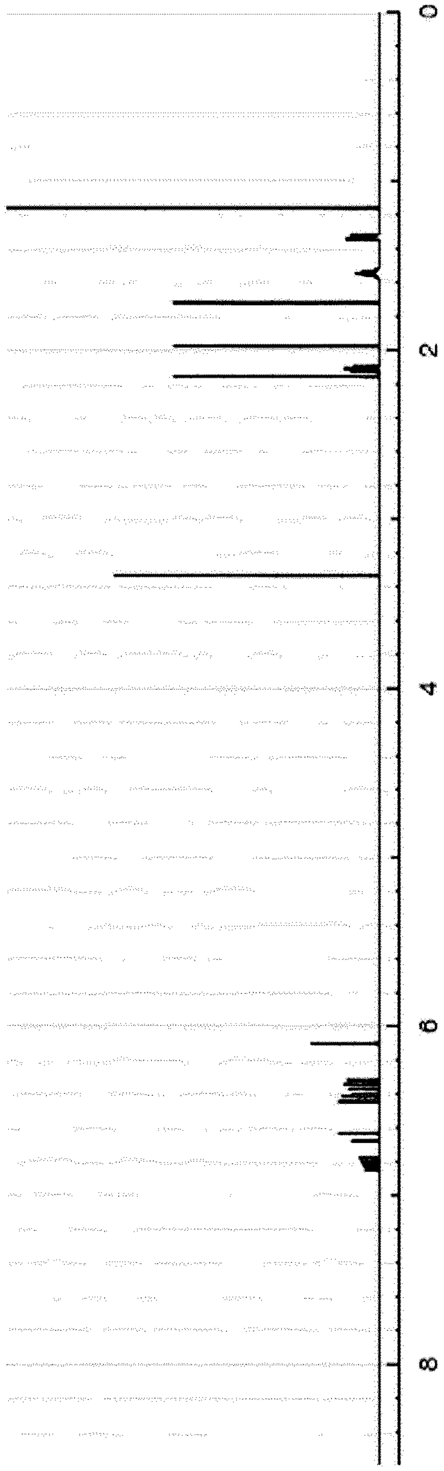


FIG. 2

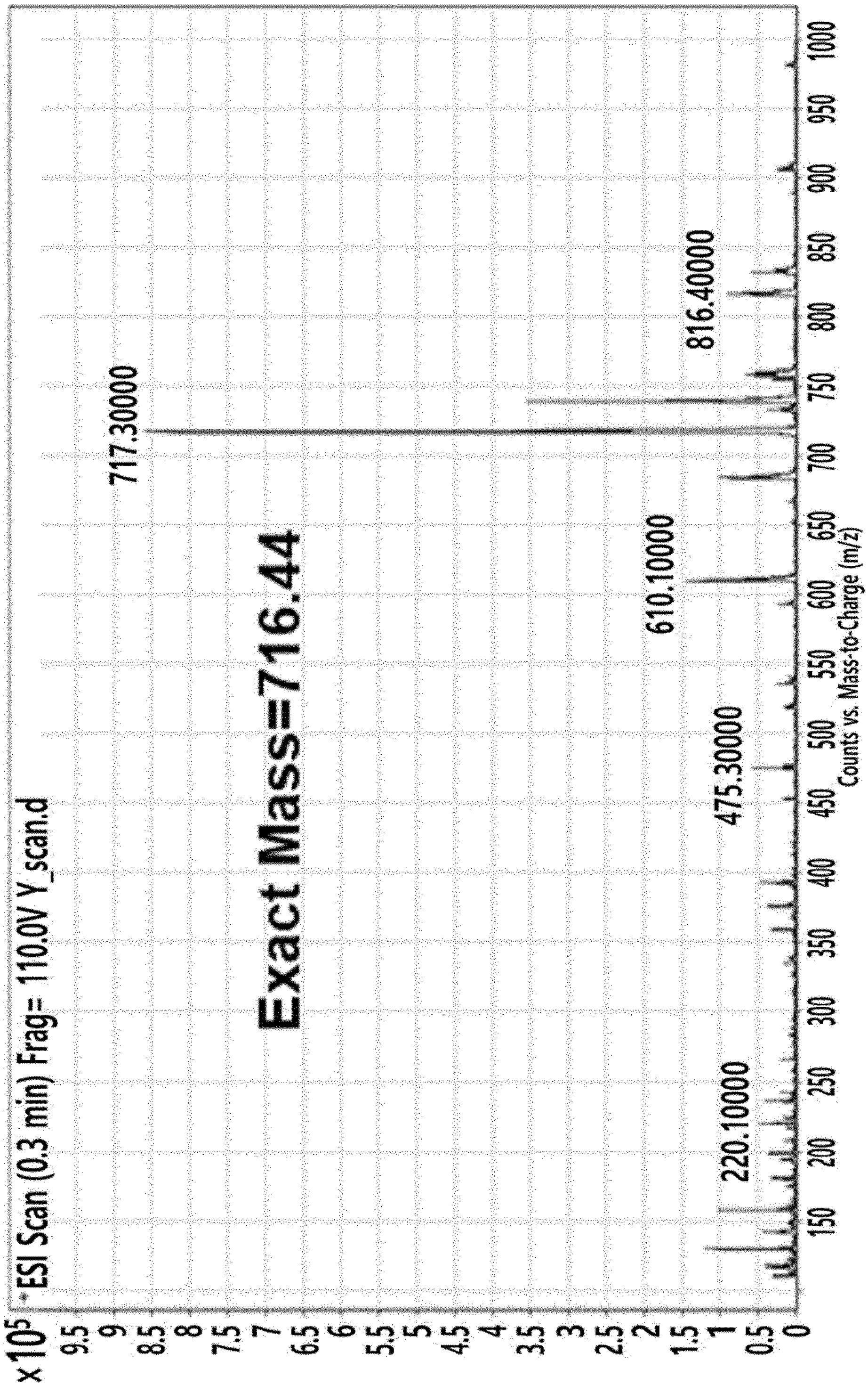
x10^5 +ESI Scan (0.3 min) Frag= 110.0V Y_scan.d
Exact Mass=716.44
717.30000
816.40000
610.10000
475.30000
220.10000
Counts vs. Mass-to-Charge (m/z)

FIG. 3

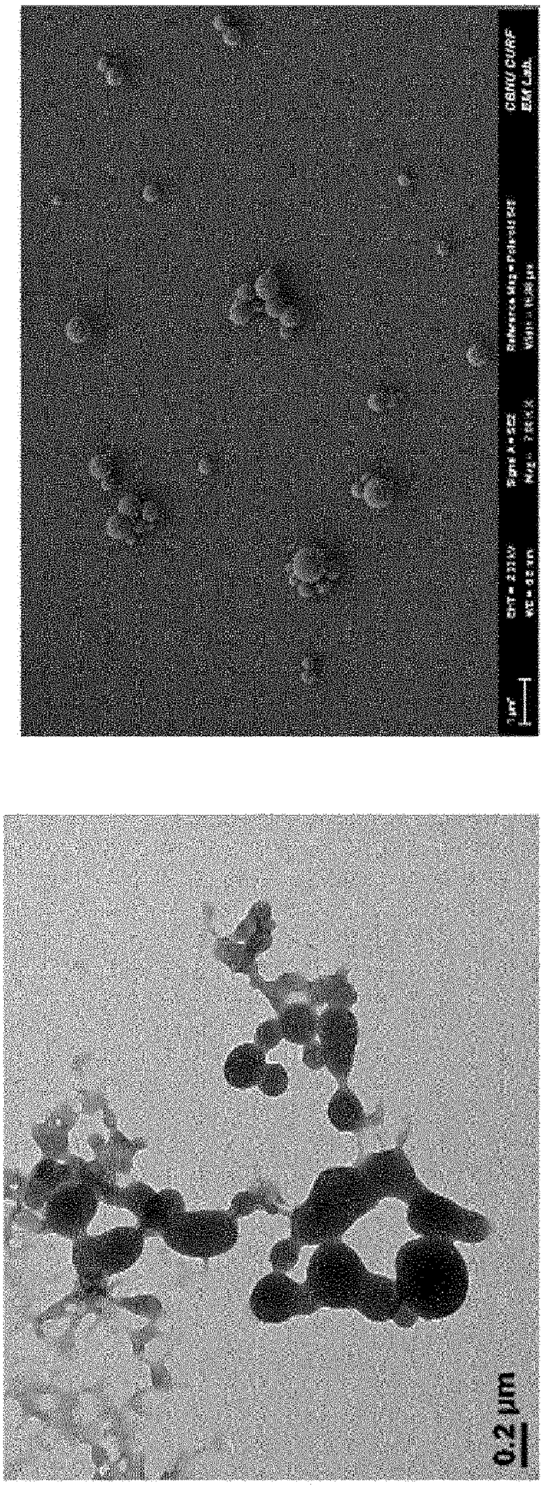

FIG. 4

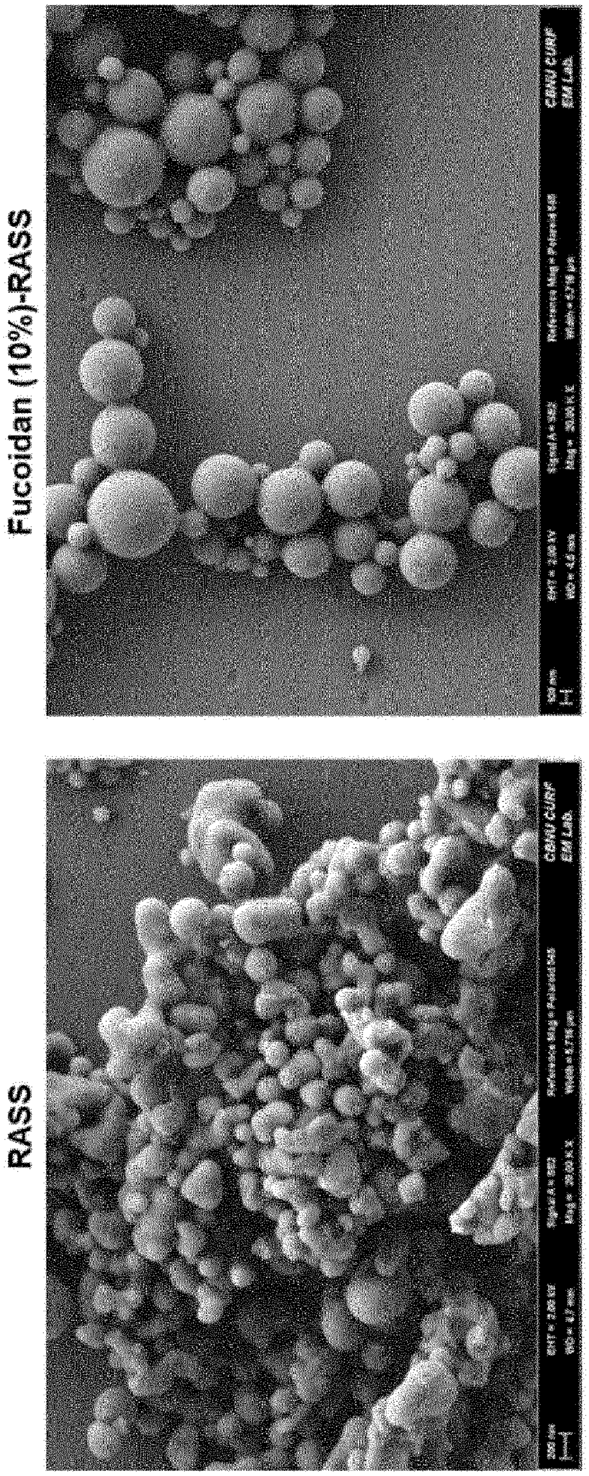

FIG. 5

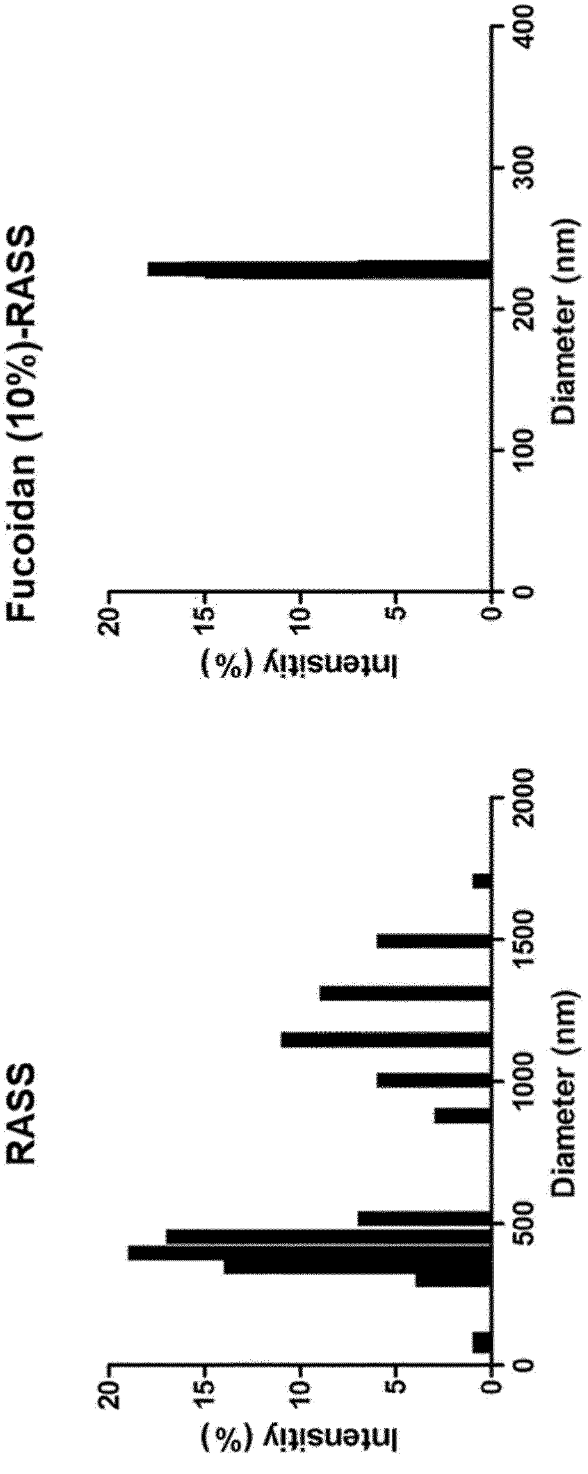
Fucoidan (10%)-RASS
Intensitiy (%)
Diameter (nm)
RASS
Intensitiy (%)
Diameter (nm)

FIG. 6

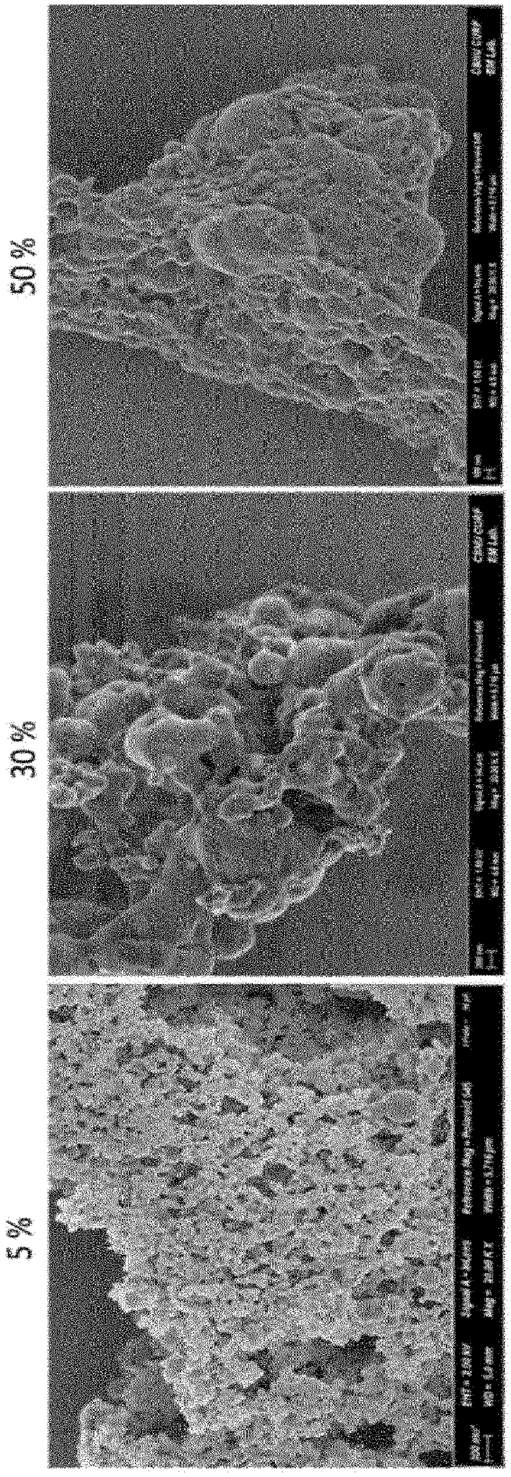

FIG. 7

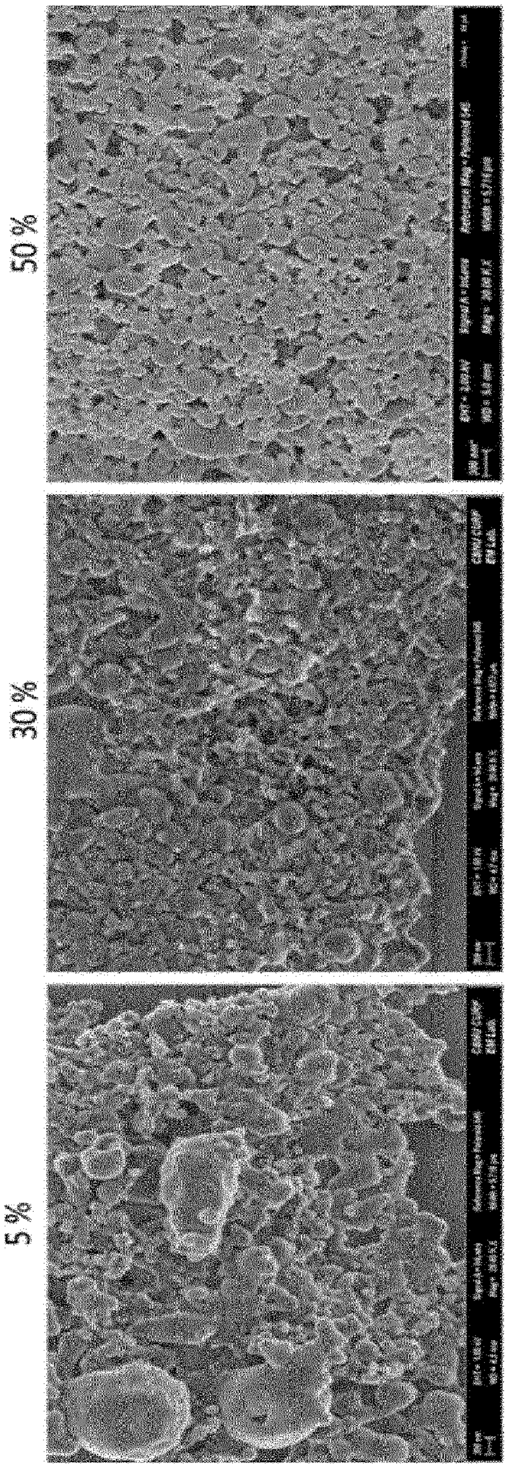

FIG. 8

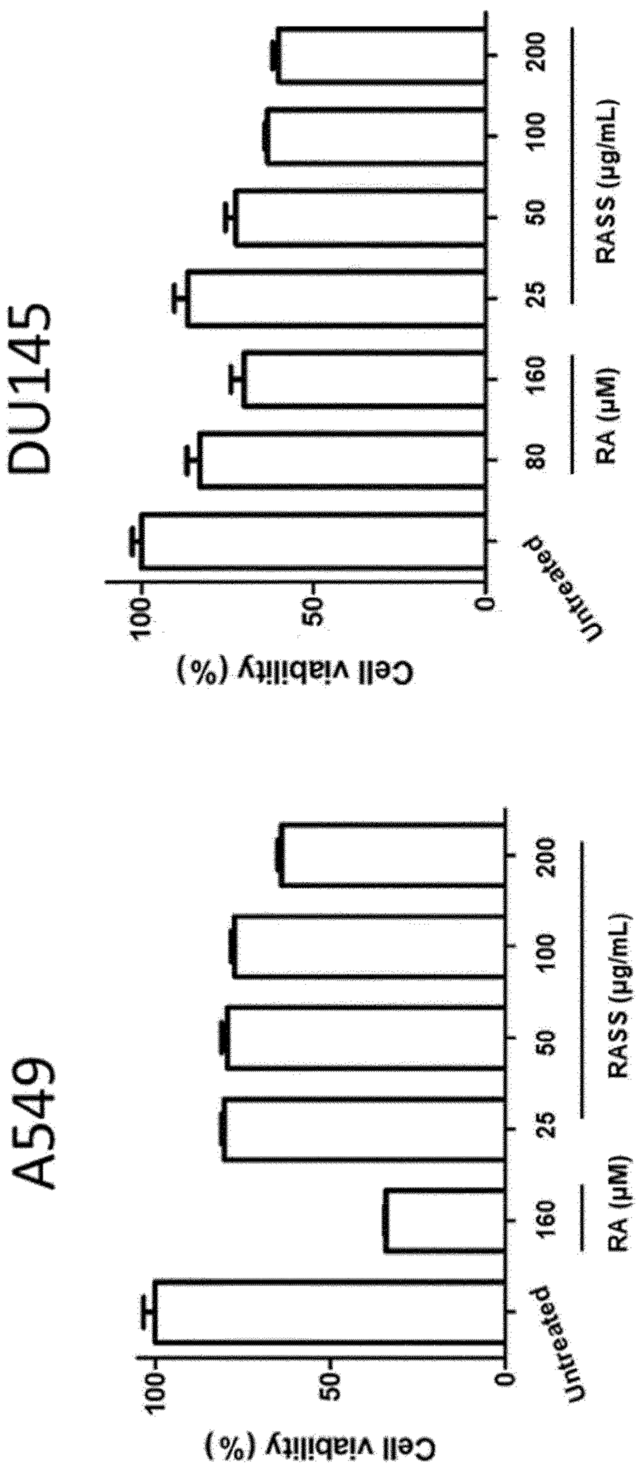
DU145
Cell viability (%)
100
50
0
Untreated
80
160
RA (μM)
25
50
100
200
RASS (μg/mL)
A549
Cell viability (%)
100
50
0
Untreated
160
RA (μM)
25
50
100
200
RASS (μg/mL)

FIG. 9

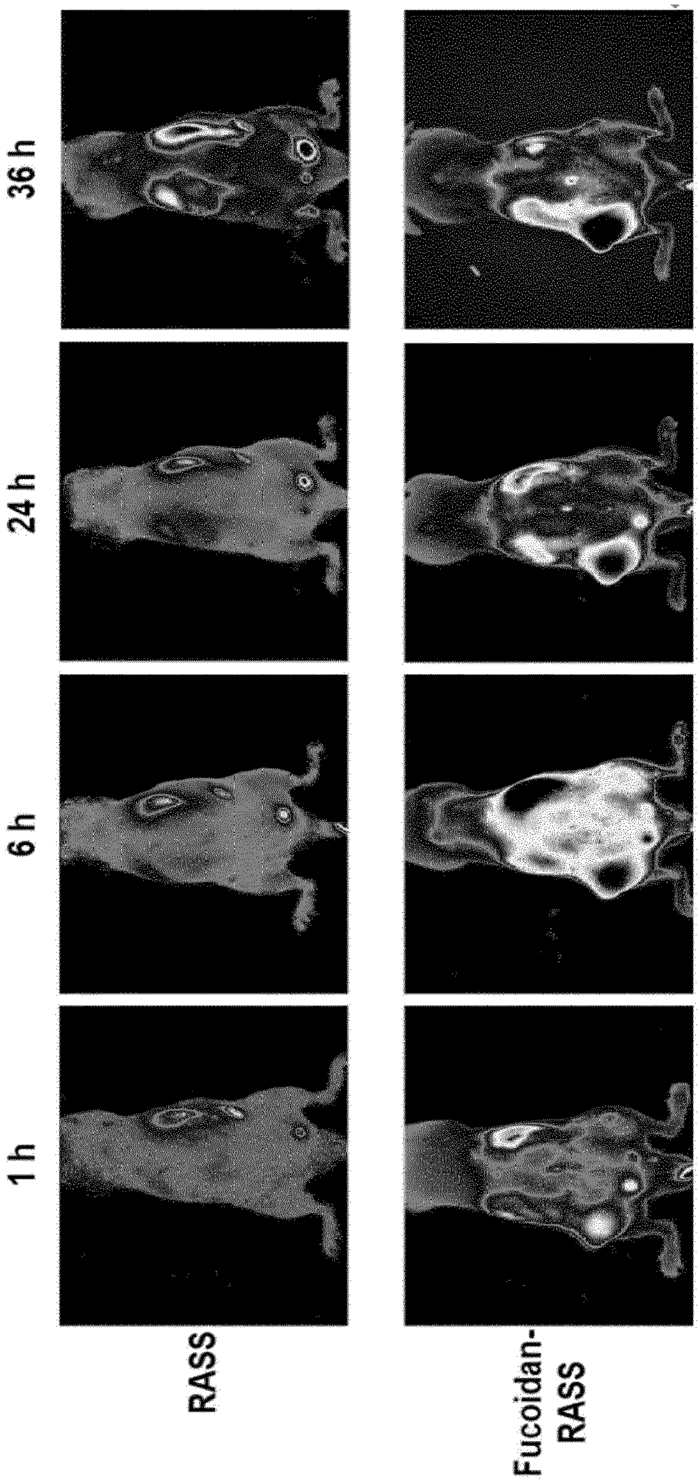

FIG. 10

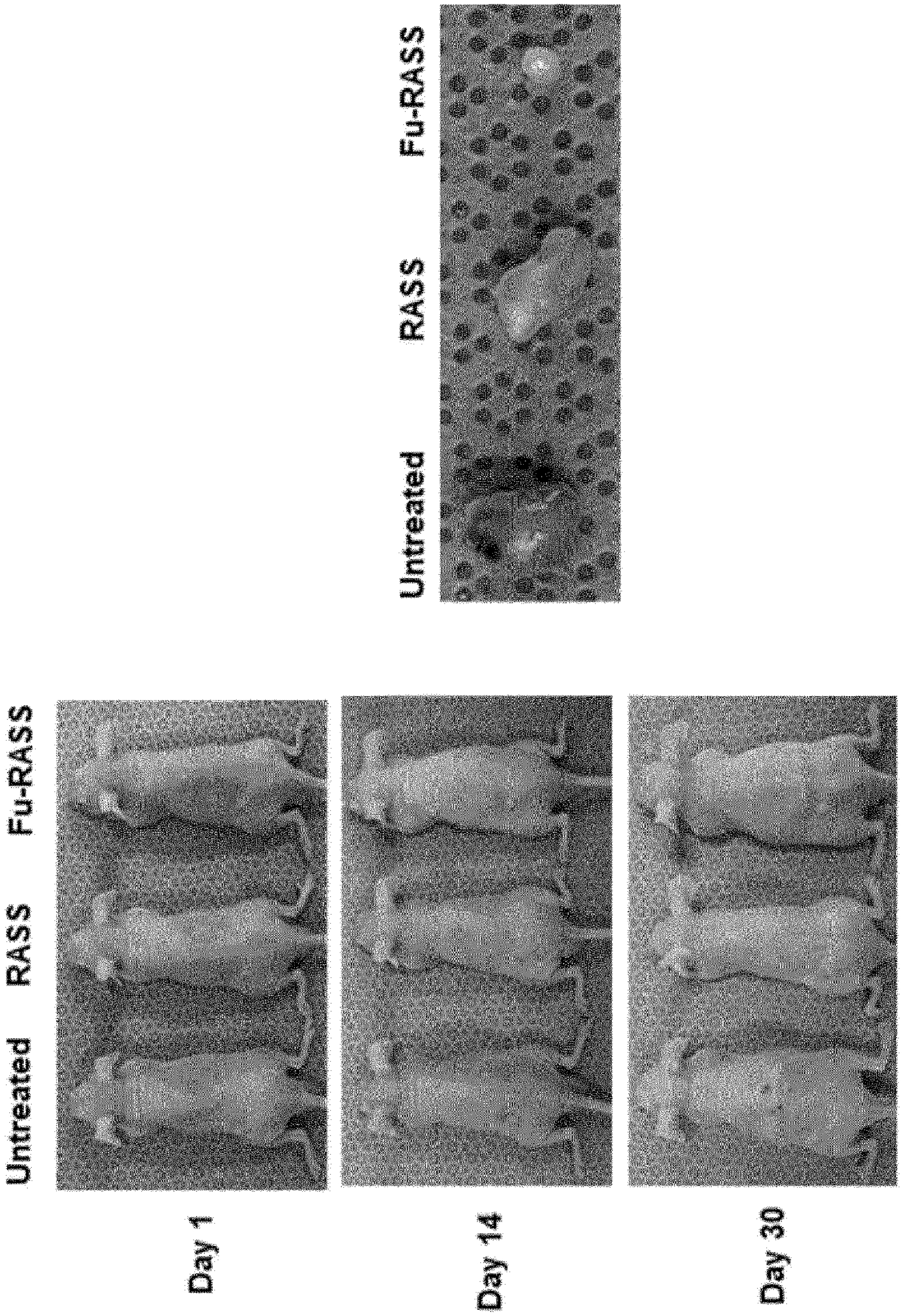
Untreated
RASS
Fu-RASS
Untreated
RASS
Fu-RASS
Day 1
Day 14
Day 30

FIG. 11

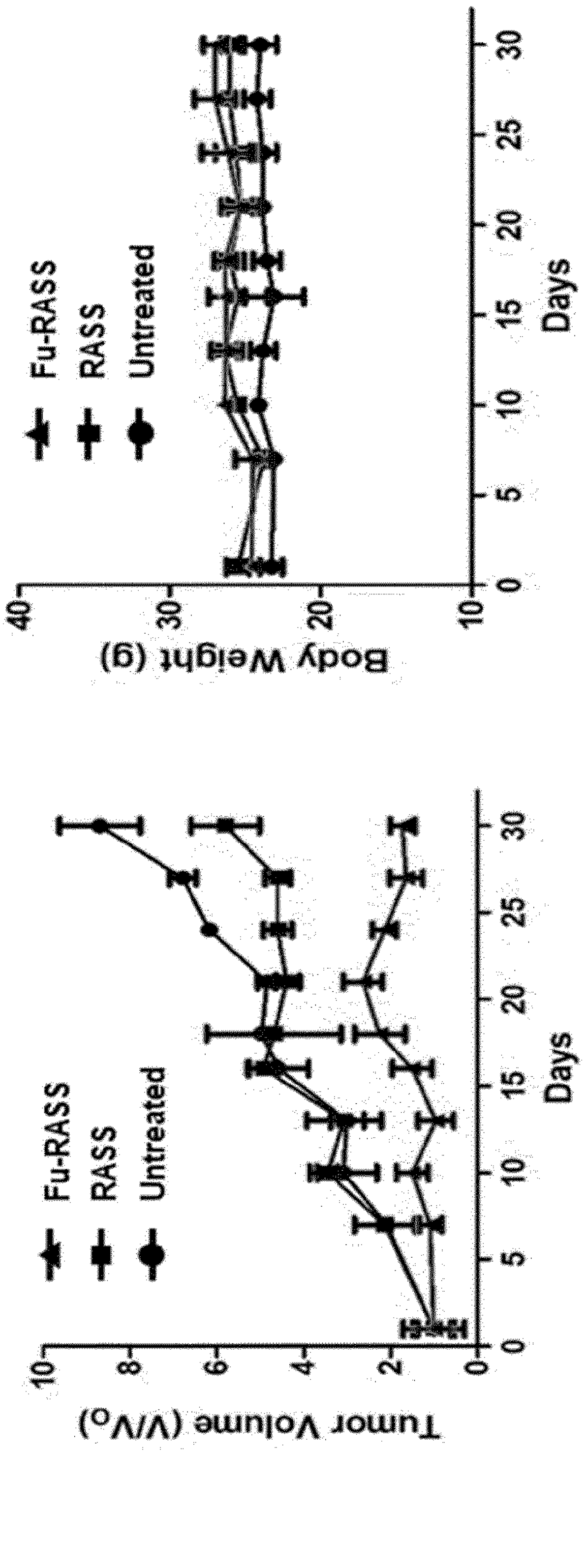
Fu-RASS
RASS
Untreated
Body Weight (g)
Days
Tumor Volume (V/V$_o$)
Days

FIG. 12

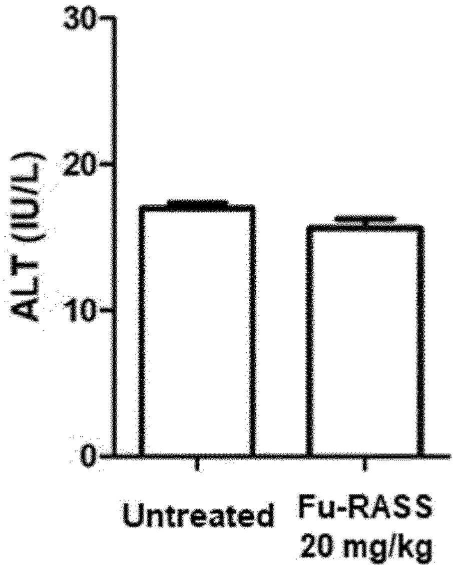

FIG. 13

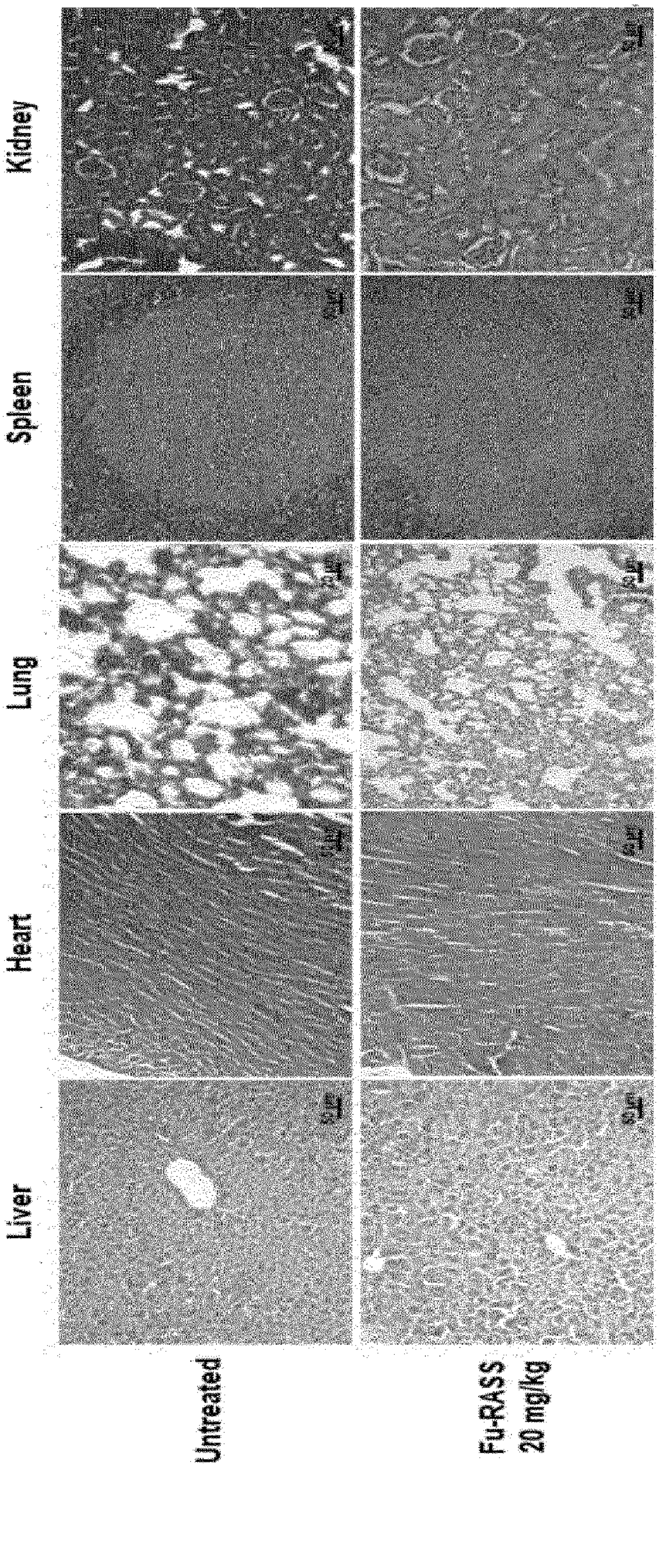

FIG. 14

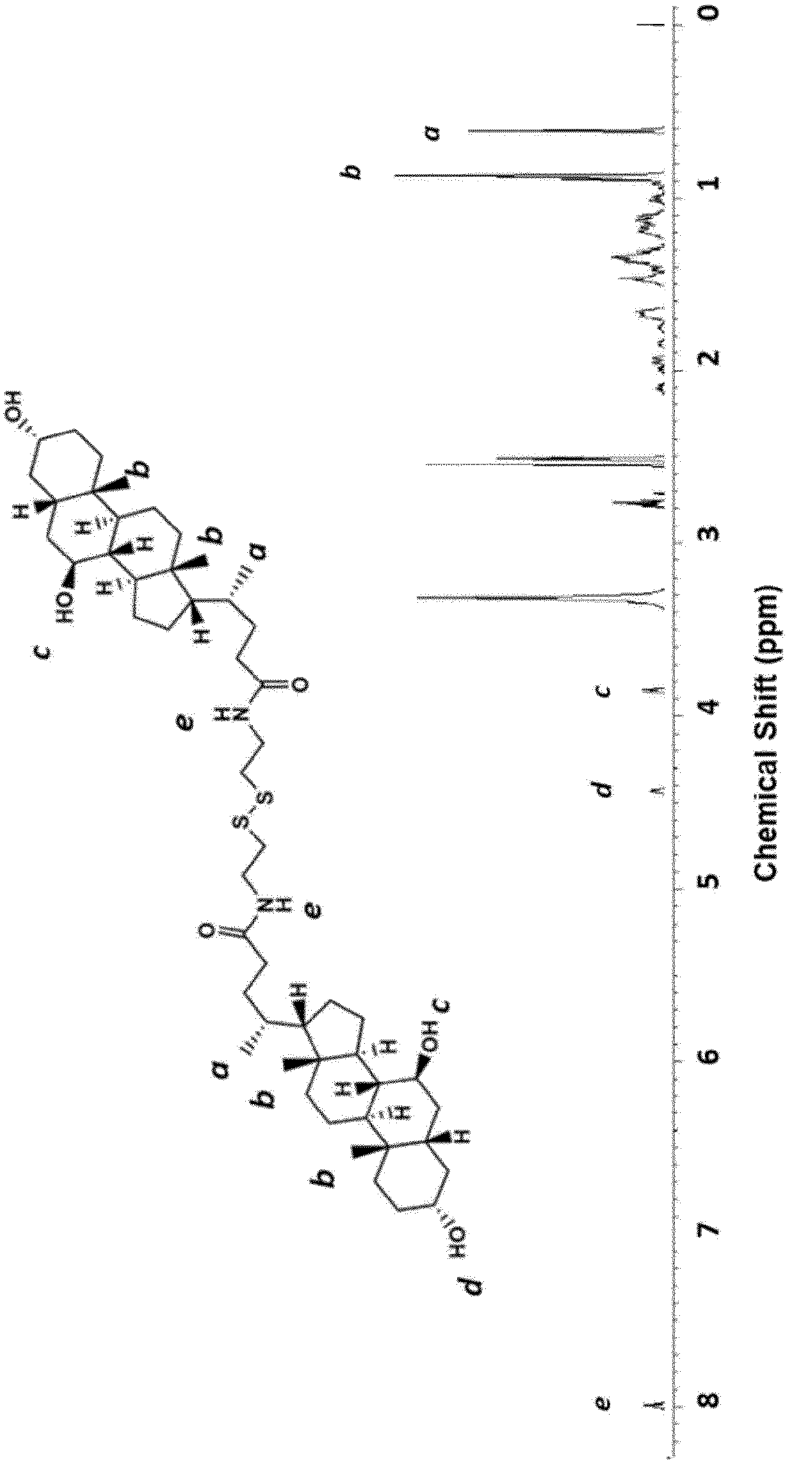

FIG. 15

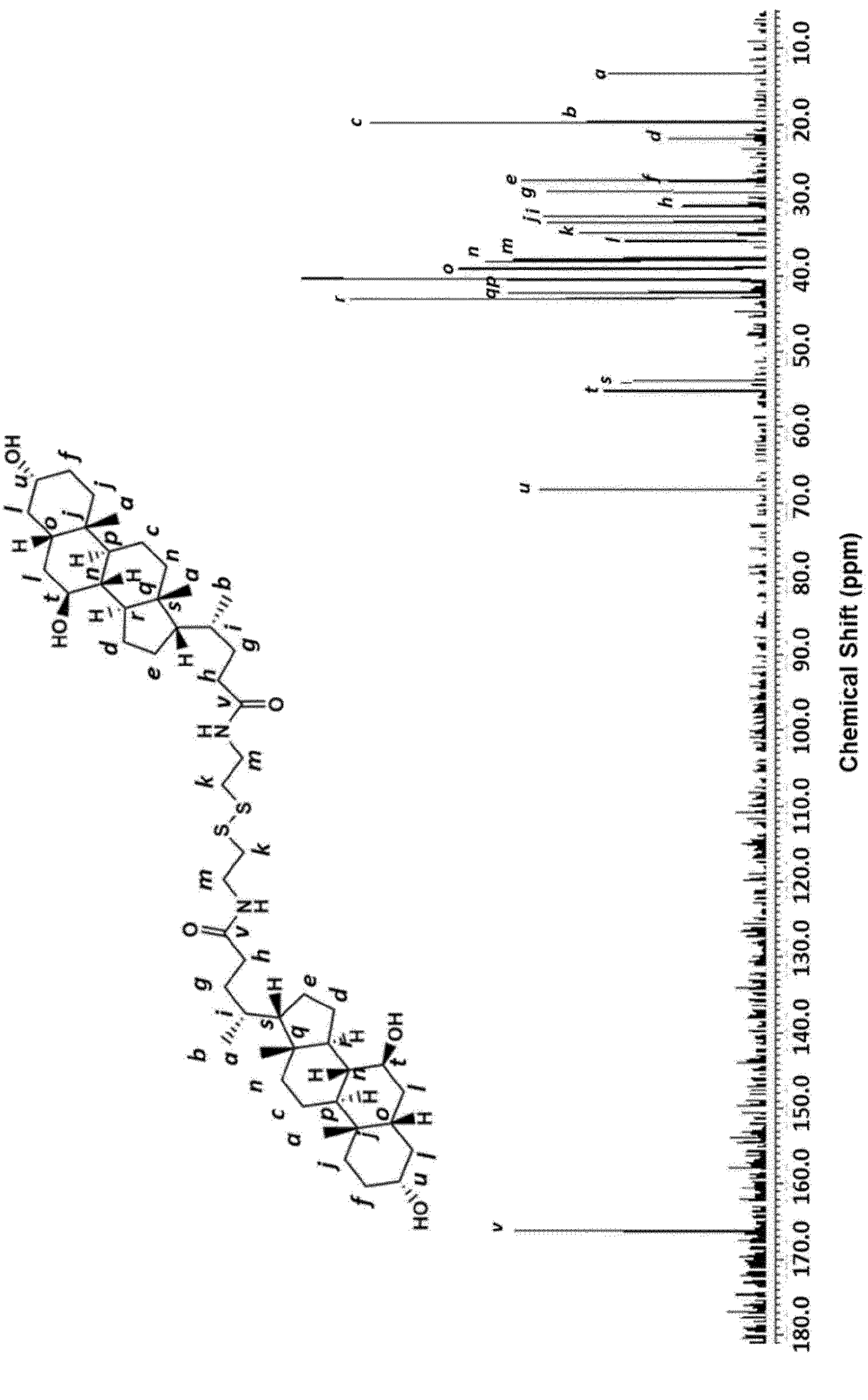
Chemical Shift (ppm)
10.0
20.0
30.0
40.0
50.0
60.0
70.0
80.0
90.0
100.0
110.0
120.0
130.0
140.0
150.0
160.0
170.0
180.0

FIG. 16

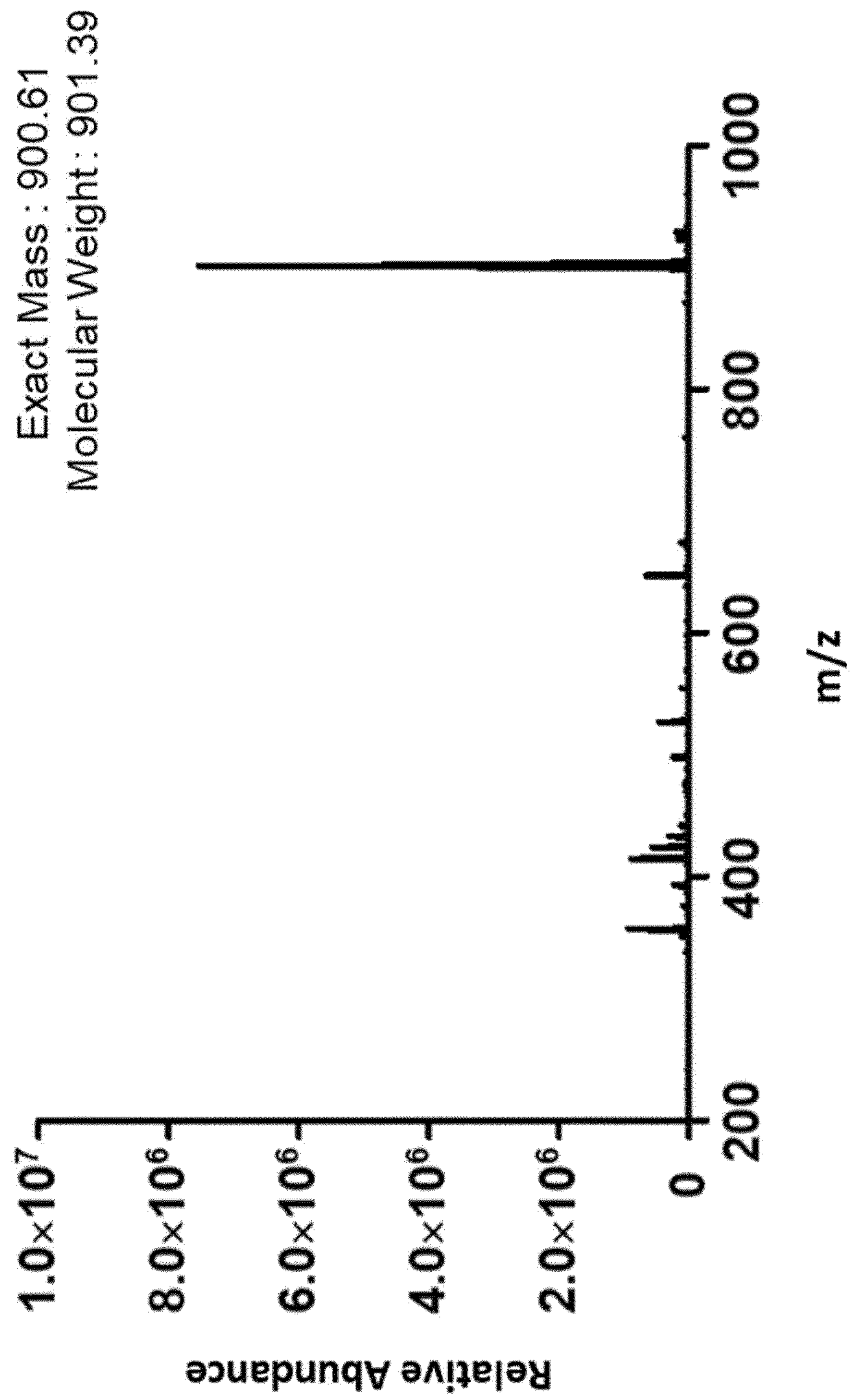
Exact Mass : 900.61
Molecular Weight : 901.39
Relative Abundance
1.0×10^7
8.0×10^6
6.0×10^6
4.0×10^6
2.0×10^6
0
200
400
600
800
1000
m/z

FIG. 17

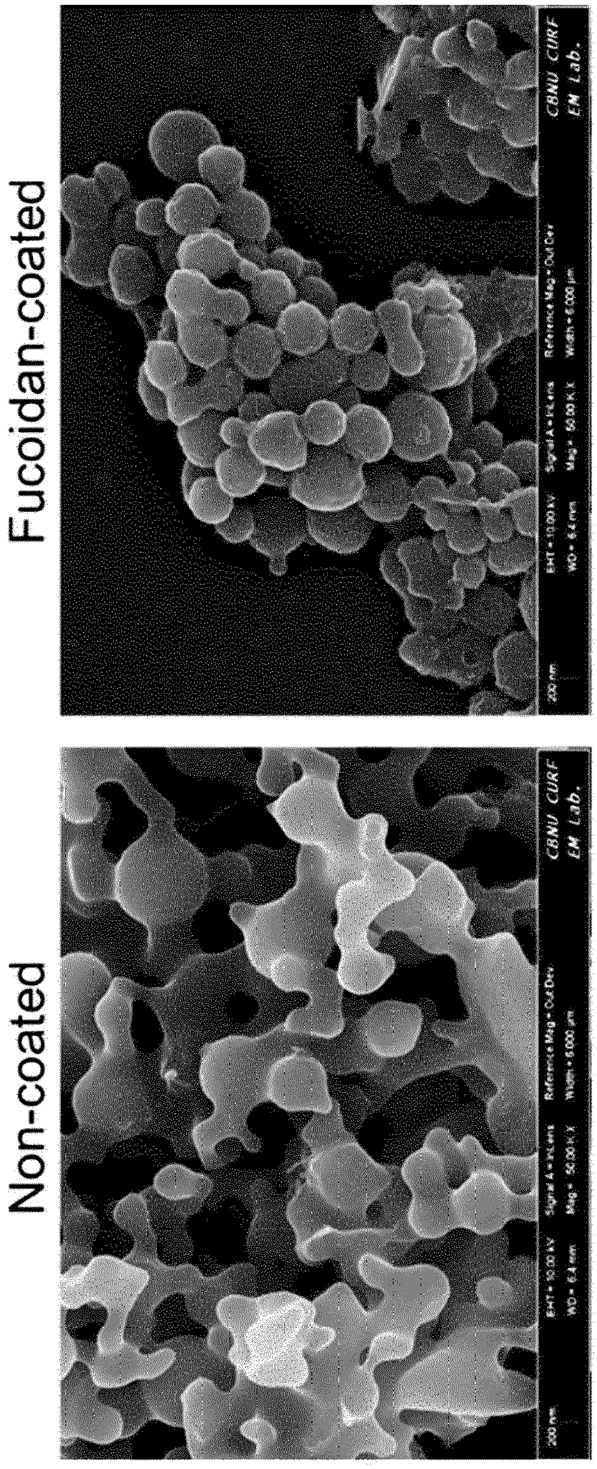

FIG. 18

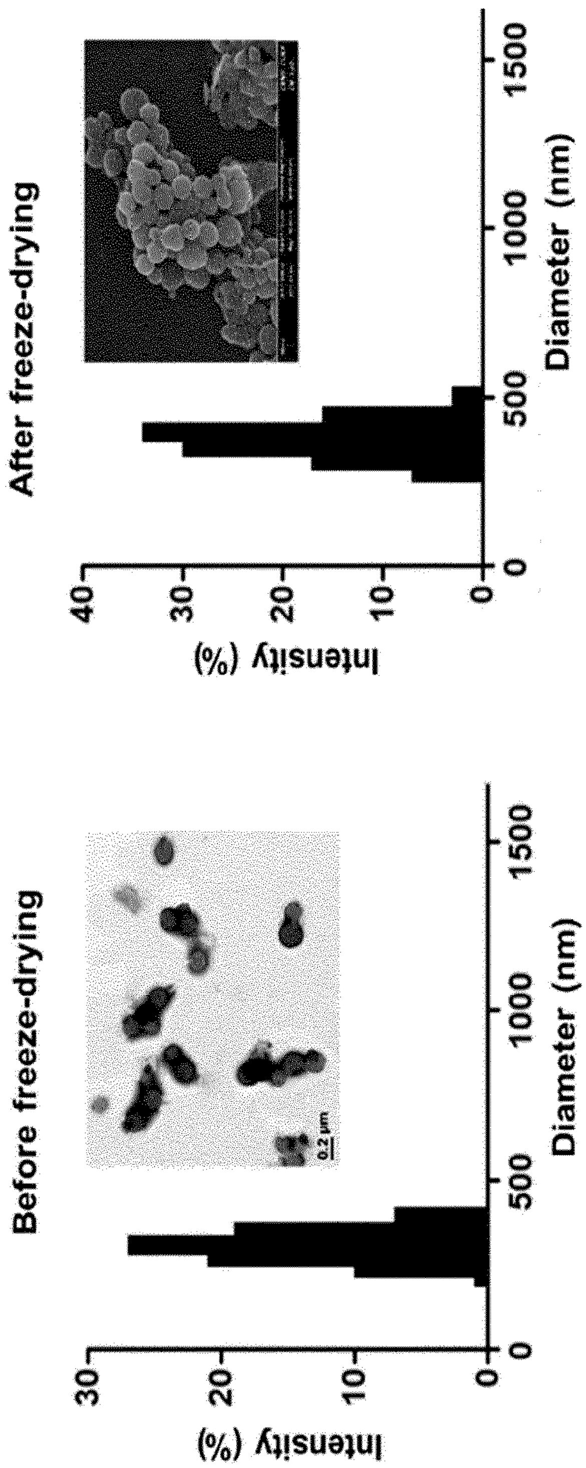
After freeze-drying
Intensity (%)
40
30
20
10
0
0
500
1000
1500
Diameter (nm)
Before freeze-drying
Intensity (%)
30
20
10
0
0
500
1000
1500
Diameter (nm)
0.2 μm

FIG. 19

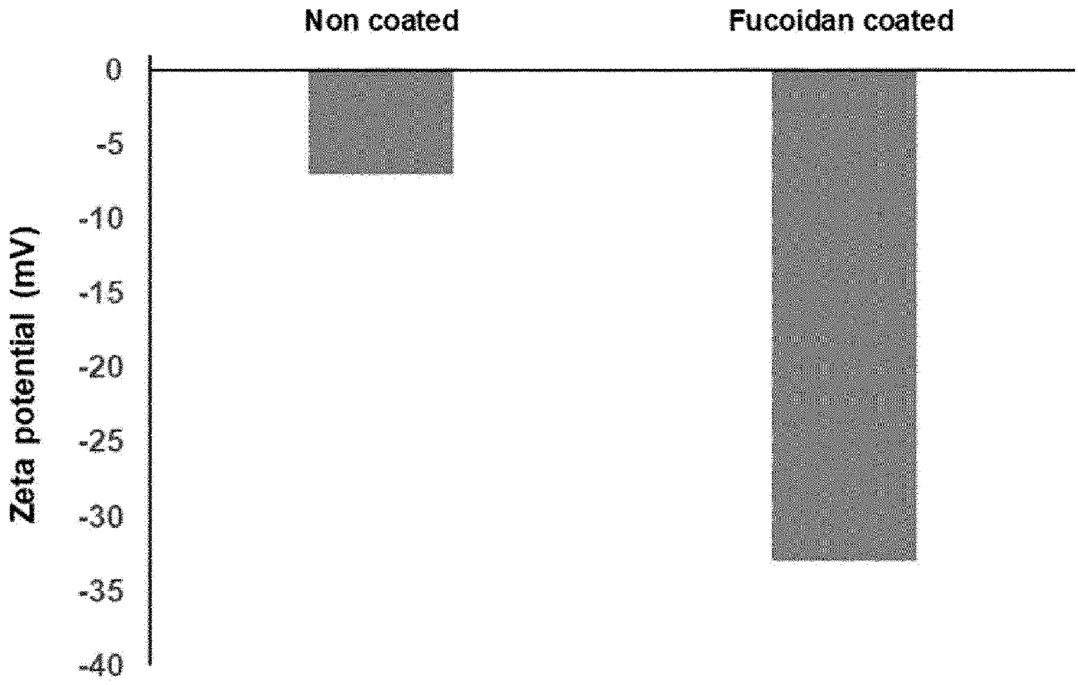
Non coated
Fucoidan coated
0
-5
-10
-15
-20
-25
-30
-35
-40
Zeta potential (mV)

FIG. 20

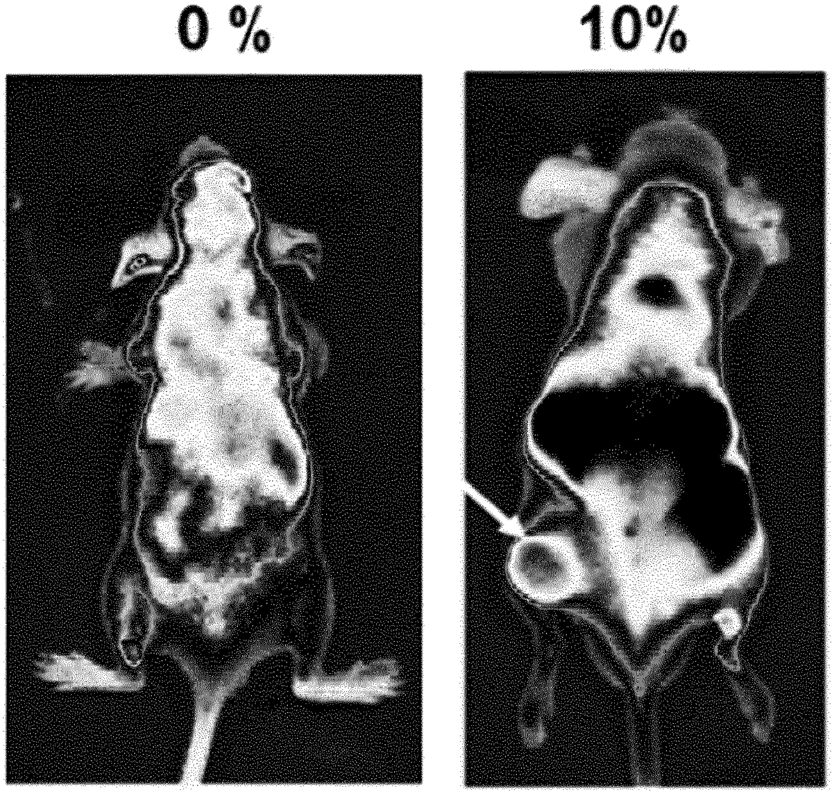

FIG. 21

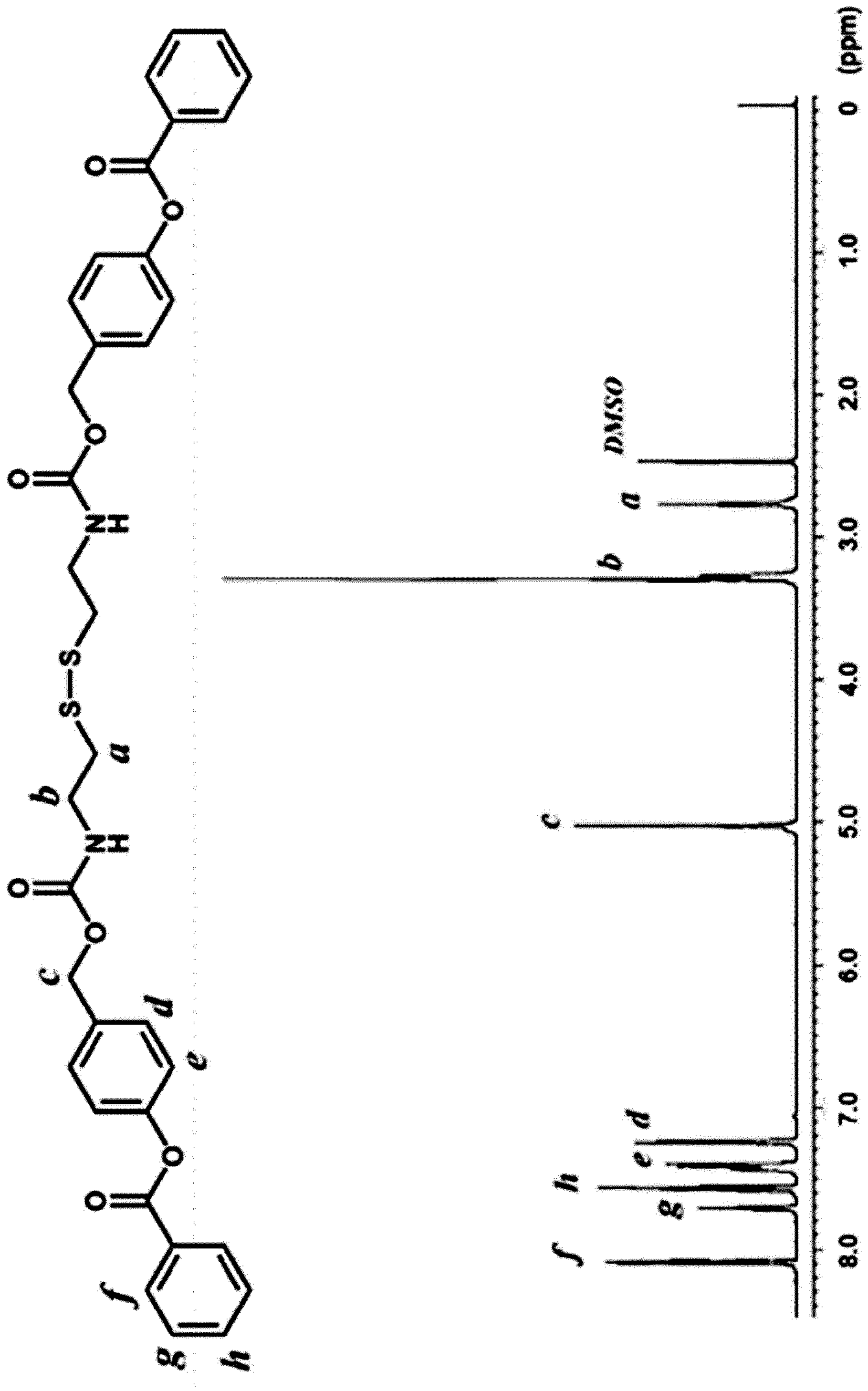

FIG. 22

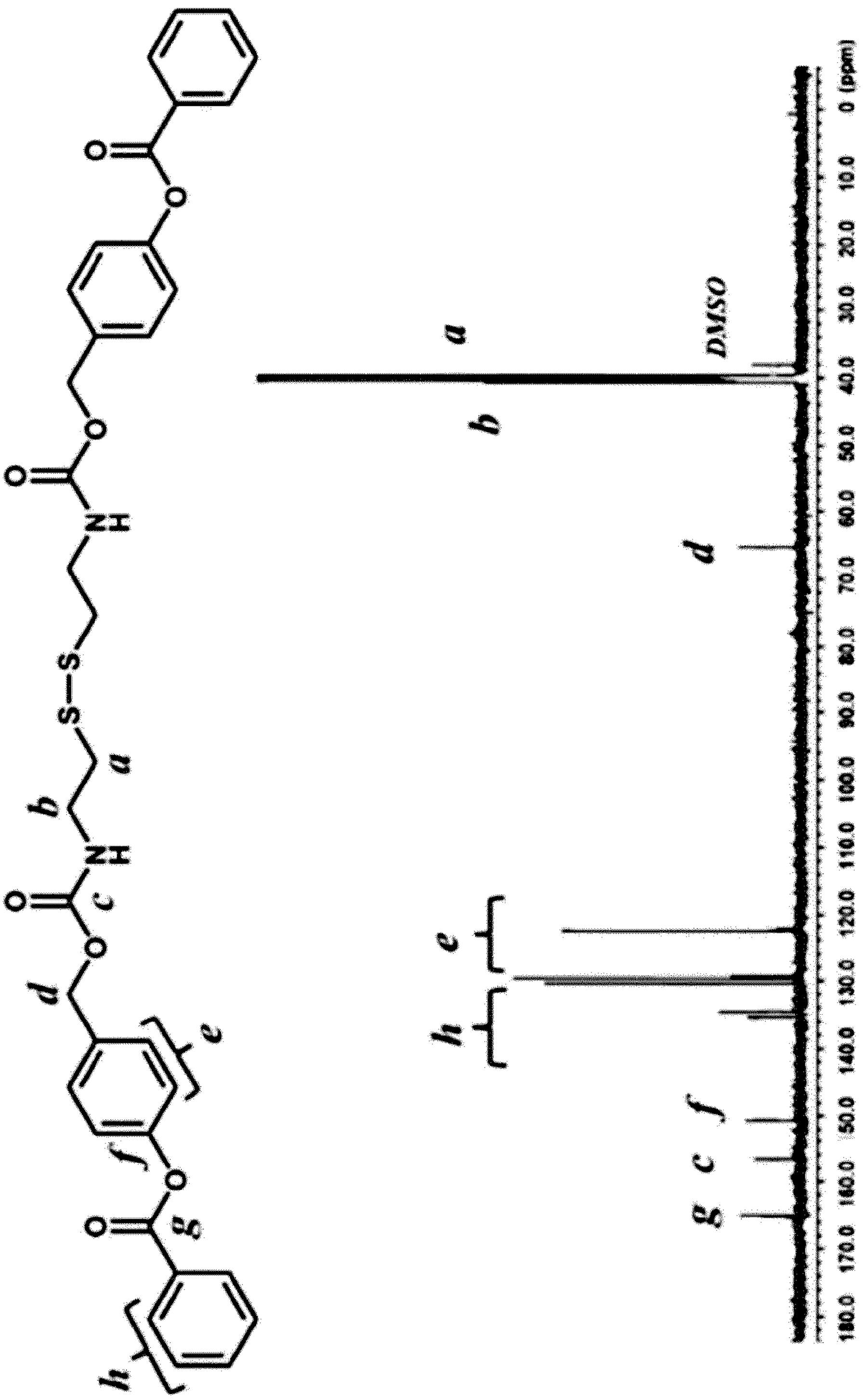

FIG. 23

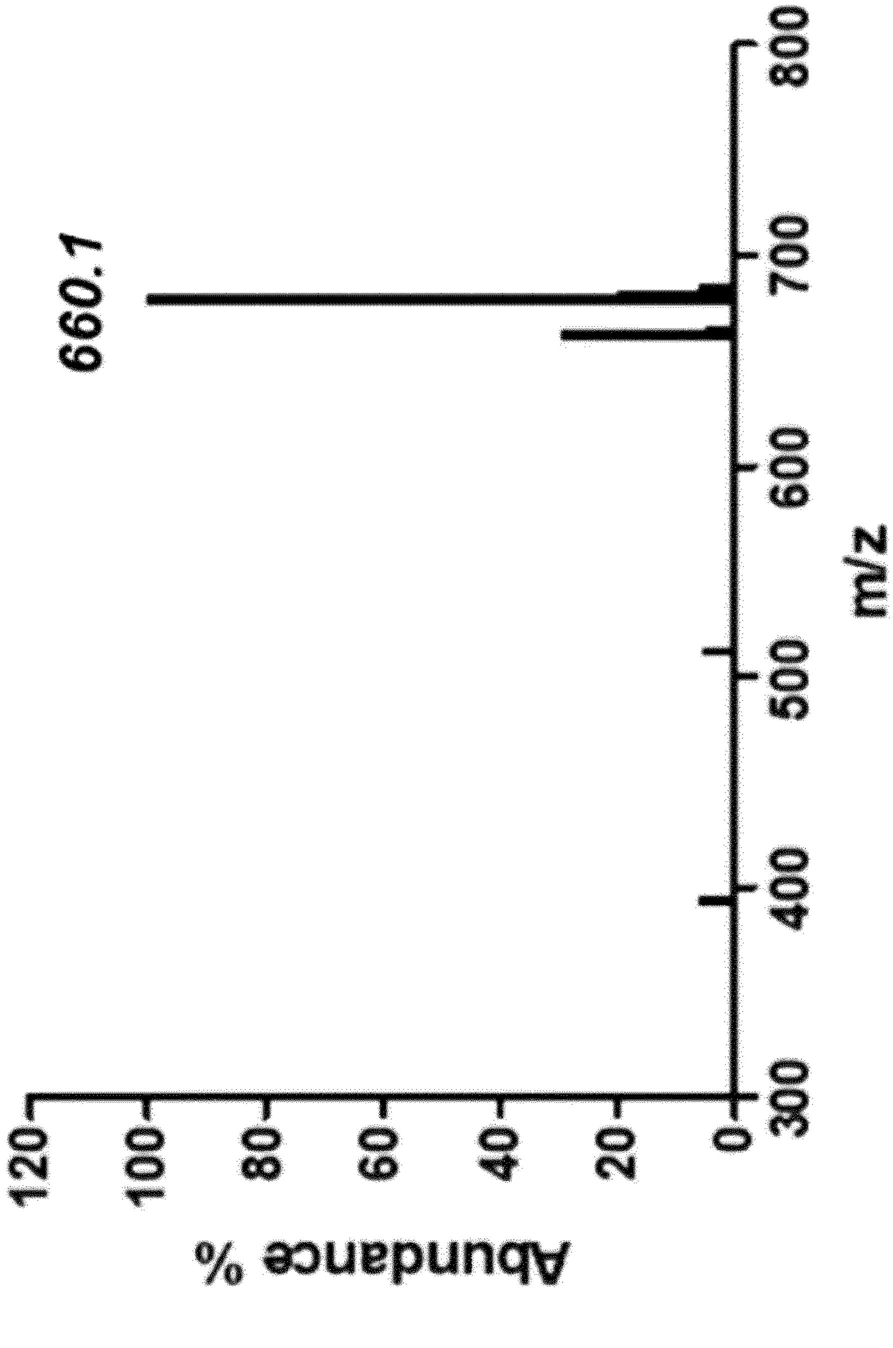

FIG. 24

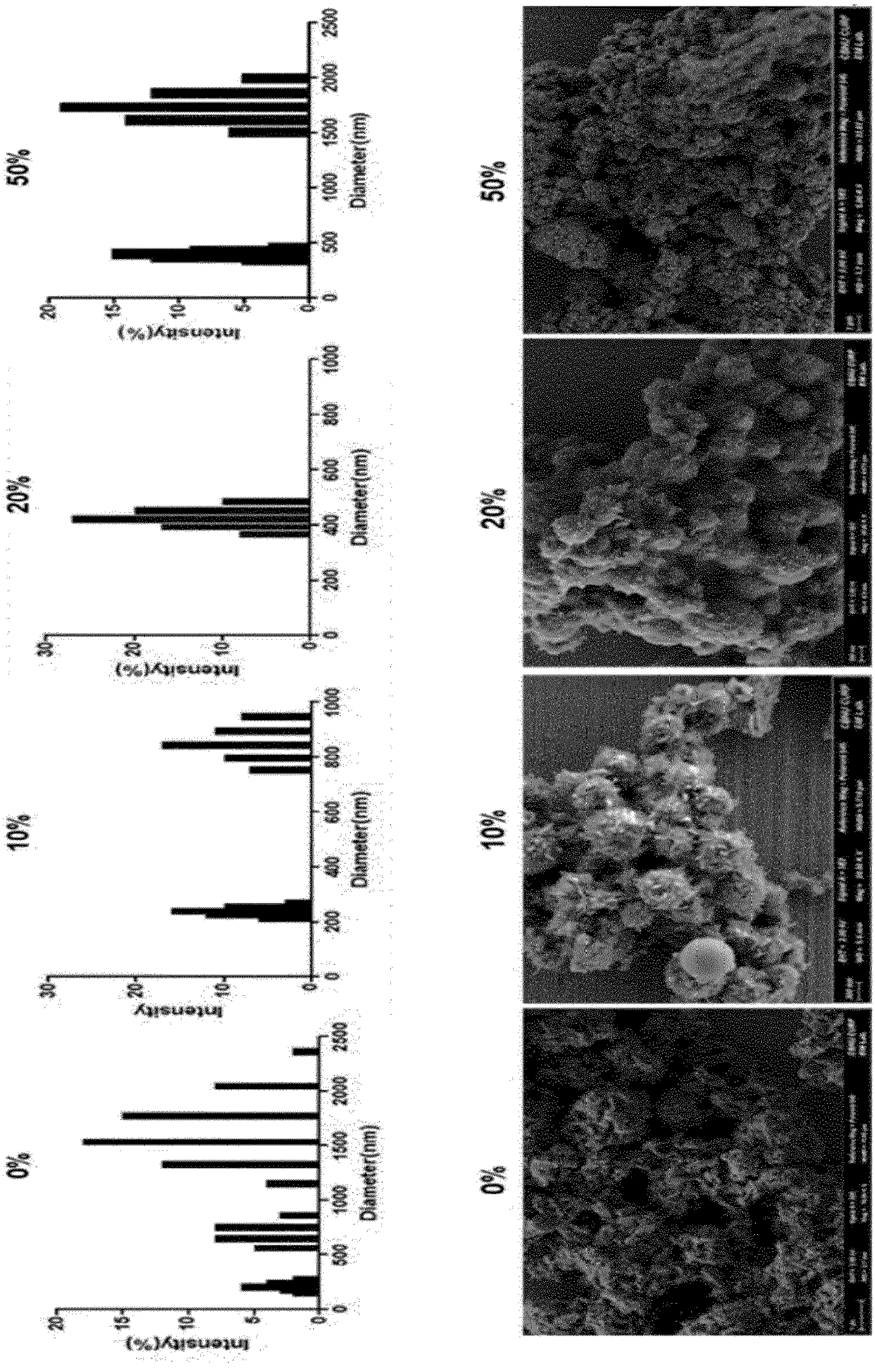
0%
10%
20%
50%
Intensity(%)
Intensity
Diameter(nm)
0
5
10
15
20
30
200
400
500
600
800
1000
1500
2000
2500

FIG. 25

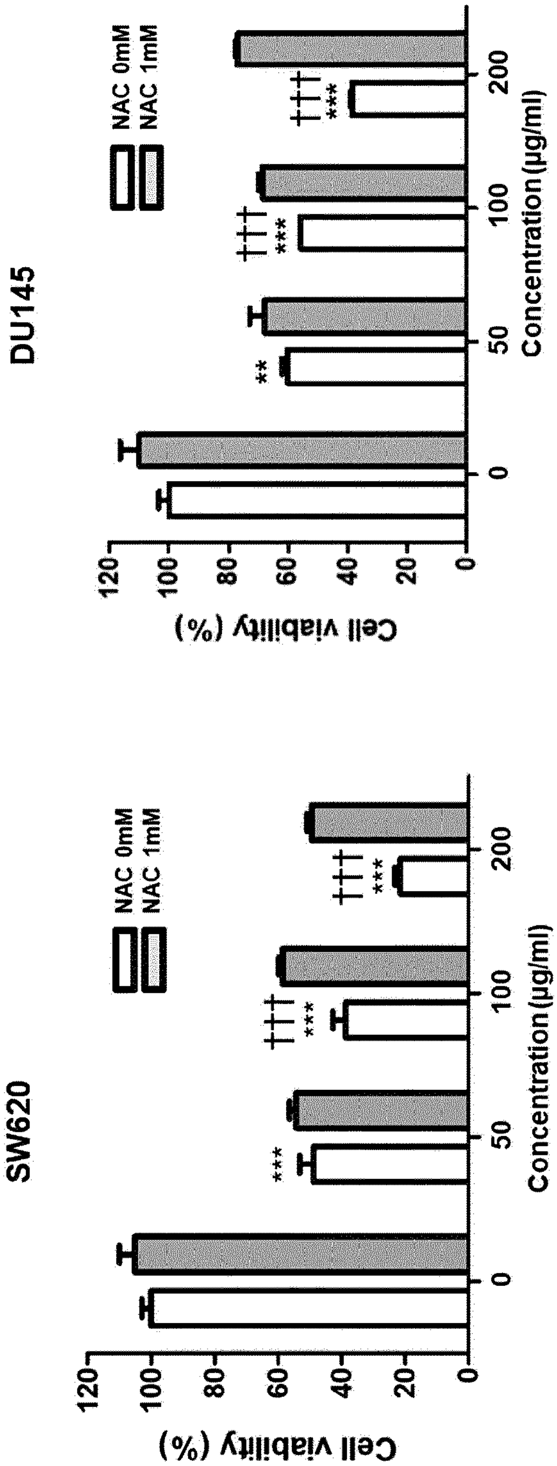
DU145
NAC 0mM
NAC 1mM
Cell viability (%)
Concentration (µg/ml)
SW620
NAC 0mM
NAC 1mM
Cell viability (%)
Concentration (µg/ml)

FIG. 26

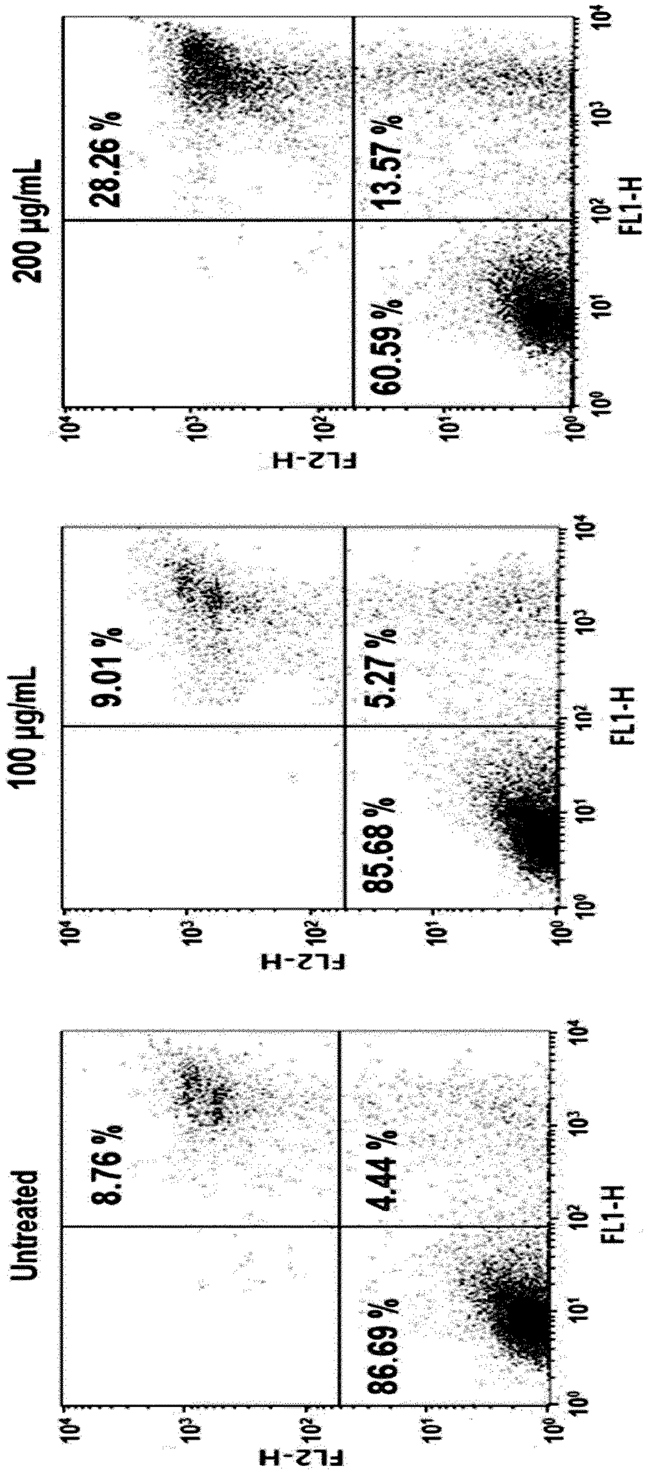

Untreated
8.76 %
4.44 %
86.69 %
100 µg/mL
9.01 %
5.27 %
85.68 %
200 µg/mL
28.26 %
13.57 %
60.59 %
FL1-H
FL2-H

FIG. 27

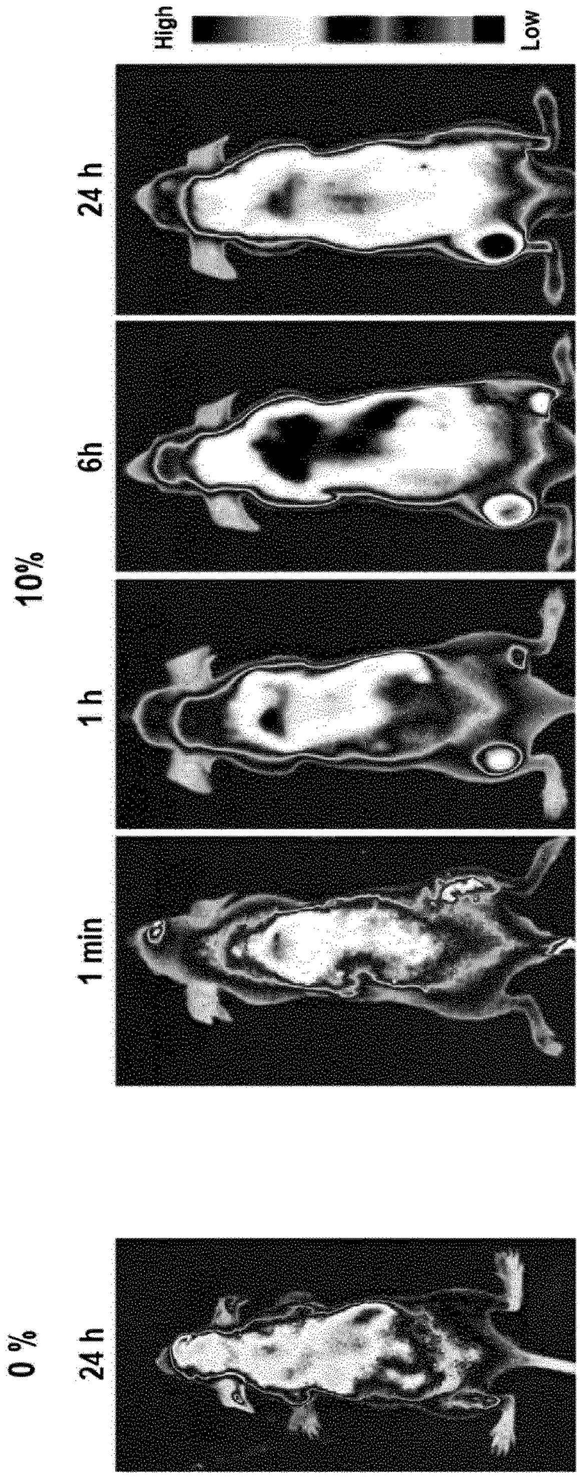

FIG. 28

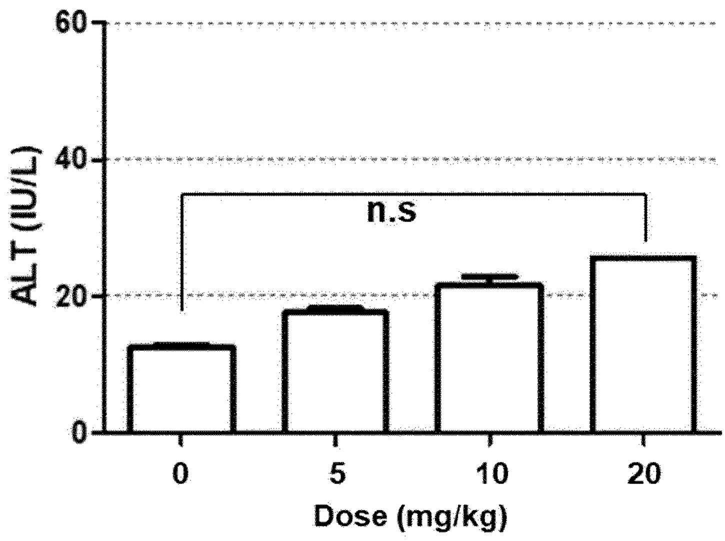

FIG. 29

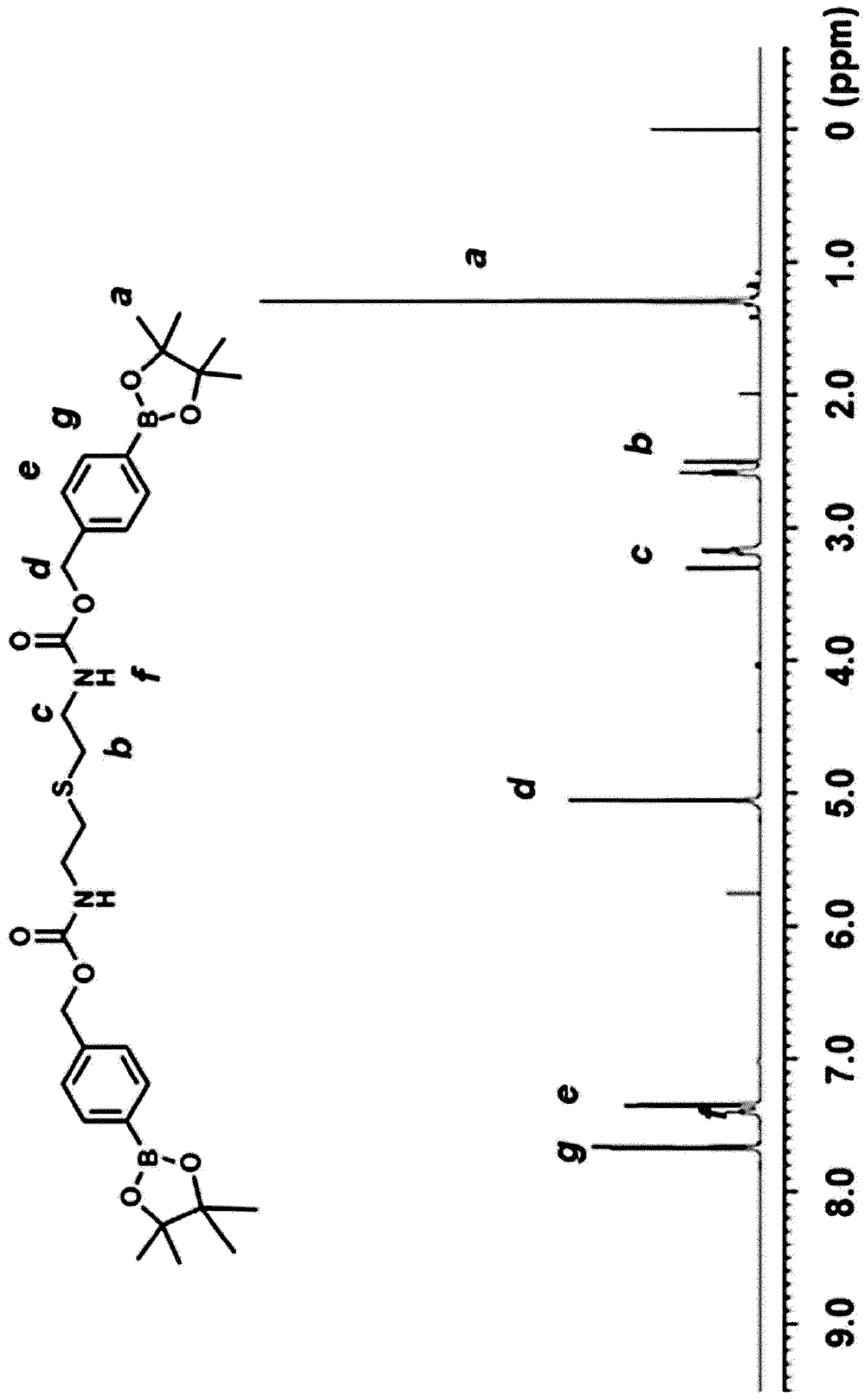

FIG. 30

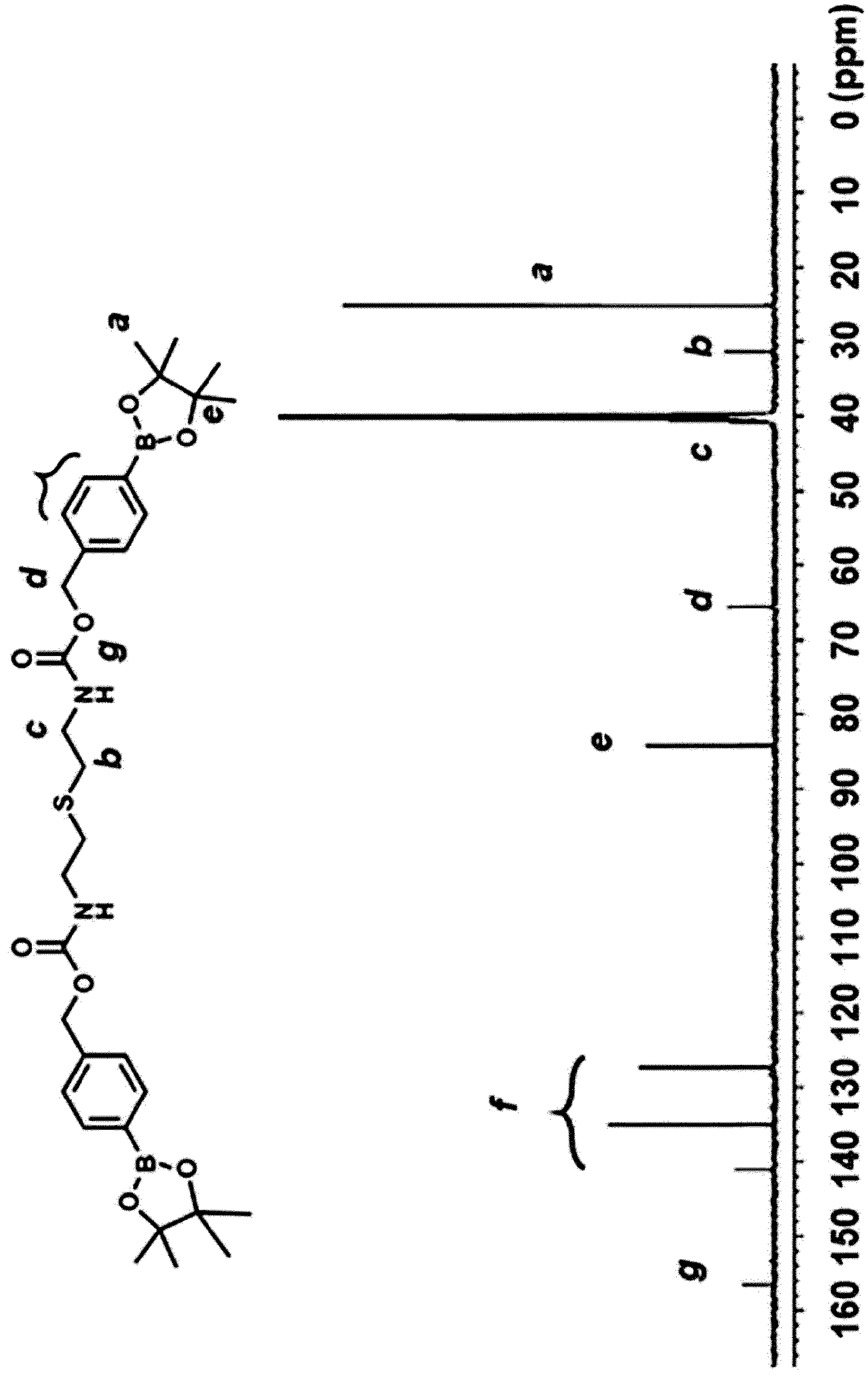

FIG. 31

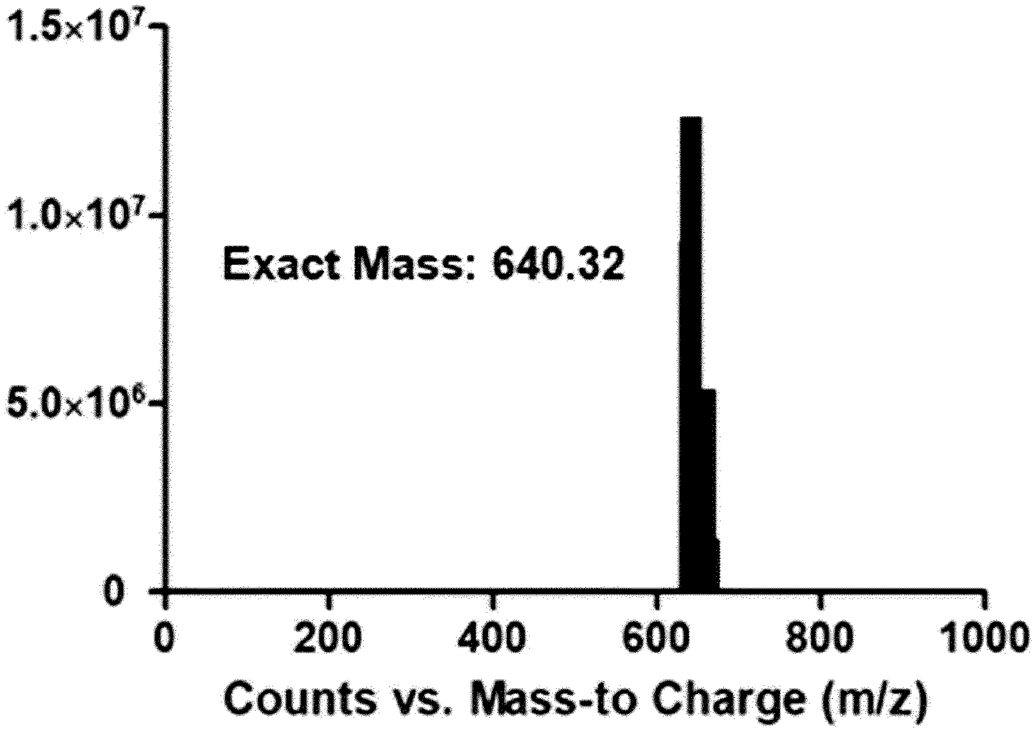
1.5×10^7
1.0×10^7
5.0×10^6
0
Exact Mass: 640.32
0
200
400
600
800
1000
Counts vs. Mass-to Charge (m/z)

FIG. 32

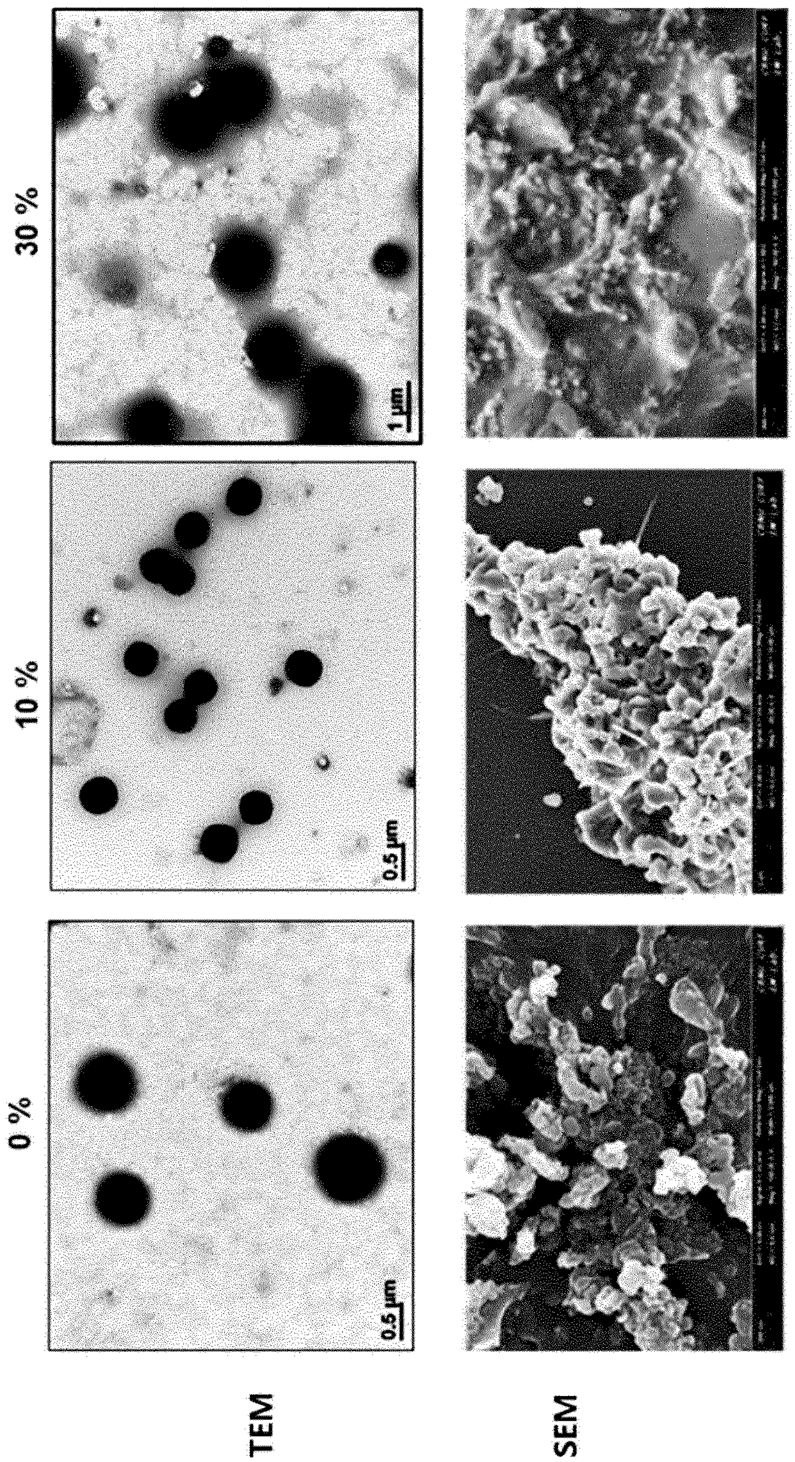

FIG. 33

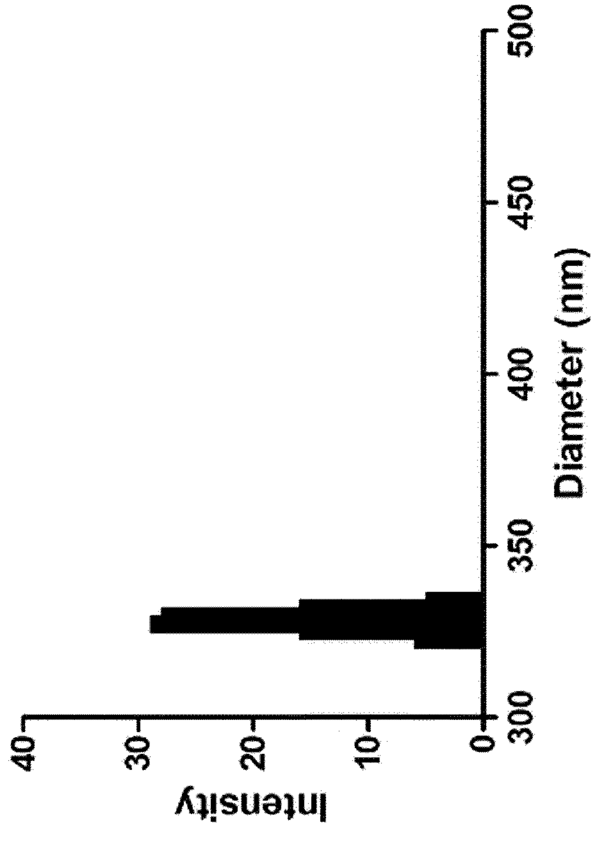

FIG. 34

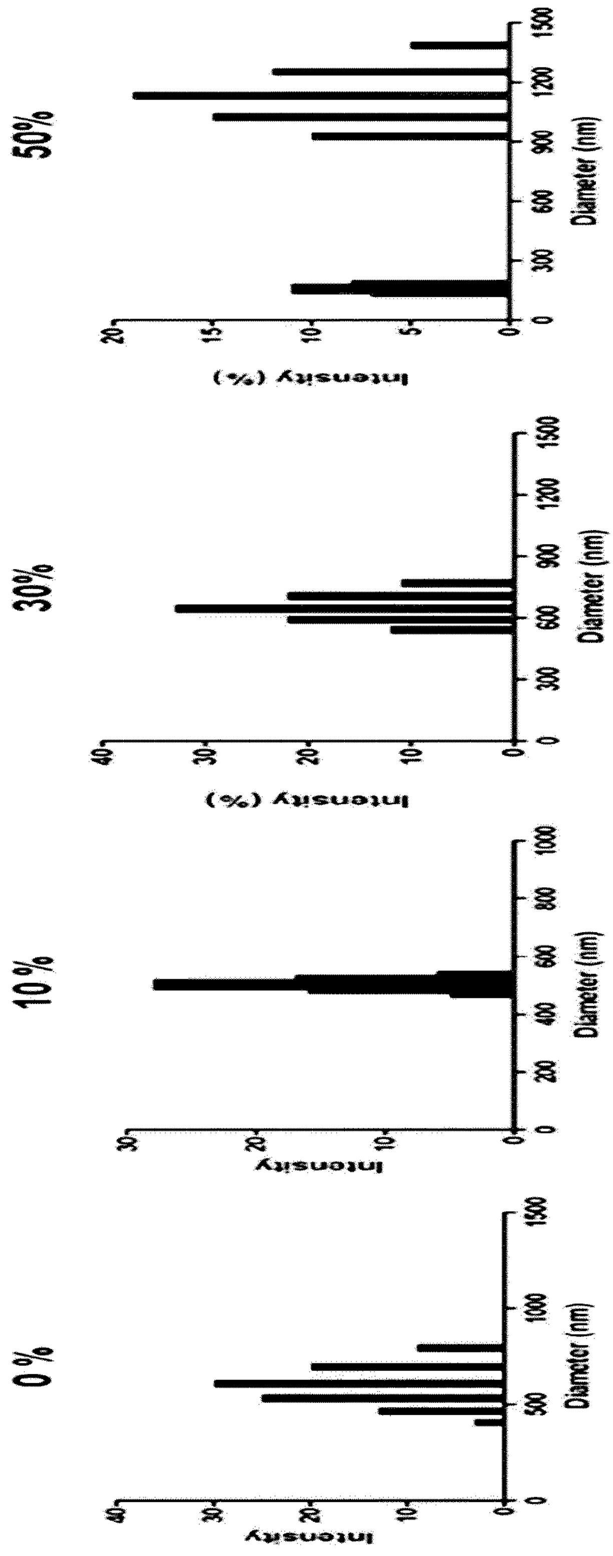
50%
Intensity (%)
Diameter (nm)
30%
Intensity (%)
Diameter (nm)
10 %
Intensity
Diameter (nm)
0 %
Intensity
Diameter (nm)

FIG. 35

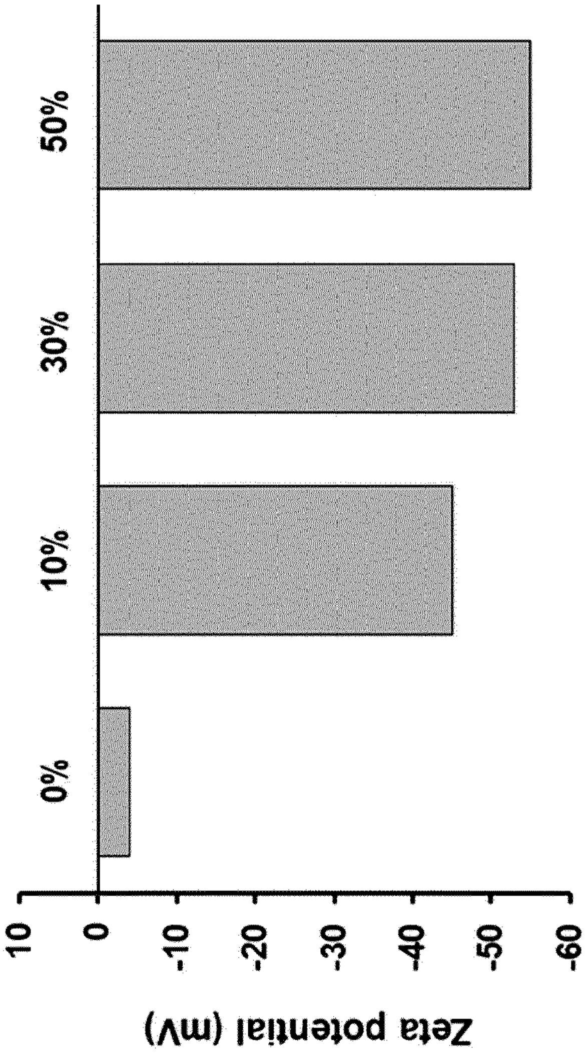
Zeta potential (mV)
10
0
-10
-20
-30
-40
-50
-60
0%
10%
30%
50%

FIG. 36

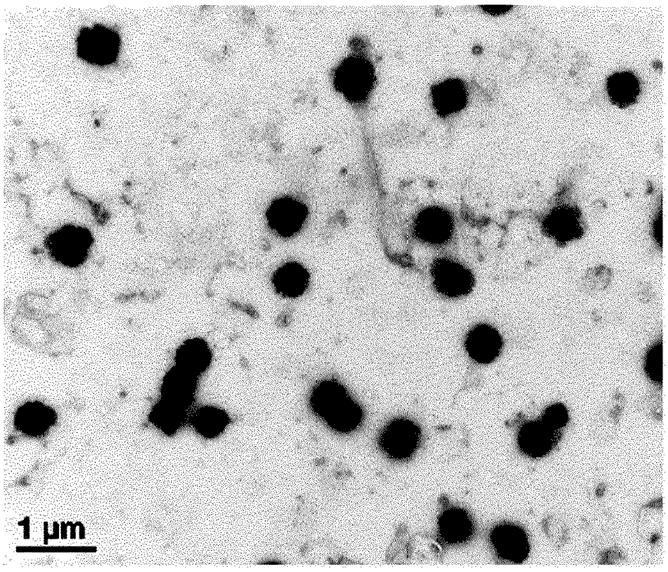


FIG. 37

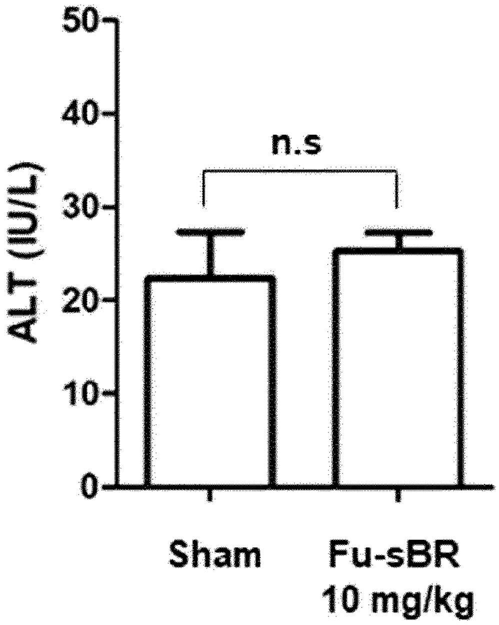

FIG. 38

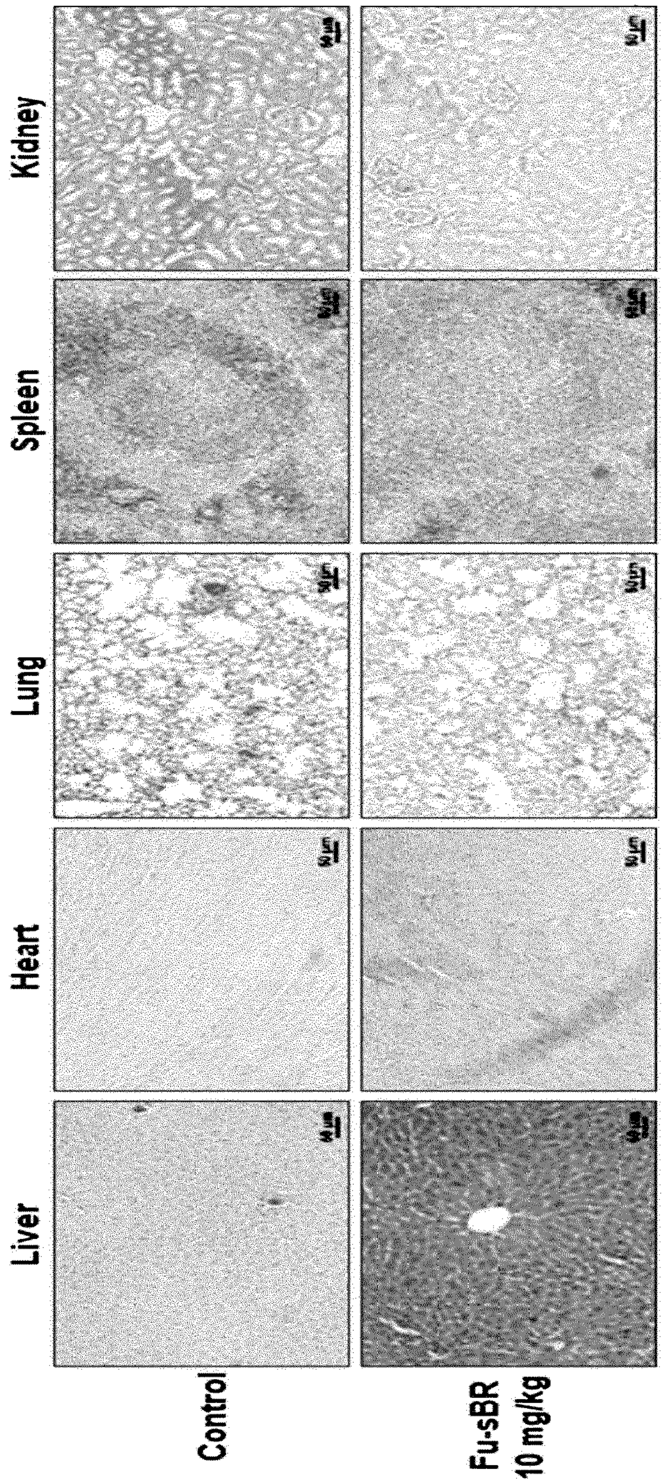

FIG. 39

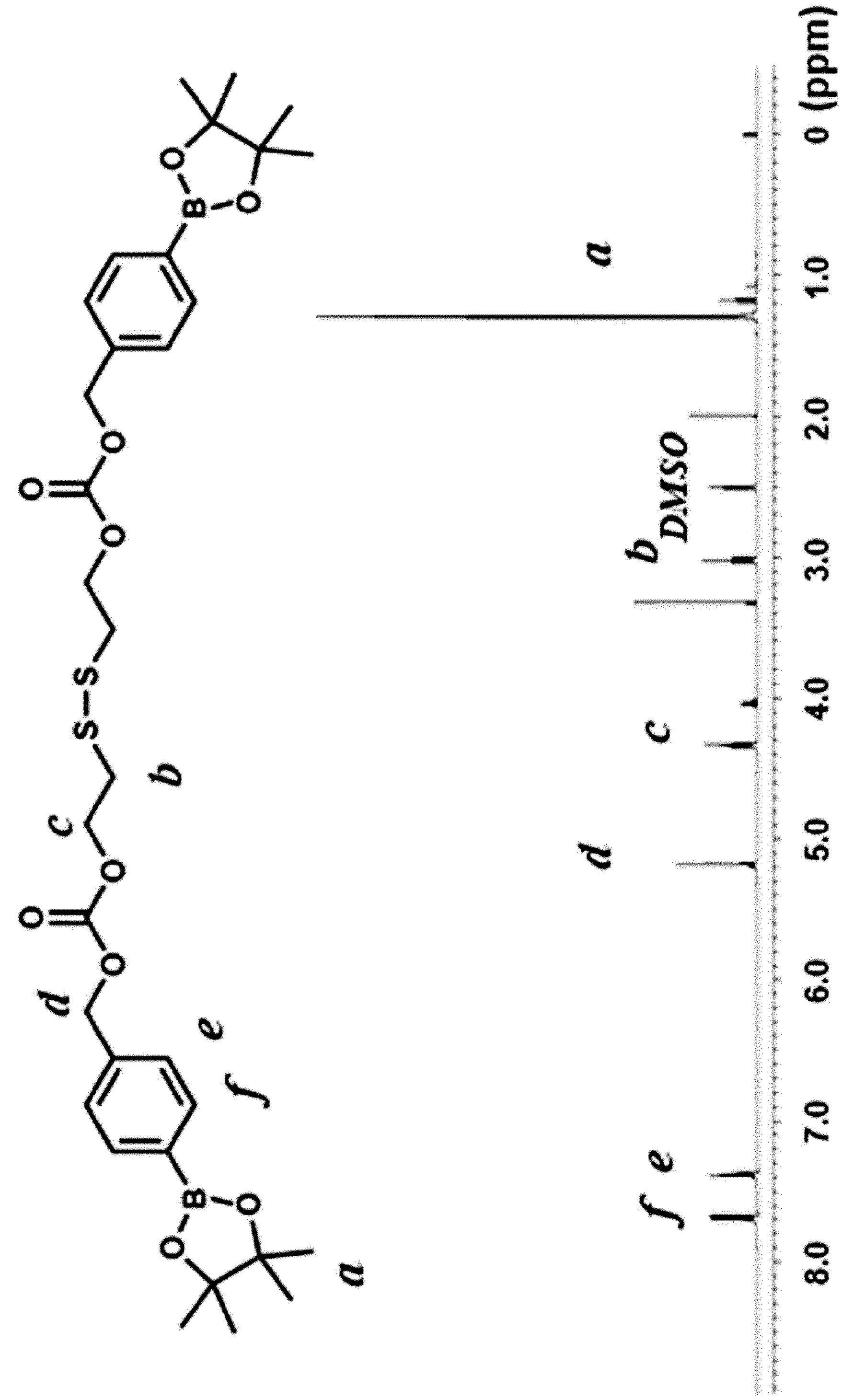

FIG. 40

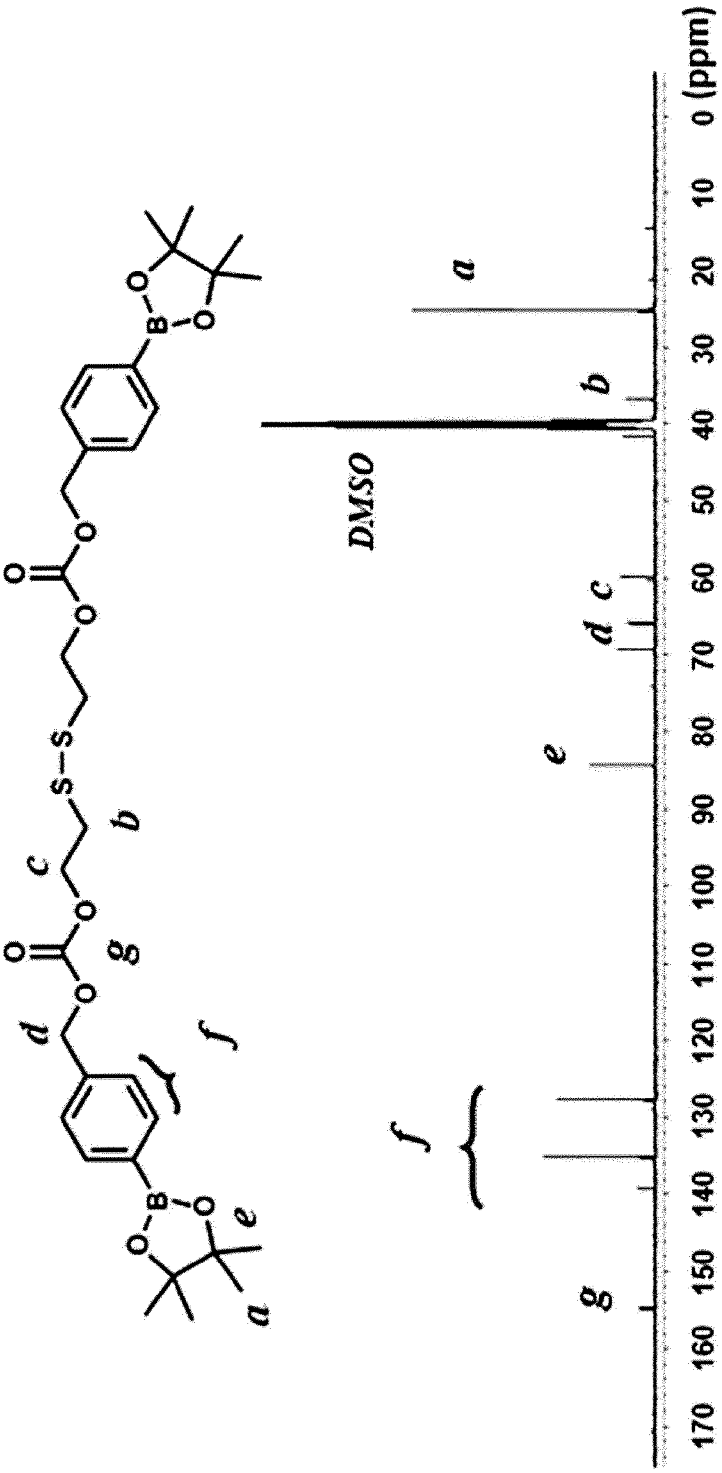
DMSO
a
b
c
d
e
f
g
0 10 20 30 40 50 60 70 80 90 100 110 120 130 140 150 160 170
(ppm)

FIG. 41

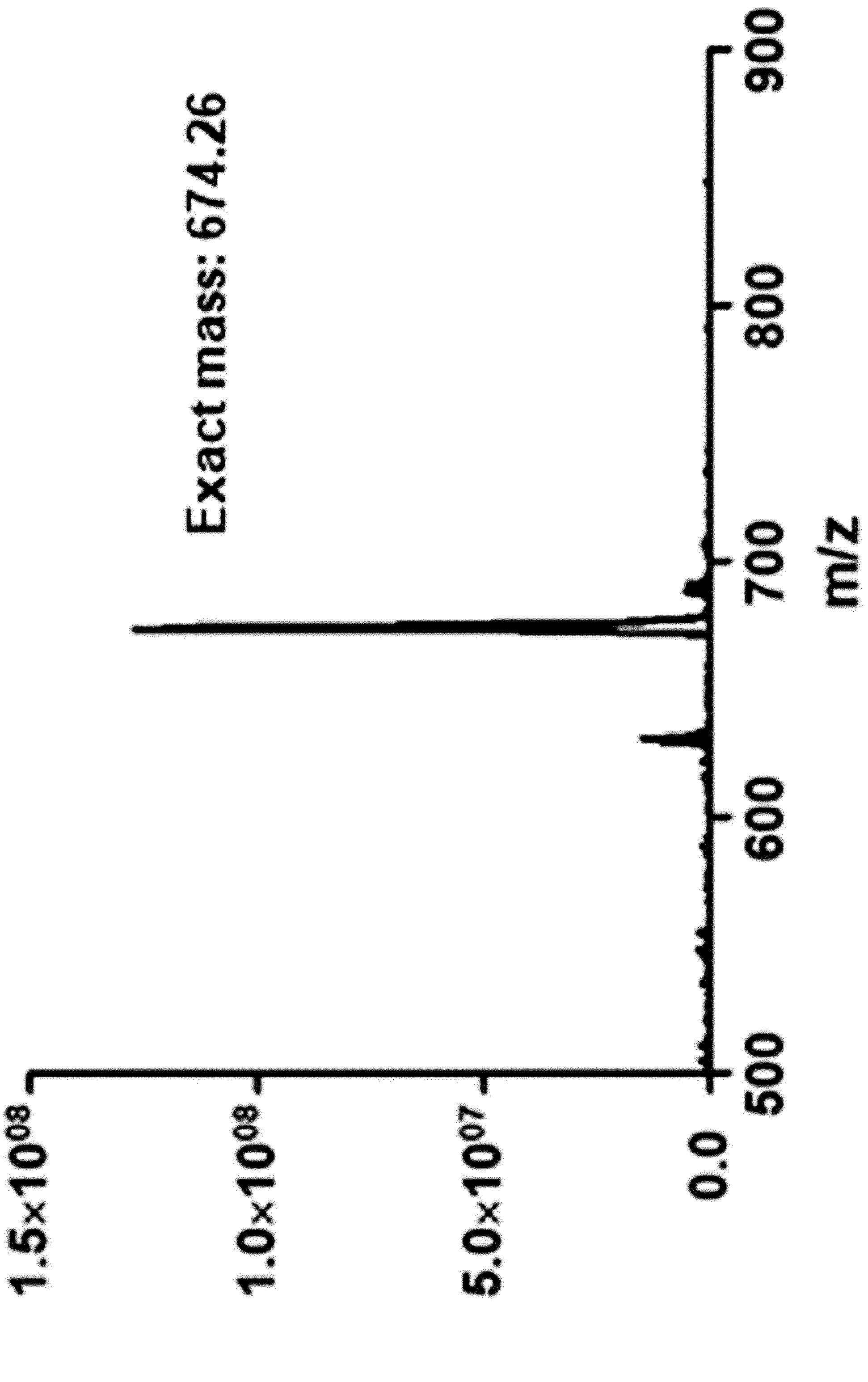
Exact mass: 674.26
1.5×10^08
1.0×10^08
5.0×10^07
0.0
500
600
700
800
900
m/z

FIG. 42

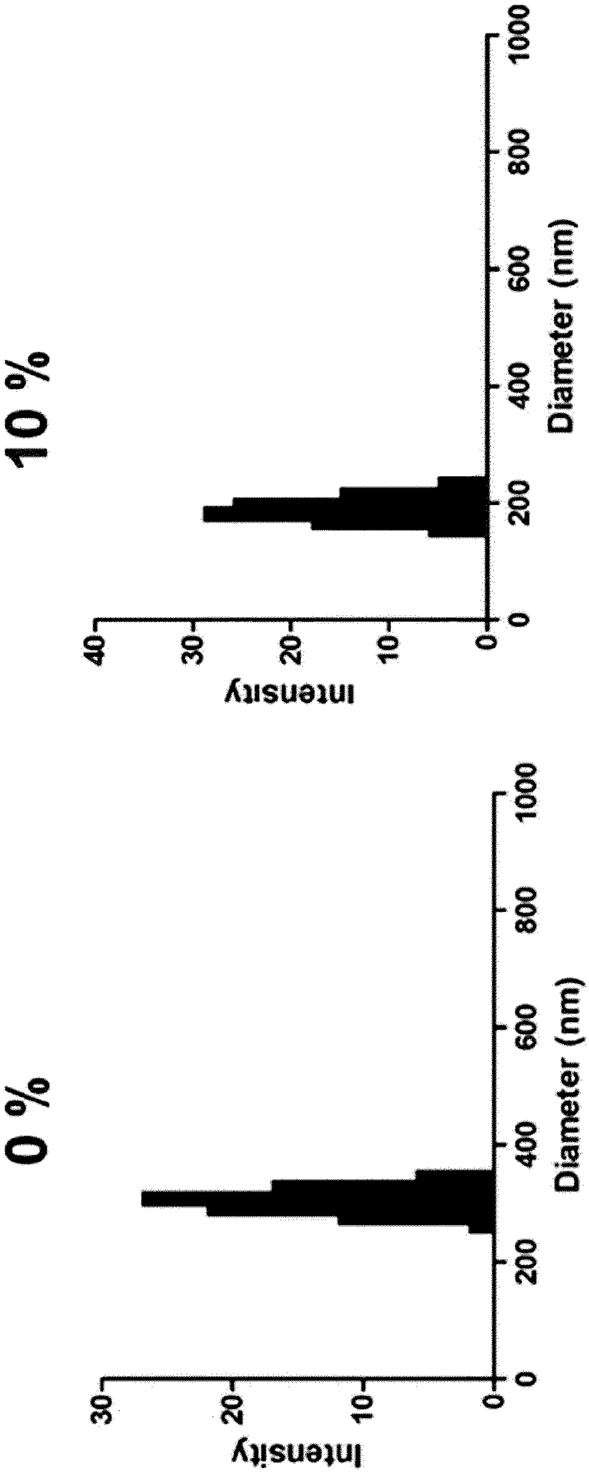

FIG. 43

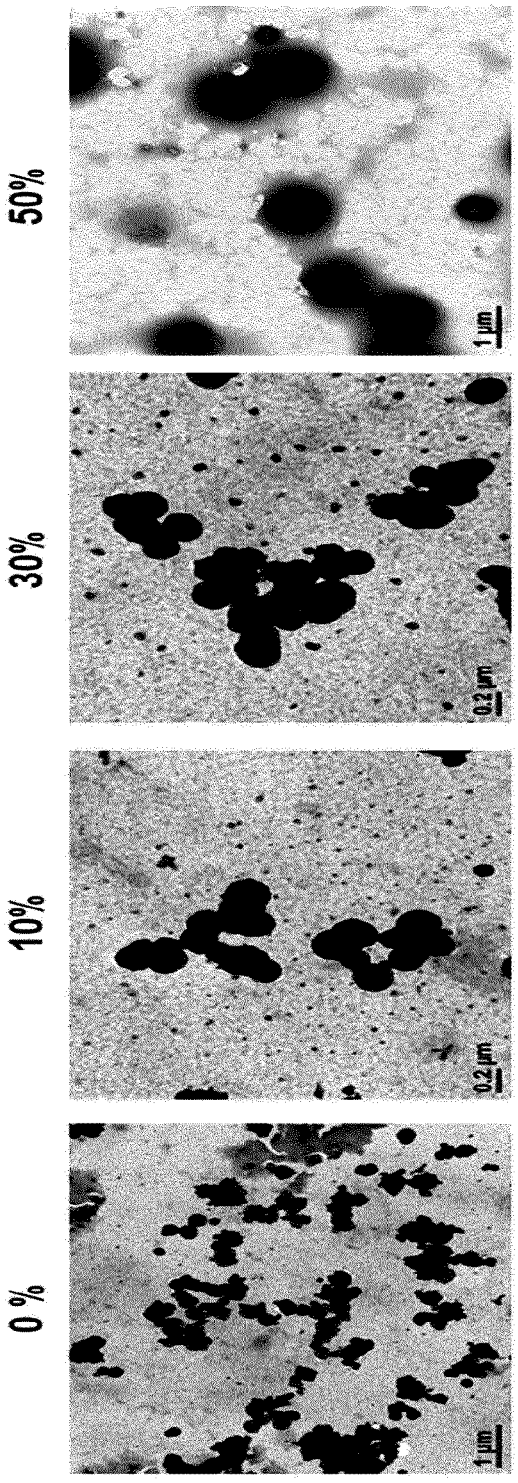

FIG. 44

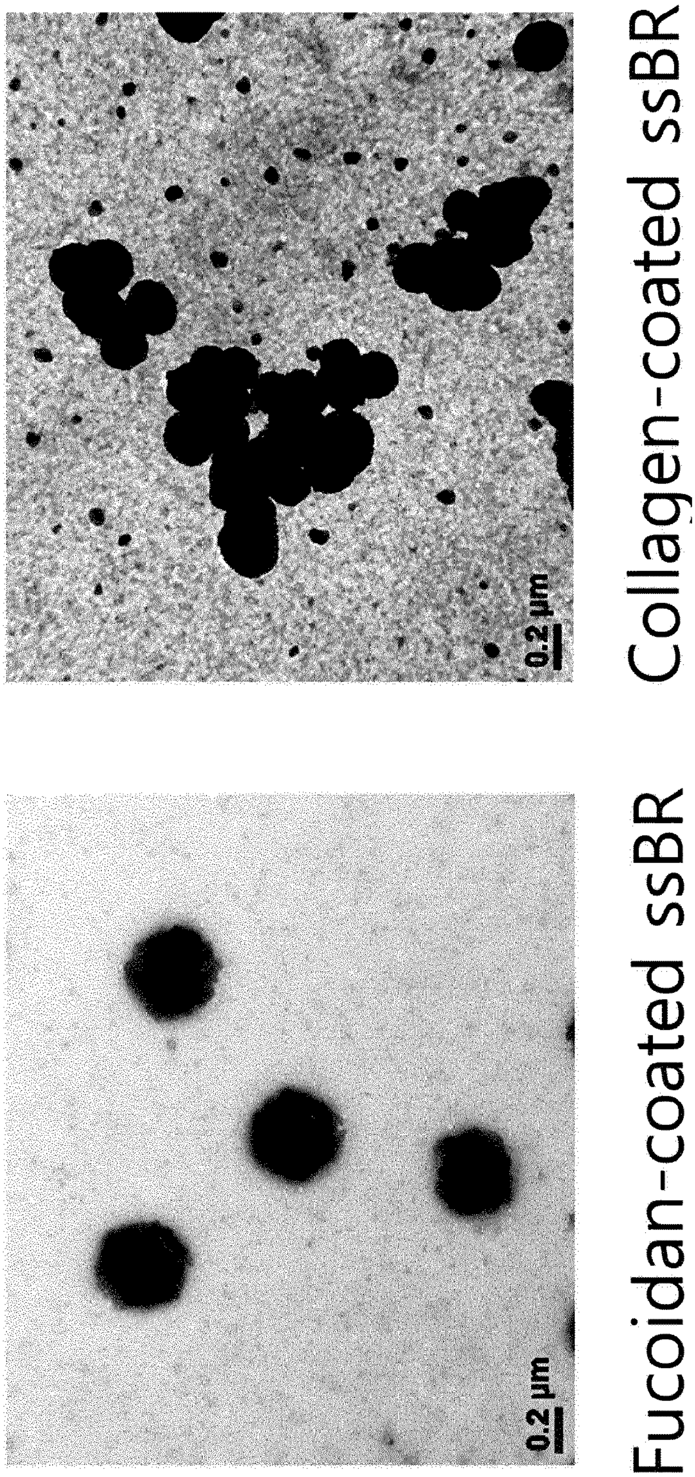

FIG. 45

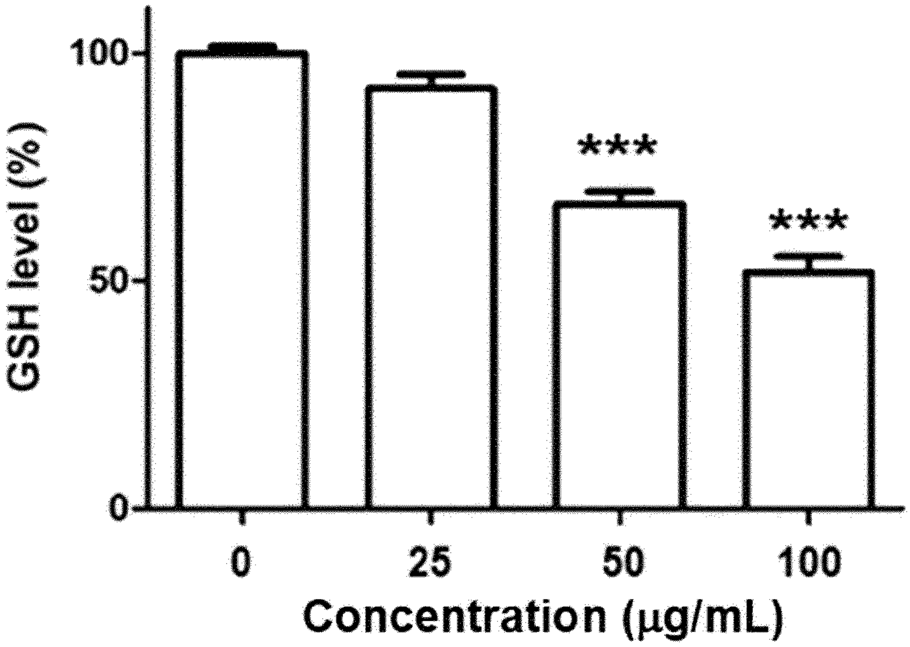
GSH level (%)
100
50
0
***
***
0
25
50
100
Concentration (μg/mL)

FIG. 46

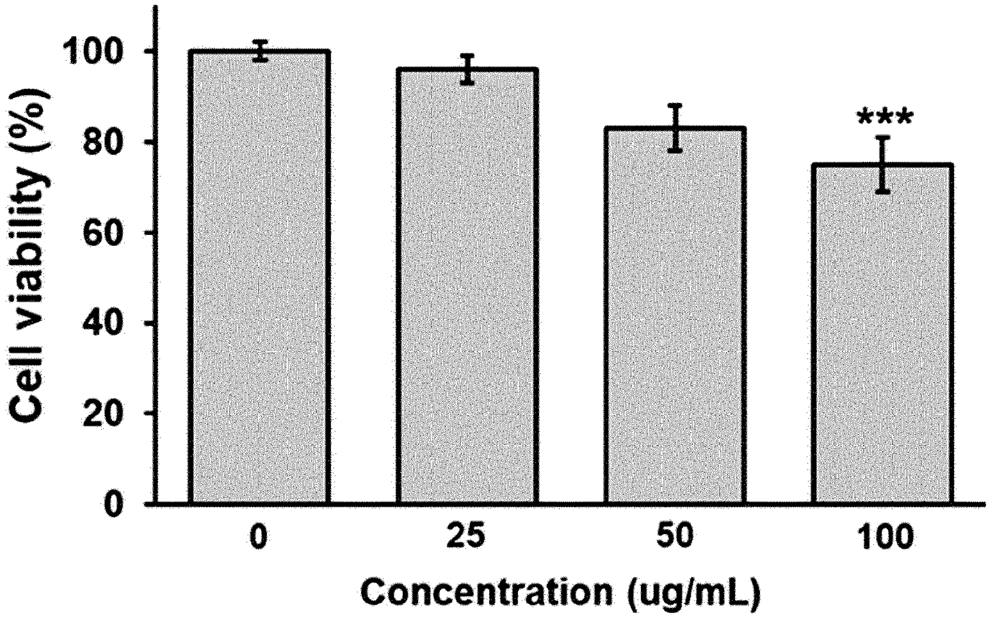
Cell viability (%)
100
80
60
40
20
0
***
0
25
50
100
Concentration (ug/mL)

FIG. 47

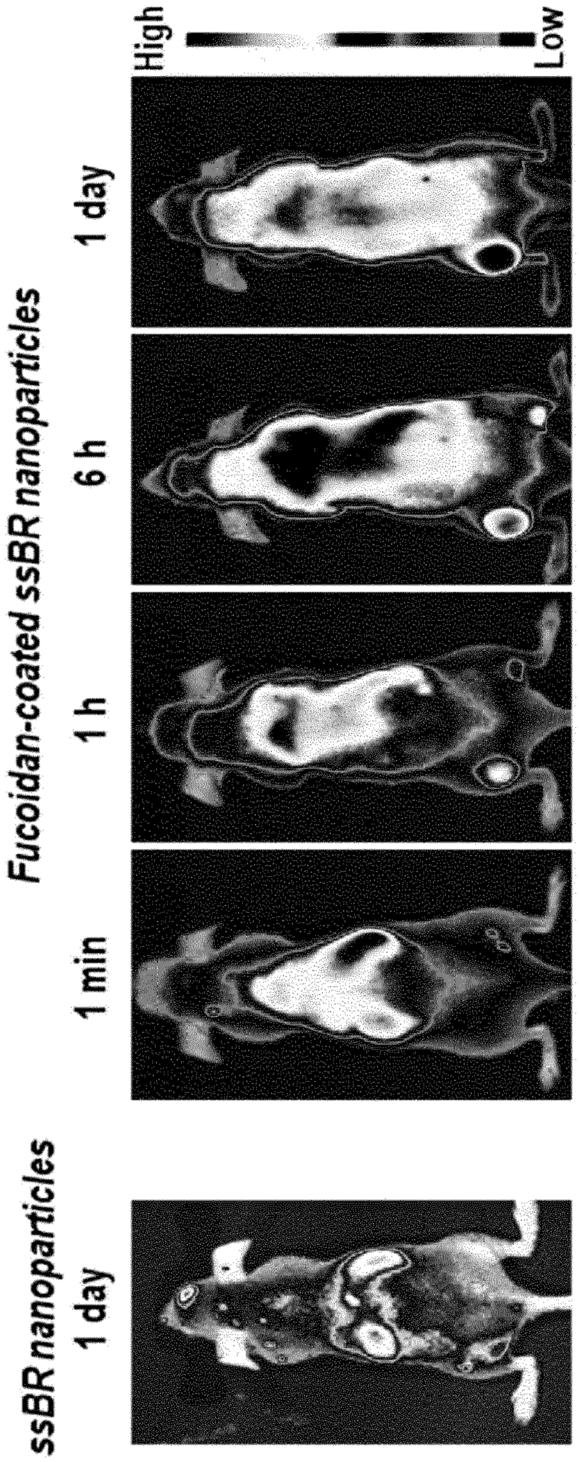
High
Low
Fucoidan-coated ssBR nanoparticles
1 min
1 h
6 h
1 day
ssBR nanoparticles
1 day

FIG. 48

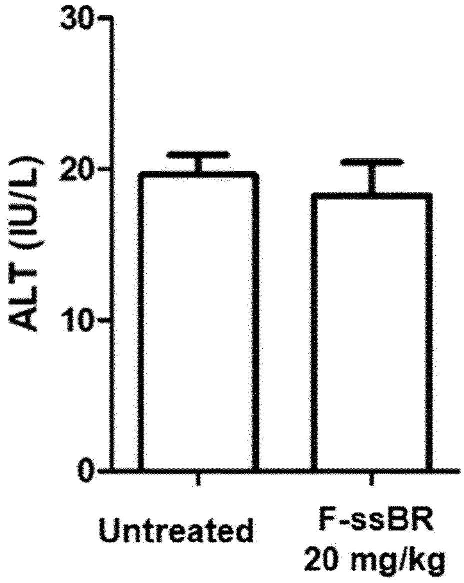

FIG. 49

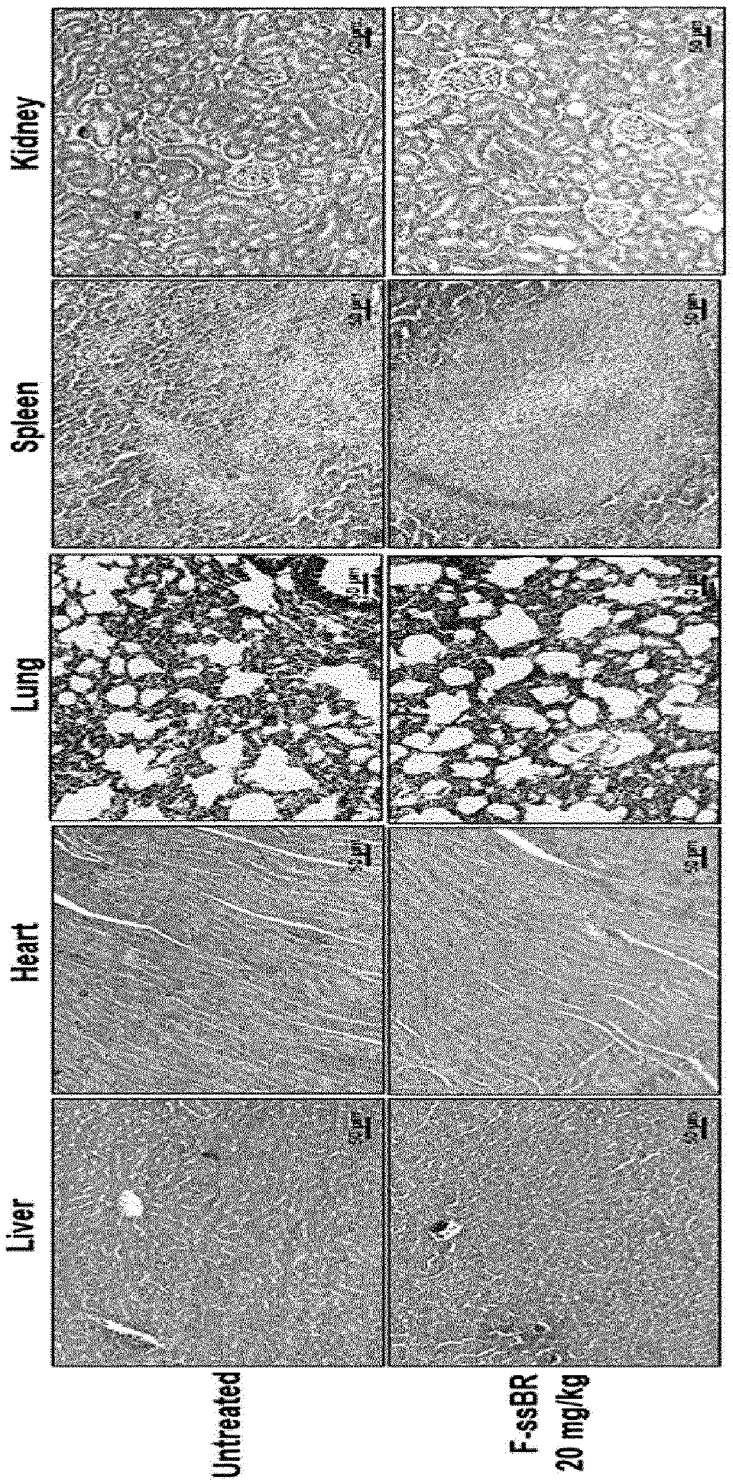

FIG. 50

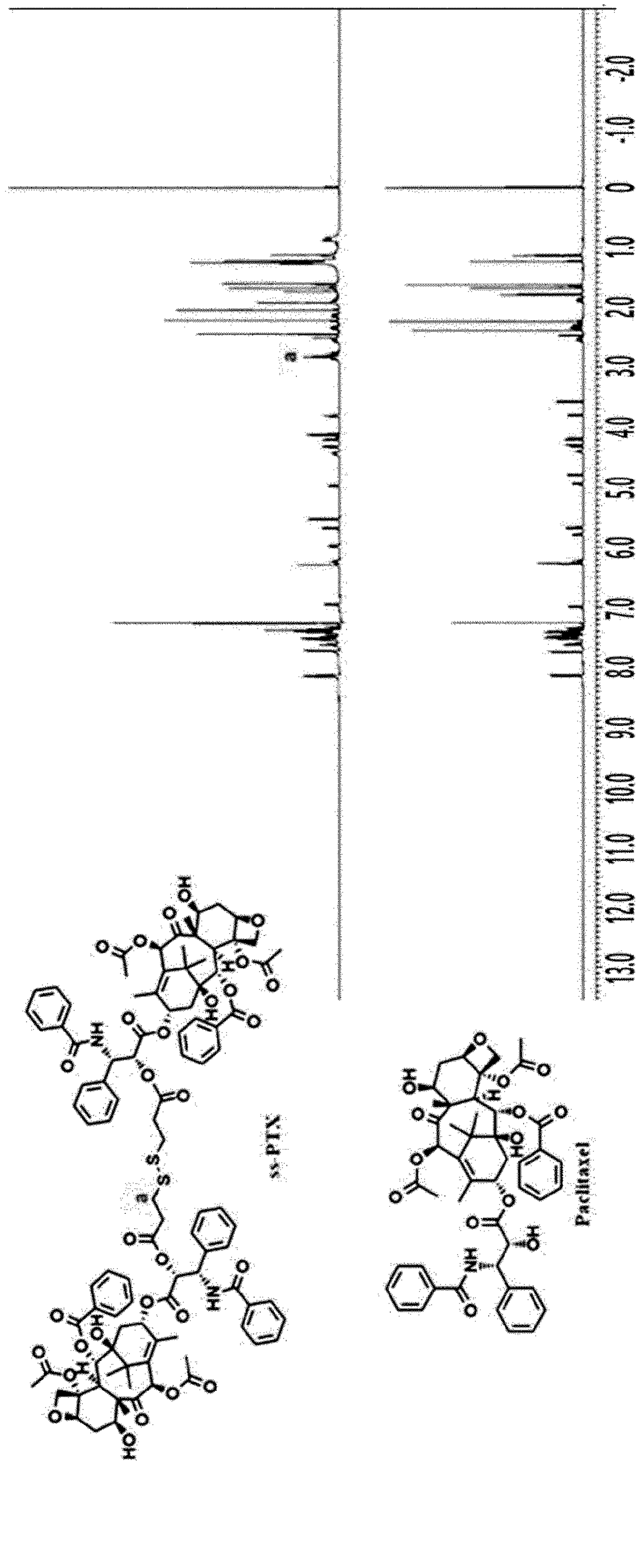
ss-PTX
Paclitaxel
a
13.0
12.0
11.0
10.0
9.0
8.0
7.0
6.0
5.0
4.0
3.0
2.0
1.0
0
-1.0
-2.0

FIG. 51

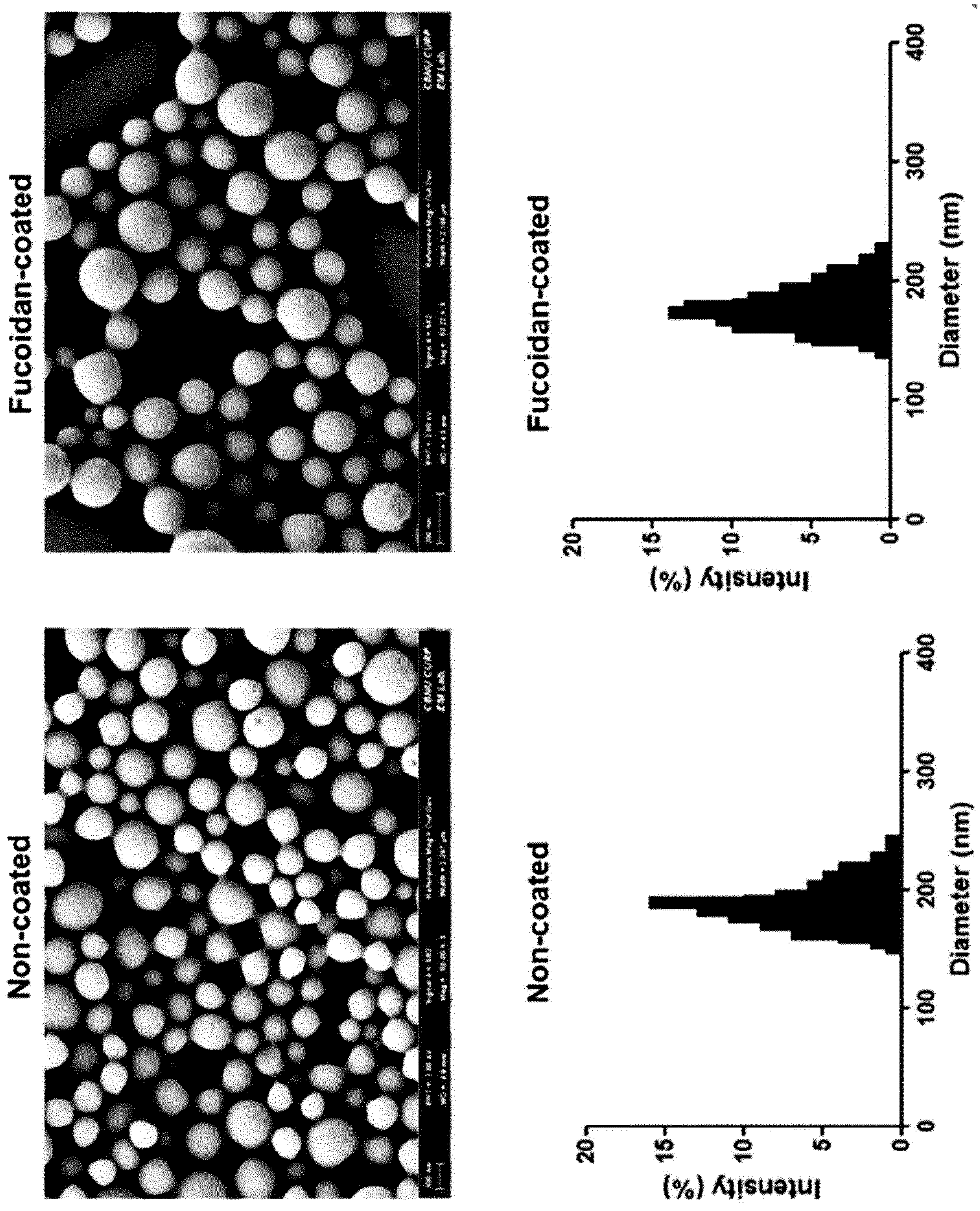
Fucoidan-coated
Non-coated
Fucoidan-coated
Non-coated
Intensity (%)
Diameter (nm)
0
5
10
15
20
0
100
200
300
400
Intensity (%)
Diameter (nm)
0
5
10
15
20
0
100
200
300
400

FIG. 52

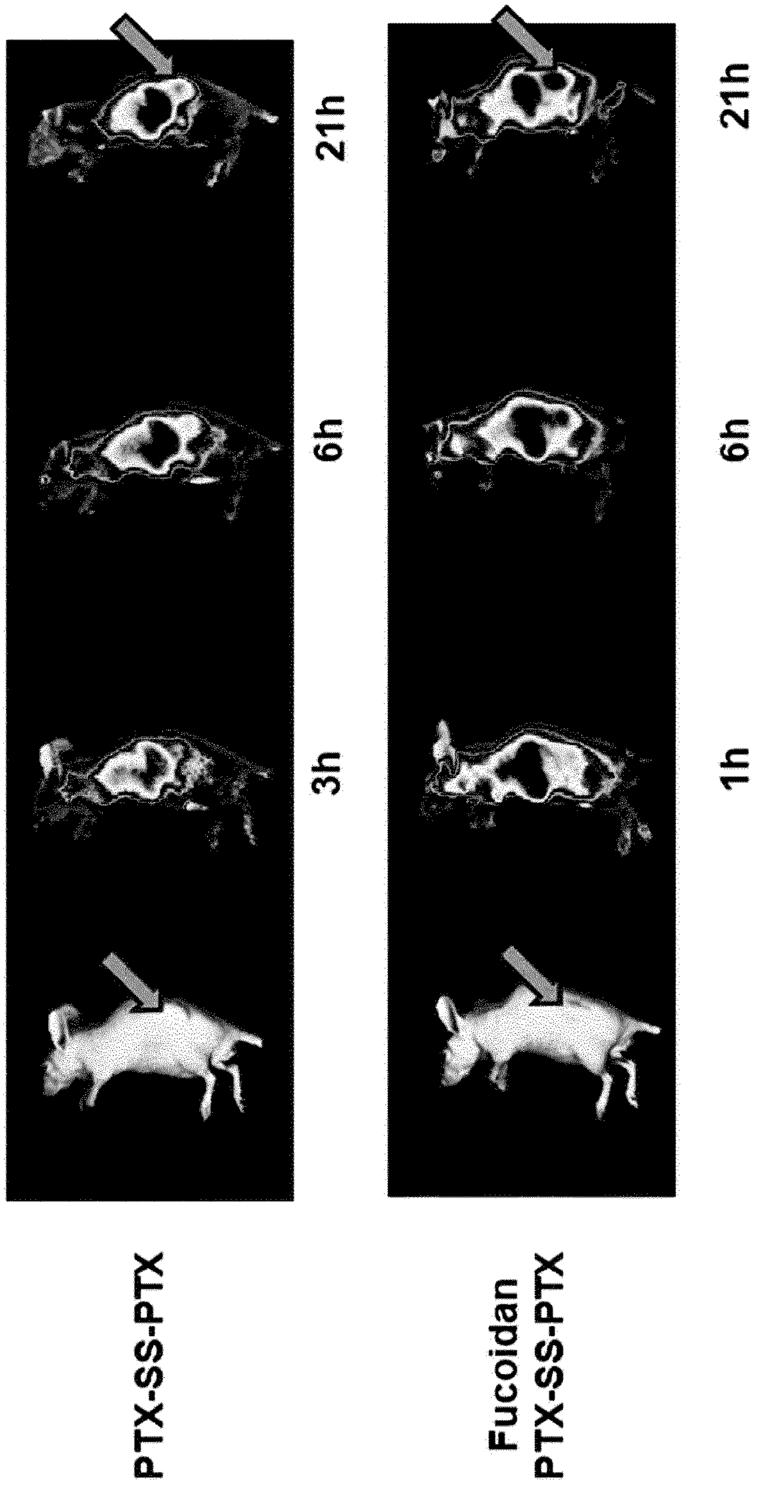

FIG. 53

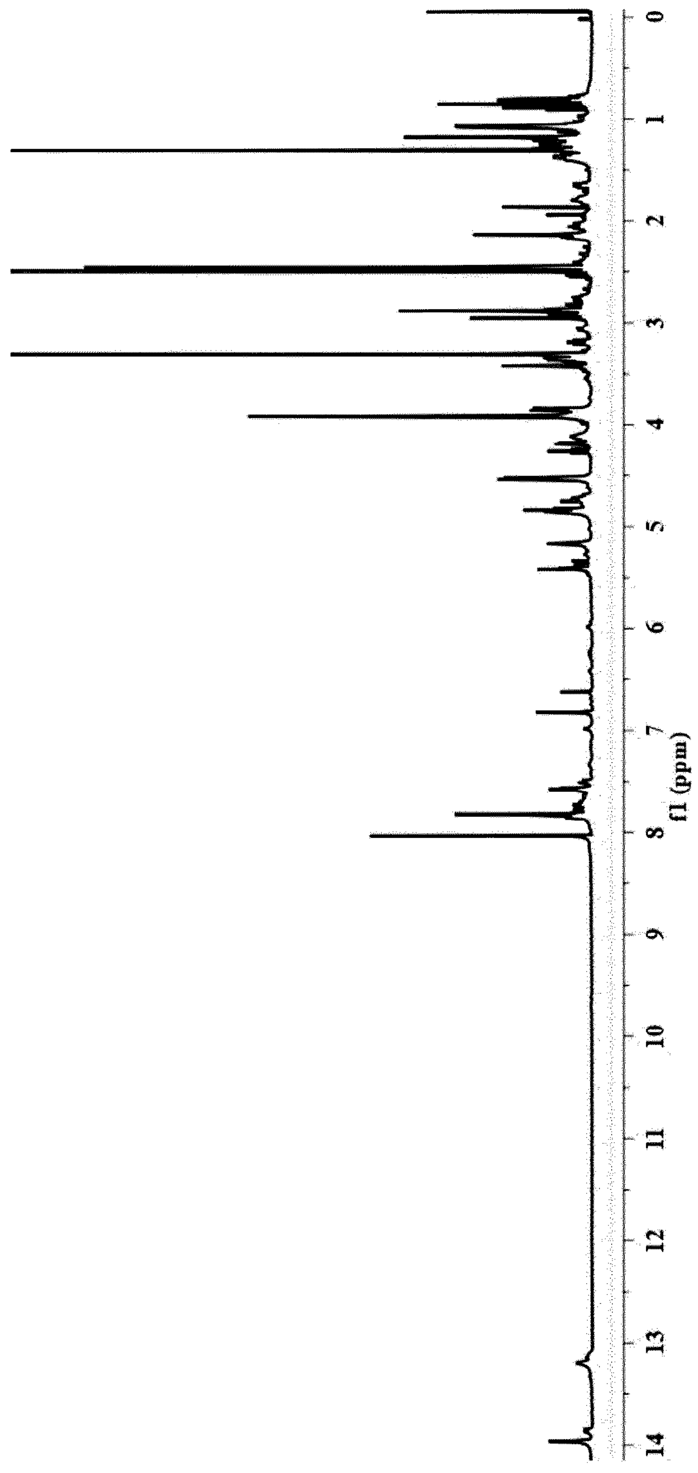

FIG. 54

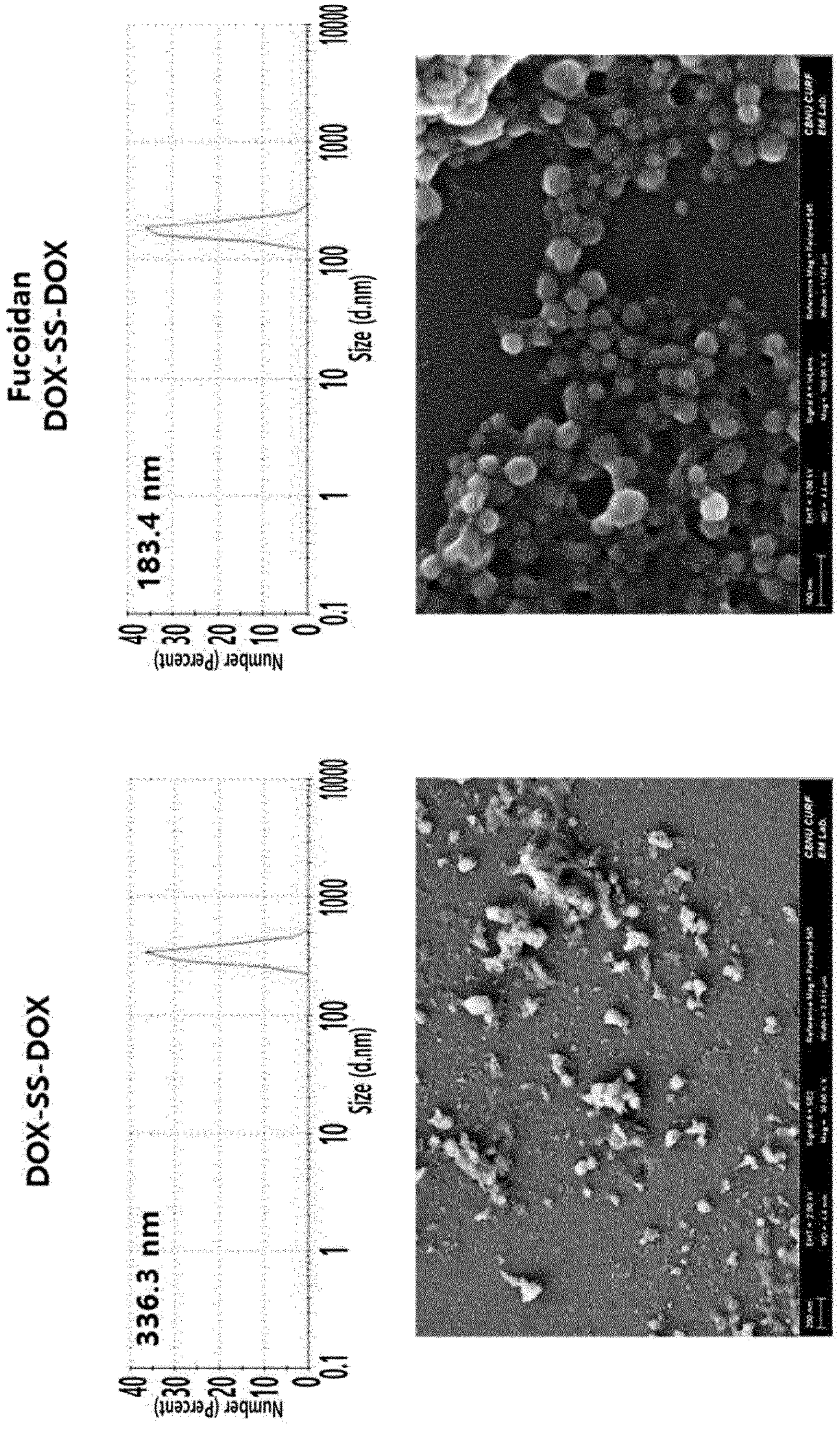
Fucoidan
DOX-SS-DOX
183.4 nm
Number (Percent)
Size (d.nm)
DOX-SS-DOX
336.3 nm
Number (Percent)
Size (d.nm)

FIG. 55

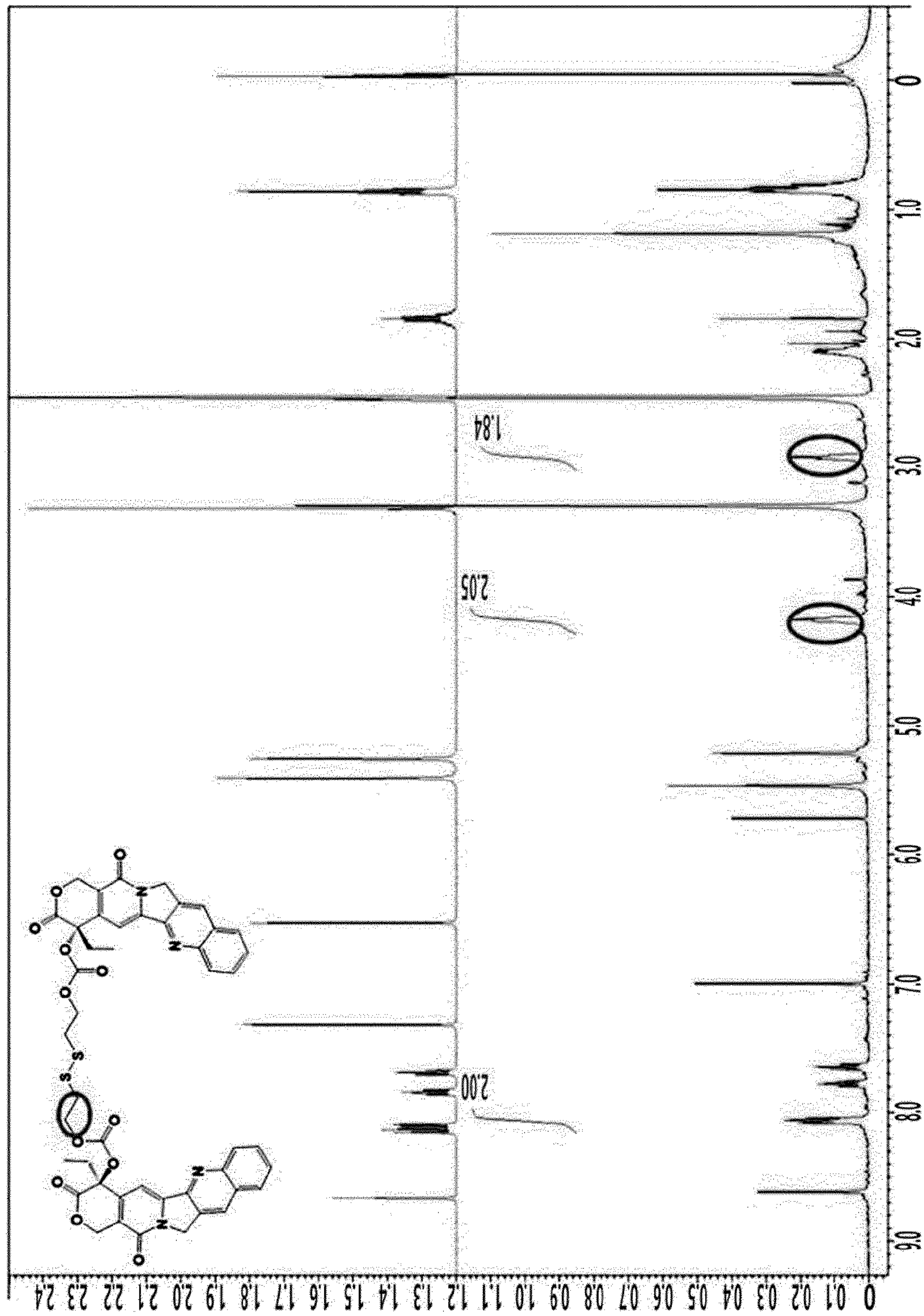

FIG. 56

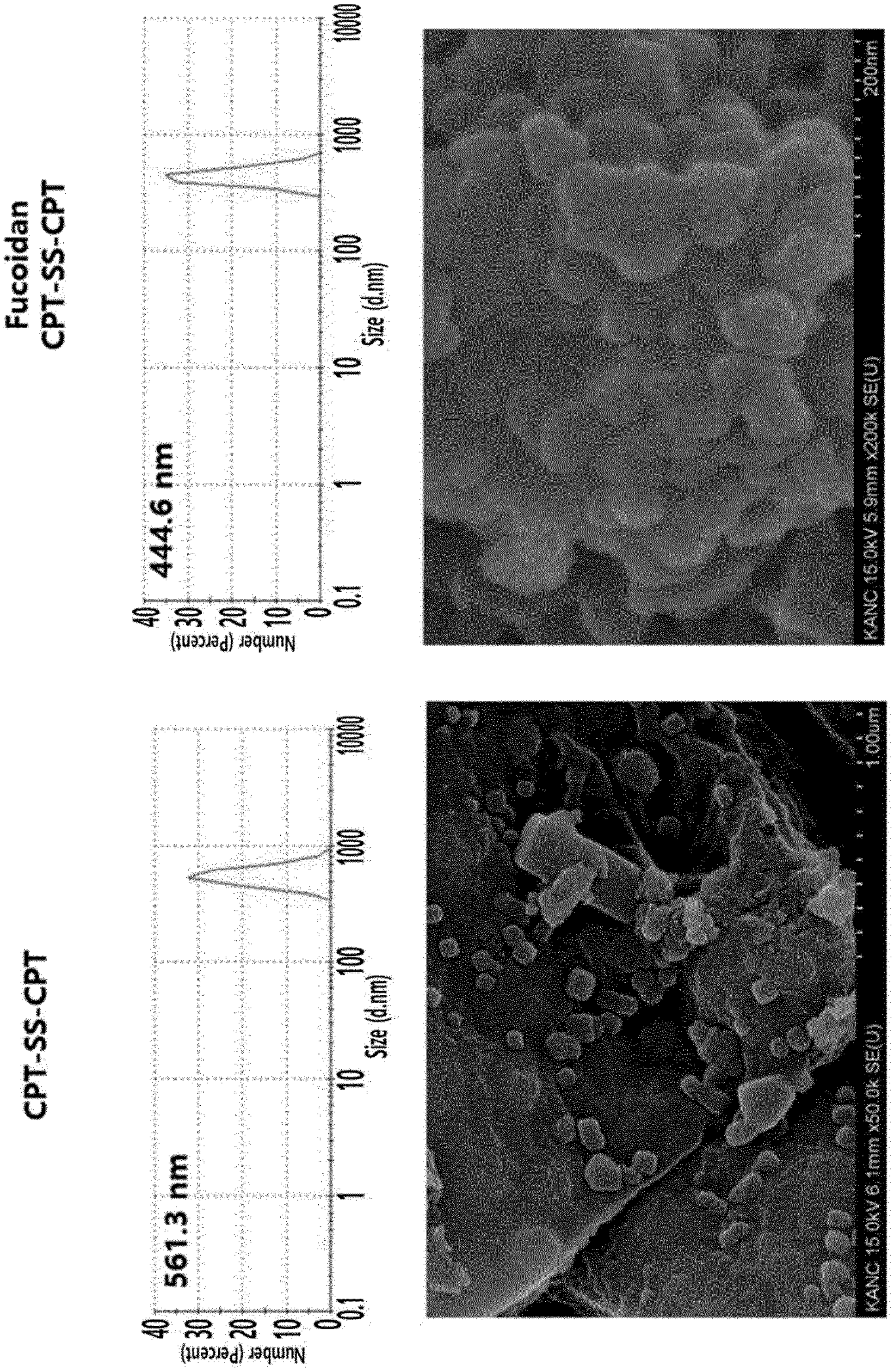
Fucoidan
CPT-SS-CPT
444.6 nm
Number (Percent)
Size (d.nm)
KANC 15.0kV 5.9mm x200k SE(U)
200nm
CPT-SS-CPT
561.3 nm
Number (Percent)
Size (d.nm)
KANC 15.0kV 6.1mm x50.0k SE(U)
1.00um

FIG. 57

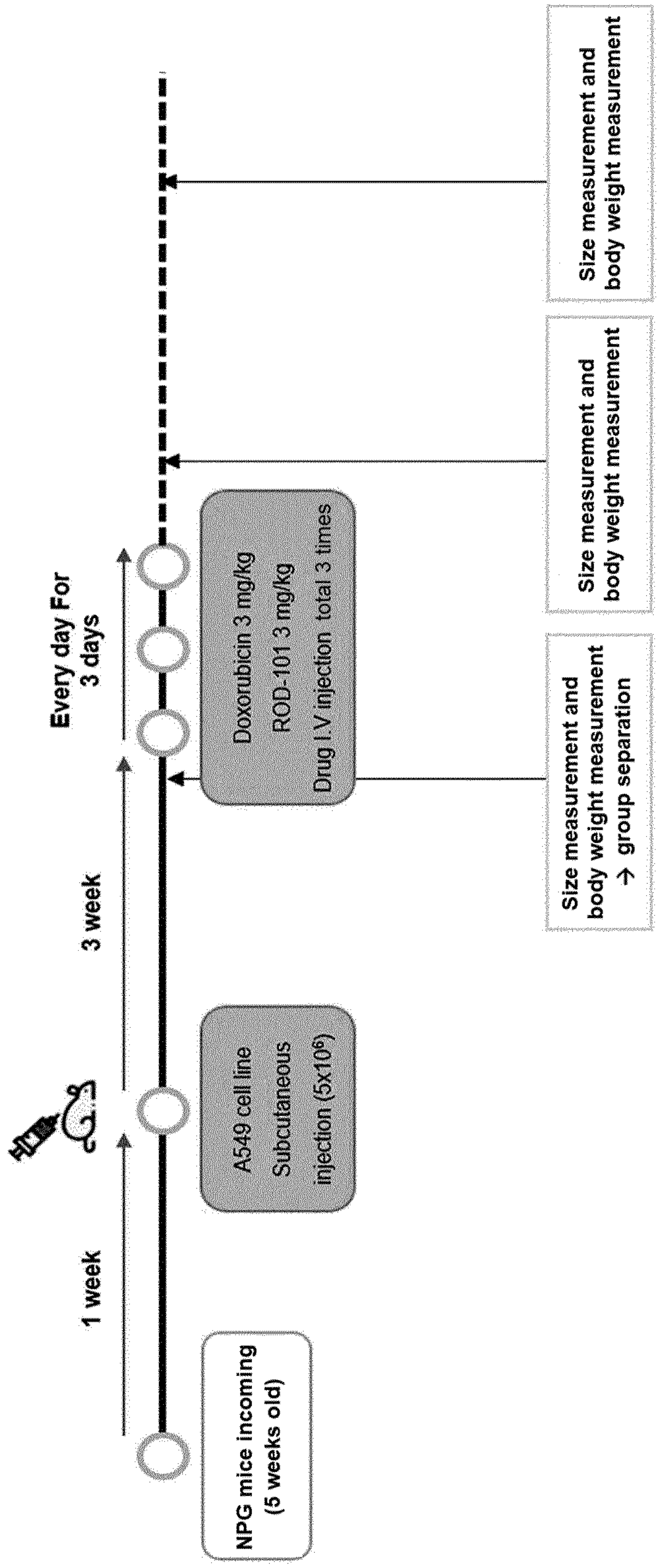

NPG mice incoming (5 weeks old)
1 week
A549 cell line Subcutaneous injection (5x10^6)
3 week
Every day For 3 days
Doxorubicin 3 mg/kg
ROD-101 3 mg/kg
Drug I.V injection total 3 times
Size measurement and body weight measurement → group separation
Size measurement and body weight measurement
Size measurement and body weight measurement

FIG. 58

| Group | | Animal no. | March 12 | | March 18 | | March 22 | |
|---|---|---|---|---|---|---|---|---|
| | | | body weight | volume | body weight | volume | body weight | volume |
| G5 | Sham Group (A549) | 1 | 19.5 | 55.39 | 20.0 | 132.16 | 20.6 | 135.46 |
| | | 2 | 23.1 | 56.66 | 23.4 | 217.70 | 23.5 | 224.16 |
| | | 3 | 23.5 | 47.13 | 23.7 | 134.98 | 24.2 | 144.95 |
| | | AV | 22.03 | 53.06 | 22.37 | 161.61 | 22.77 | 168.19 |
| | | SD | 2.20 | 5.17 | 2.06 | 48.59 | 1.91 | 48.70 |
| G6 | Dox (A549) | 4 | 23.1 | 73.19 | 21.3 | 118.81 | 20 | 108.16 |
| | | 5 | 23.2 | 57.11 | 20.1 | 104.15 | 17.2 | 98.6 |
| | | 6 | 21.8 | 53.99 | 22 | 89.18 | 14.4 | 76.25 |
| | | AV | 22.70 | 61.43 | 21.13 | 104.05 | 17.20 | 94.34 |
| | | SD | 0.78 | 10.30 | 0.96 | 14.82 | 2.80 | 16.38 |
| G7 | ROD-101 (A549) | 7 | 19.5 | 67.83 | 21.8 | 130.42 | 20.1 | 73.53 |
| | | 8 | 23.1 | 58.74 | 23.7 | 82.03 | 22.2 | 77.16 |
| | | 9 | 23.5 | 53.09 | 20.8 | 75.96 | 25.2 | 88.77 |
| | | AV | 22.03 | 59.89 | 22.10 | 96.14 | 22.50 | 79.82 |
| | | SD | 2.20 | 7.44 | 1.47 | 29.84 | 2.56 | 7.96 |

FIG. 59

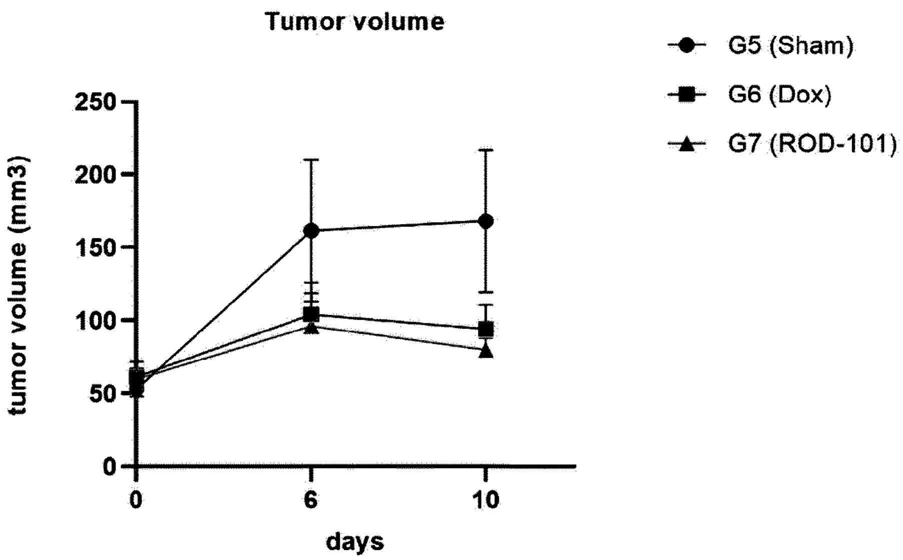

FIG. 60

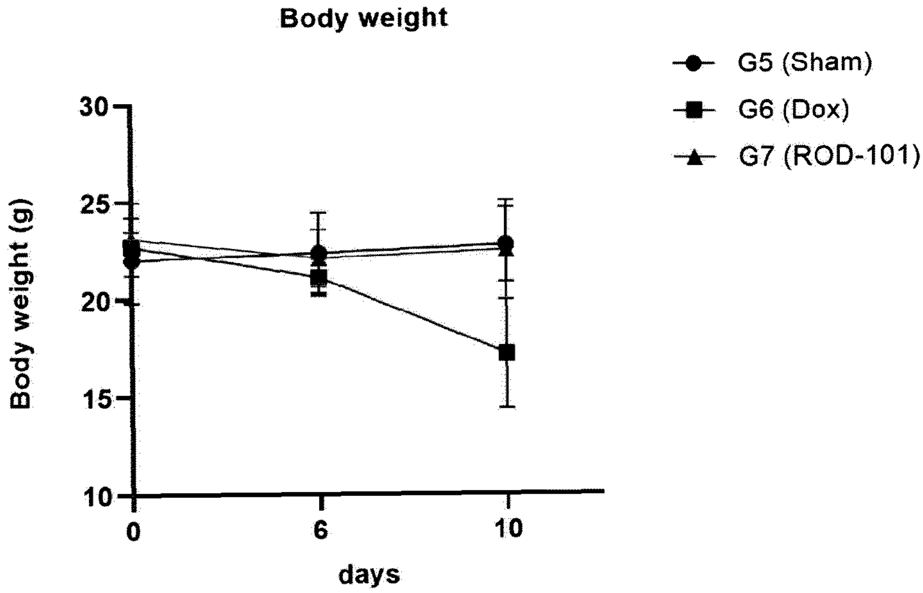
Body weight
G5 (Sham)
G6 (Dox)
G7 (ROD-101)
30
25
20
15
10
Body weight (g)
0
6
10
days

p

**INTERNATIONAL SEARCH REPORT**

International application No. **PCT/KR2021/003765**

**A. CLASSIFICATION OF SUBJECT MATTER**

**C07C 403/24**(2006.01)i; **C07J 41/00**(2006.01)i; **C07C 69/76**(2006.01)i; **C07C 323/26**(2006.01)i; **C07F 5/02**(2006.01)i; **C07H 15/252**(2006.01)i; **C07D 305/14**(2006.01)i; **C07D 491/22**(2006.01)i; **A61K 47/69**(2017.01)i; **A61K 47/36**(2006.01)i

According to International Patent Classification (IPC) or to both national classification and IPC

**B. FIELDS SEARCHED**

Minimum documentation searched (classification system followed by classification symbols)

C07C 403/24(2006.01); A61K 31/195(2006.01); C07C 323/58(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 레티노이드 (retinoid), 이합체 (dimer), 링커 (linker)

**C. DOCUMENTS CONSIDERED TO BE RELEVANT**

| Category* | Citation of document, with indication, where appropriate, of the relevant passages | Relevant to claim No. |
|---|---|---|
| X<br>Y | DE 4032187 A1 (HERMES FABRIK PHARMAZEUTISCHER PRÄPARATE FRANZ GRADINGER GMBH & CO) 16 April 1992 (1992-04-16)<br>See claims 1-47. | 1-3<br>4-9,64-69 |
| Y | LU, L. et al. Liposomes assembled from dimeric retinoic acid phospholipid with improved pharmacokinetic properties. European Journal of Pharmaceutical Sciences. 2018, vol. 112, pp. 186-194.<br>See abstract; and formula 1. | 4-9,64-69 |
| Y | ATASHRAZM, F. et al. Fucoidan enhances the therapeutic potential of arsenic trioxide and all-trans retinoic acid in acute promyelocytic leukemia, in vitro and in vivo. Oncotarget. 2016, vol. 7, no. 29, pp. 46 028-46041.<br>See abstract. | 5-9,66-69 |
| Y | HAN, L. et al. Redox-sensitive micelles for targeted intracellular delivery and combination chemotherapy of paclitaxel and all-trans-retinoid acid. Asian Journal of Pharmaceutical Sciences. 2019, vol. 14, pp. 531-542.<br>See abstract; and figures 1 and 2. | 4-9,64-69 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 August 2021** | **23 August 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No.
PCT/KR2021/003765

C. DOCUMENTS CONSIDERED TO BE RELEVANT

| Category* | Citation of document, with indication, where appropriate, of the relevant passages | Relevant to claim No. |
|---|---|---|
| A | PEI, Q. et al. Glutathione-responsive paclitaxel dimer nanovesicles with high drug content. Biomaterials Science. 2017, vol. 5, pp. 1517-1521. See abstract. | 1-9,64-69 |

Form PCT/ISA/210 (second sheet) (July 2019)

**INTERNATIONAL SEARCH REPORT**

International application No. **PCT/KR2021/003765**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **70**
because they relate to subject matter not required to be searched by this Authority, namely:

Claim 70 pertains to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The invention of group 1 (claims 1-9 and claims 64-69 (partial)) pertains to a retinoid isomer compound represented by chemical formula 1a,
The invention of group 2 (claims 10-18 and claims 64-69 (partial)) pertains to an ursodeoxycholic acid isomer compound represented by chemical formula 2a,
The invention of group 3 (claims 19-27 and claims 64-69 (partial)) pertains to a 4-(hydroxymethyl)phenyl benzoate isomer compound represented by chemical formula 3a,
The invention of group 4 (claims 28-36 and claims 64-69 (partial)) pertains to a 4-(hydroxymethyl)phenylboronic acid pinacol ester isomer compound represented by chemical formula 4a,
The invention of group 5 (claims 37-45 and claims 64-69 (partial)) pertains to a paclitaxel isomer compound represented by chemical formula 5a,
The invention of group 6 (claims 46-54 and claims 64-69 (partial)) pertains to a doxorubicin isomer compound represented by chemical formula 6a,
The invention of group 7 (claims 55-63 and claims 64-69 (partial)) pertains to a camptothechin isomer compound represented by chemical formula 7a.

The technical feature shared by all of these claims pertains to an isomer compound linked with a linker. However, the isomer compound linked with a linker corresponds to a technology found in D5 (PEI, Q. et al. Glutathione-responsive paclitaxel dimer nanovesicles with high drug content. Biomaterials Science. 2017, vol. 5, pages 1517-1521).

Therefore, these claims have no common special technical feature which makes a contribution over the prior art under PCT Rule 13.2, and thus lack unity of invention.

* Claim 70 pertains to a method for treatment of the human body (PCT Article 17(2)(a)(i) and PCT Rule 39.1(iv)), and thus pertains to a subject matter excluded from the international search report.

Form PCT/ISA/210 (continuation of first sheet) (July 2019)

**INTERNATIONAL SEARCH REPORT**

International application No.
**PCT/KR2021/003765**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-9 and 64-69 (part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/KR2021/003765**

| Patent document cited in search report | Publication date (day/month/year) | Patent family member(s) | Publication date (day/month/year) |
|---|---|---|---|
| DE 4032187 A1 | 16 April 1992 | DE 4032187 C2 | 30 March 1995 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MILENA MENOZZI et al.** *JPharmSci,* 01 June 1984